# EUROPEAN PATENT SPECIFICATION

(11) **EP 3 567 149 B1**
(45) Date of publication and mention of the grant of the patent: **26.05.2021**
(21) Application number: 19154432.9
(22) Date of filing: 30.01.2019
(51) Int. Cl.: D04H 1/488, D04H 1/498, D04H 1/44, D04H 1/54, D04H 1/558, D04H 1/559, D04H 1/732, D04H 1/736, D21F 9/00, D21F 11/04, D21H 21/52, D21H 27/30, D21H 27/32, A01N 25/34, D04H 1/407, D04H 1/413, A61L 9/012, A61L 9/014, F24F 3/14

(54) **SHEET MANUFACTURING APPARATUS AND SHEET MANUFACTURING METHOD**
BLATTHERSTELLUNGSVORRICHTUNG UND BLATTHERSTELLUNGSVERFAHREN
APPAREIL ET PROCÉDÉ DE FABRICATION DE FEUILLE

(30) Priority: 30.01.2018 JP 2018013954; 30.01.2018 JP 2018013955; 30.01.2018 JP 2018013956
(43) Date of publication of application: 13.11.2019
(73) Proprietor: Seiko Epson Corporation, Tokyo 160-8801 (JP)
(72) Inventor: OTA, Tsukasa, Suwa-shi, Nagano 392-8502 (JP)
(74) Representative: Miller Sturt Kenyon

(56) References cited:
- EP-A1- 0 312 512
- WO-A1-99/25924
- WO-A1-2010/093311
- WO-A1-2014/174410
- WO-A2-98/31858
- DE-A1-102014 107 725
- FR-A1- 2 279 874
- GB-A- 1 362 666
- US-A- 3 905 864

## Description

### BACKGROUND

### 1. Technical Field

The present invention relates to a sheet manufacturing apparatus and a sheet manufacturing method.

### 2. Related Art

In the related art, an apparatus for processing a fiber into a sheet shape is known (for example, refer to JP-A-2015-183321). JP-A-2015-183321 discloses an apparatus for manufacturing a sheet by mixing materials containing fibers and a functional material with each other. In the apparatus, by heating a web on which two mixtures having different proportions of the fibers and the functional material are deposited, a sheet having different proportions of the functional material on one surface and a rear surface thereof is manufactured.

In the apparatus described in JP-A-2015-183321, since a roller that heats the web pressurizes the web and increases the density, the density and the like in a thickness direction of the manufactured sheet are uniform. Therefore, there was no disclosure of a method for adjusting the density and the like in the thickness direction of the sheet. In addition, there was no disclosure of a method of changing the state, such as density and hardness, for each layer. Furthermore, the sheet to be manufactured by the apparatus described in JP-A-2015-183321 is configured to contain the fibers and the functional material, and it was possible to make the proportions of the fibers and the functional material different, but there was no disclosure of a method for changing the type of the material of the sheet.

US 3905864 discloses a manufacturing apparatus for making a 3- or more ply sheet. The apparatus has a first station that includes fibre dispensing heads and a vacuum box for forming a first web. The first web is hot pressed by passage around the surface of a steam heated cylinder with three pressure nips provided by rollers. Similar stations make webs in a similar way. The webs formed by the first two stations are laminated by rollers, which press the webs together to form a 2-ply web. Water, binder or another solution providing barrier properties may be sprayed between the plies by sprayer. A set of pressure rolls effects further pressing. Webs from the other stations are laminated in a similar way.

### SUMMARY

An advantage of some aspects of the invention is to make it possible to adjust the density and the like in the thickness direction of a sheet when manufacturing a sheet from a material containing fibers. In addition, another advantage of some aspects of the invention is to change the state for each layer of the sheet. Furthermore, still another advantage of some aspects of the invention is to realize a change in type of the material inside the sheet when manufacturing the sheet containing fibers.

According to an aspect of the invention, there is provided a sheet manufacturing apparatus as defined in claim 1.

With this configuration, in a process of manufacturing a sheet by laminating a plurality of webs, it is possible to perform the pressurizing processing and/or the heating processing under different conditions for each layer. Therefore, it is possible to manufacture a sheet having different states for each layer.

In the sheet manufacturing apparatus, a first cut unit that cuts the first web processed by the first processing unit may further be provided, and the second web forming unit may process the first web cut by the first cut unit.

In the sheet manufacturing apparatus, the first processing unit may include first pressurizing rollers that nip and pressurize the first web.

In the sheet manufacturing apparatus, the first processing unit may include first heating rollers that nip and heat the first web.

In the sheet manufacturing apparatus, the second processing unit may include second pressurizing rollers that nip and pressurize the second web.

In the sheet manufacturing apparatus, the second processing unit may include a heating unit that heats the second web.

In the sheet manufacturing apparatus, the second processing unit may include a second pressurizing unit that pressurizes the second web and the heating unit that heats the second web pressurized by the second pressurizing unit in a non-pressurized state.

In the sheet manufacturing apparatus, a pressing processing unit that partially presses the first web and gives an uneven shape to the first web may further be provided, and the first processing unit may process the first web processed by the pressing processing unit.

In the sheet manufacturing apparatus, a printing unit that performs printing on the sheet molded by the second processing unit may further be provided.

According to still another aspect of the invention, there is provided a sheet manufacturing method as set out in claim 10.

With this configuration, in a process of manufacturing a sheet by laminating a plurality of webs, it is possible to perform the pressurizing processing and/or the heating processing under different conditions for each layer. Therefore, it is possible to manufacture a sheet having different states for each layer.

According to still another aspect of the invention, there is provided a sheet manufacturing apparatus including: a web forming unit that forms a web based on a first material containing fibers; a second material disposing unit that disposes a second material on the web formed by the web forming unit; and a sheet forming unit that manufactures a sheet by processing the web on which the second material is disposed by the second material disposing unit.

With this configuration, since it is possible to manufacture a sheet in a state where the second material overlaps the first material containing fibers, for example, it is possible to configure a sheet on which the first material and the second material overlap each other. Therefore, by changing the types of the first material and the second material, it is possible to change the type of the material inside the sheet.

In the sheet manufacturing apparatus, the first material may be a mixture containing fibers and a resin, and the sheet forming unit may heat the web and manufacture the sheet.

In the sheet manufacturing apparatus, the second material disposing unit may dispose the second material made of a material other than the fibers and the resin contained in the first material on the web.

In the sheet manufacturing apparatus, the second material disposing unit may dispose the second material containing a solid material having particles larger than particles of the resin contained in the first material on the web.

In the sheet manufacturing apparatus, the web forming unit may include a dispersing unit that disperses the first material in the air, and a depositing unit that deposits the first material dispersed by the dispersing unit and forms the web, and the second material disposing unit may dispose the second material on the web deposited by depositing unit.

In the sheet manufacturing apparatus, a third material disposing unit that is disposed between the second material disposing unit and the sheet forming unit, and disposes a third material on the web on which the second material is disposed by the second material disposing unit may further be provided.

In the sheet manufacturing apparatus, a printing unit that performs printing on the sheet manufactured by the sheet forming unit may further be provided.

In the sheet manufacturing apparatus, the sheet forming unit may include a pressurizing unit that pressurizes the web, and a heating unit that heats the web pressurized by the pressurizing unit in a non-pressurized state.

In the sheet manufacturing apparatus, a pressing processing unit that partially presses the web and gives an uneven shape to the web may further be provided.

According to still another aspect of the invention, there is provided a sheet manufacturing method including: forming a web based on a first material containing fibers; disposing a second material on the web formed in the forming; and manufacturing a sheet by processing the web on which the second material is disposed in the disposing.

With this configuration, since it is possible to manufacture a sheet in a state where the second material overlaps the first material containing fibers, for example, it is possible to configure a sheet on which the first material and the second material overlap each other. Therefore, by changing the types of the first material and the second material, it is possible to change the type of the material inside the sheet.

### BRIEF DESCRIPTION OF THE DRAWINGS

Embodiments of the invention will now be described with reference to the accompanying drawings, wherein like numbers reference like elements.
Fig. 1 is a schematic configuration view of a sheet manufacturing apparatus according to a first embodiment.
Fig. 2 is a view illustrating a detailed configuration of a mixing unit.
Fig. 3 is a sectional view of a sheet manufactured by the sheet manufacturing apparatus according to the first embodiment.
Fig. 4 is a schematic configuration view of a sheet manufacturing apparatus according to a second embodiment.
Fig. 5 is a view illustrating a detailed configuration of a mixing unit according to the second embodiment.
Fig. 6 is a sectional view of a sheet manufactured by the sheet manufacturing apparatus according to the second embodiment.
Fig. 7 is an explanatory view illustrating a configuration of a processing roller according to a third embodiment.
Fig. 8 is an explanatory view illustrating a configuration of a processing roller according to a fourth embodiment.
Fig. 9 is a schematic configuration view of a sheet manufacturing apparatus according to a fifth embodiment.
Fig. 10 is a schematic configuration view of a sheet manufacturing apparatus according to a sixth embodiment.
Fig. 11 is a schematic configuration view of a sheet manufacturing apparatus according to a seventh embodiment.
Fig. 12 is a sectional view of a sheet manufactured by the sheet manufacturing apparatus.
Fig. 13 is a schematic configuration view of a sheet manufacturing apparatus according to an eighth embodiment.
Fig. 14 is an explanatory view illustrating a configuration of a processing roller according to the eighth embodiment.
Fig. 15 is an explanatory view illustrating a configuration of a processing roller according to a ninth embodiment.
Fig. 16 is a schematic configuration view of a sheet manufacturing apparatus according to a tenth embodiment.
Fig. 17 is a schematic configuration view of a sheet manufacturing apparatus according to an eleventh embodiment.
Fig. 18 is a schematic configuration view of a sheet manufacturing apparatus according to a twelfth embodiment.
Fig. 19 is a schematic configuration view of a sheet manufacturing apparatus according to a thirteenth embodiment.
Fig. 20 is a side view of a main part side of a solid feeder.
Fig. 21 is a view illustrating a configuration example of a sheet manufactured by the sheet manufacturing apparatus.
Fig. 22 is a schematic configuration view of a sheet manufacturing apparatus according to a fourteenth embodiment.
Fig. 23 is an explanatory view illustrating a configuration of a processing roller according to a fifteenth embodiment.
Fig. 24 is a schematic configuration view of a sheet manufacturing apparatus according to a sixteenth embodiment.
Fig. 25 is a schematic configuration view of a sheet manufacturing apparatus according to a seventeenth embodiment.
Fig. 26 is a schematic configuration view of a sheet manufacturing apparatus according to an eighteenth embodiment.
Fig. 27 is an explanatory view illustrating a configuration of a processing roller according to a nineteenth embodiment.

### DESCRIPTION OF EXEMPLARY EMBODIMENTS

Hereinafter, appropriate embodiments of the invention will be described in detail with reference to the drawings. In addition, the embodiments which will be described below do not limit the contents of the invention described in the Claims. In addition, not all of the configurations which will be described below are necessarily essential configuration elements of the invention.

### 1. First Embodiment

### 1-1. Overall Configuration of Sheet Manufacturing Apparatus

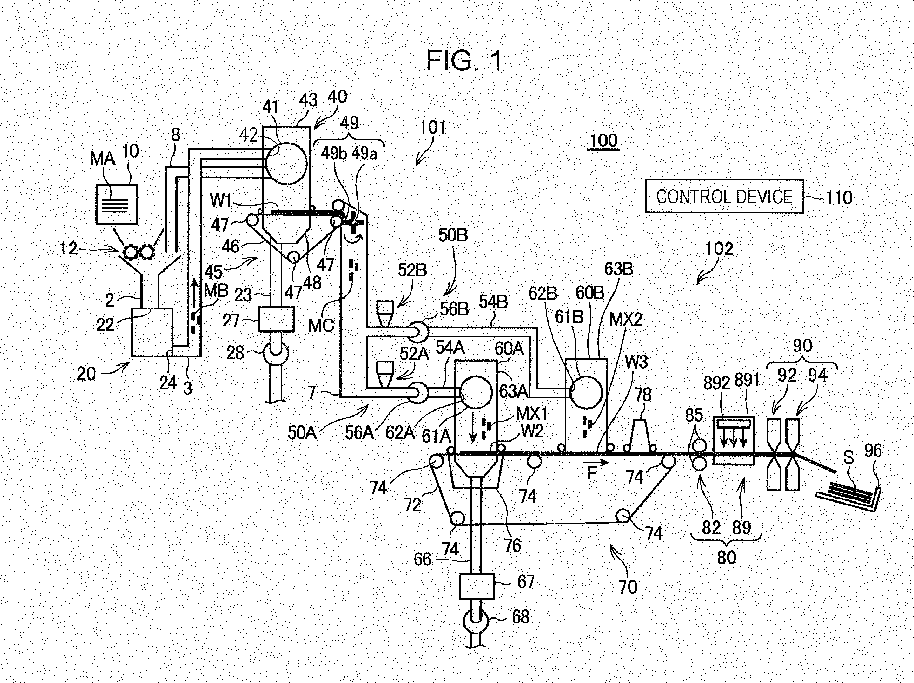

Fig. 1 is a schematic view illustrating a configuration of a sheet manufacturing apparatus 100.

The sheet manufacturing apparatus 100 executes regeneration processing of fiberizing a raw material MA containing fibers and regenerating the fiberized material into a new sheet S. The sheet manufacturing apparatus 100 is capable of manufacturing a plurality of types of sheets S, and for example, by mixing additives with the fiberized raw material MA, according to the use, it is possible to adjust the bonding strength or whiteness of the sheet S, or to add a function of color, scent, flame retardancy and the like. Further, the sheet manufacturing apparatus 100 can adjust density, thickness, size, and shape of the sheet S. As a representative example of the sheet S, in addition to a sheet-like product, such as a printing paper sheet having a fixed size of A4 or A3, a cleaning sheet (for example, a floor cleaning sheet), an oil stain preventing sheet, or a toilet cleaning sheet, a shape of a paper tray and the like can be employed.

**The** sheet manufacturing apparatus 100 broadly includes a defibration processing unit 101 that obtains a material of the sheet S by refining the raw material MA, and a manufacturing unit 102 that manufactures the sheet S by processing the material obtained by the defibration processing unit 101.

The defibration processing unit 101 includes a supply unit 10, a coarse crushing unit 12, a defibrating unit 20, a sorting unit 40, a material molding unit 45, and a rotation body 49. The manufacturing unit 102 includes a first mixing unit 50A and a second mixing unit 50B, a web forming unit 70, a processing unit 80, and a cutting unit 90. The web forming unit 70 includes a first depositing unit 60A and a second depositing unit 60B. The rotation body 49 may be classified into the defibration processing unit 101 or the manufacturing unit 102.

The sheet manufacturing apparatus 100 includes a control device 110 that controls each portion of the sheet manufacturing apparatus 100.

The supply unit 10 is an automatic charging device that accommodates the raw material MA therein and continuously charges the raw material MA into the coarse crushing unit 12. For example, the supply unit 10 includes a stocker (not illustrated) for stacking the raw material MA and a feeder (not illustrated) for feeding the raw material MA from the stocker. The raw material MA may be a material that contains fibers, for example, waste paper, discarded paper, or pulp sheet.

The coarse crushing unit 12 is a shredder that cuts the raw material MA supplied by the supply unit 10 with a blade. The raw material MA cut by the coarse crushing unit 12 is transported to the defibrating unit 20 via a pipe 2.

The defibrating unit 20 defibrates a coarse debris of the raw material MA. Defibration is a process of disentangling the raw material MA in a state where a plurality of fibers are bound to each other into single fibers or a group of a small number of fibers. The raw material MA can also be called a defibrated material. By defibrating the raw material MA by the defibrating unit 20, in addition to the effect of disentangling the fibers contained in the raw material MA, it is possible to expect an effect of separating substances, such as resin particles, ink, toner, blot preventing agent and the like that adhere to the raw material MA, from the fibers. The material that has passed through the defibrating unit 20 is called a defibrated material MB.

In addition to the disentangled fibers, the defibrated material MB contains inclusions separated from the fiber when the defibrating unit 20 disentangles the fibers. The inclusions are particles or powder, and the components of the inclusions are, for example, resin particles contained in the raw material MA, coloring agents, such as ink or toner, or additives, such as blot preventing material or paper strength enhancing agent. The resin particle is a resin mixed for binding the plurality of fibers to each other at the time of manufacturing the raw material MA. The shape of the fiber contained in the defibrated material MB is, for example, a string shape or a ribbon shape. The fibers contained in the defibrated material MB may exist in an independent state while not being intertwined with other fibers, or may exist in a state of forming a so-called "dummy" while being in a form of a mass intertwined with other fibers.

The defibrating unit 20 performs defibration by a dry process. The dry process indicates processing in the air, such as atmosphere, rather than in liquid. The defibrating unit 20 can be configured, for example, by using a defiberizer, such as an impeller-mill. Specifically, the defibrating unit 20 includes a rotor (not illustrated) that rotates and a liner (not illustrated) positioned at an outer circumference of the rotor (not illustrated), and defibrates the coarse debris by nipping the coarse debris between the rotor and the liner.

From the coarse crushing unit 12 to the defibrating unit 20, the coarse debris is transported by an airflow. A configuration in which the airflow is generated by the defibrating unit 20 may be employed, or the airflow may be generated by providing a blower (not illustrated) on an upstream side or a downstream side of the defibrating unit 20 in a transport direction of the coarse debris or the defibrated material MB. In addition, the defibrated material MB is sent from the defibrating unit 20 through a pipe 3 to the sorting unit 40 by the airflow. The airflow by which the defibrated material MB is transported to the sorting unit 40 may be generated by the defibrating unit 20, or the airflow of the above-described blower may be used.

**The** sorting unit 40 sorts the components contained in the defibrated material MB according to the size of the fibers. The size of the fiber mainly refers to the length of the fiber.

**The** sorting unit 40 includes a drum unit 41 and a housing unit 43 that accommodates the drum unit 41 therein. The drum unit 41 is a member that functions as a sieve, and includes, for example, a mesh, a filter, a screen, and the like that function as a sieve having an opening. The drum unit 41 has a cylindrical shape that is rotationally driven by a motor, and at least a part of a circumferential surface is a mesh. The mesh of the drum unit 41 is configured with wire gauze, expanded metal obtained by stretching a metal plate having cuts, punching metal, and the like. The defibrated material MB introduced into the drum unit 41 from an introduction port 42 is divided into a passing material that passes through the opening of the drum unit 41 and a residue that does not pass through the opening, due to the rotation of the drum unit 41. The passing material that has passed through the opening contains fibers or particles smaller than the opening, and the fibers or particles are called a first sorted material. The residue contains fibers, undefibrated pieces, or dams larger than the opening, and the fibers, undefibrated pieces, or dams are called a second sorted material. The first sorted material descends inside the housing unit 43 toward the material molding unit 45. The second sorted material is sent to the defibrated unit 20 through a pipe 8.

Instead of the sorting unit 40, the sheet manufacturing apparatus 100 may include a classifier that separates the first sorted material and the second sorted material. The classifier is, for example, a cyclone classifier, an elbow jet classifier, and an eddy classifier. The classifier may be configured to separate smaller components or components having a low density among the components contained in the first sorted material. For example, from the first sorted material, it is possible to adopt a configuration in which resin particles, colorants, or additives which are pulled off from the fibers by the defibrating unit 20 are separated by the classifier and removed.

**The** material molding unit 45 includes a mesh belt 46, a stretching roller 47, and a suction unit 48. The mesh belt 46 is an endless metal belt, and is stretched over the plurality of stretching rollers 47. The mesh belt 46 circulates around a track configured with the stretching roller 47. A part of the track of the mesh belt 46 is flat under the drum unit 41, and the mesh belt 46 configures a flat surface.

Multiple openings are provided in the mesh belt 46, and components larger than the opening of the mesh belt 46 in the first sorted material that descends from the drum unit 41 to the mesh belt 46 are deposited on the mesh belt 46. Components smaller than the opening of the mesh belt 46 in the first sorted material pass through the opening. The components that pass through the opening of the mesh belt 46 are called a third sorted material.

The suction unit 48 is connected to a first dust collecting unit 27 via a pipe 23. The first dust collecting unit 27 includes a filter (not illustrated) for separating the third sorted material from the airflow, and a first collection blower 28 is installed on the downstream side of the first dust collecting unit 27. The first collection blower 28 suctions the air from the first dust collecting unit 27. Due to a suctioning force of the first collection blower 28, the third sorted material that has passed through the opening of the mesh belt 46 is sent from the suction unit 48 to the first dust collecting unit 27, and is collected by the filter of the first dust collecting unit 27. Since the first sorted material that descends from the drum unit 41 is attracted to the mesh belt 46 by the airflow suctioned by the suction unit 48, there is an effect of promoting the deposition.

The components deposited on the mesh belt 46 are molded in a web shape. The components are called a web-like material W1. In this manner, the material molding unit 45 forms the web-like material W1 from the first sorted material sorted by the sorting unit 40. The web-like material W1 has fibers larger than the opening of the mesh belt 46 among the components contained in the first sorted material as the main components thereof, and is formed in a state of being soft and bulging containing a large amount of air. The web-like material W1 is transported to the rotation body 49 according to the movement of the mesh belt 46.

The rotation body 49 includes a base portion 49a connected to a driving unit (not illustrated), such as a motor and a protrusion portion 49b that protrudes from the base portion 49a, and as the base portion 49a rotates in a direction R, the protrusion portion 49b rotates around the base portion 49a. The protrusion portion 49b has, for example, a plate-like shape. In the example of Fig. 1, four protrusion portions 49b are provided at equivalent intervals on the base portion 49a.

The rotation body 49 is positioned in the end portion of the flat part of the track of the mesh belt 46. In the end portion, since the track of the mesh belt 46 is bent downward, the mesh belt 46 bends downward and moves. Therefore, the web-like material W1 transported by the mesh belt 46 protrudes from the mesh belt 46 and comes into contact with the rotation body 49. The web-like material W1 is disentangled by the collision of the protrusion portion 49b with the web-like material W1 and becomes a small fiber mass. The mass is transported through a pipe 7 positioned below the rotation body 49. Since the web-like material W1 has a soft structure formed by depositing fibers on the mesh belt 46 as described above, the web-like material W1 is easily divided when colliding with the rotation body 49.

The material obtained by dividing the web-like material W1 by the rotation body 49 is called a material MC. The material MC is obtained by removing the third sorted material from the above-described first sorted material, and the main component thereof is fiber.

The position of the rotation body 49 is a position where the protrusion portion 49b can come into contact with the web-like material W1 and is provided at a position where the protrusion portion 49b does not come into contact with the mesh belt 46. It is preferable that the distance between the protrusion portion 49b and the mesh belt 46 at the position where the protrusion portion 49b and the mesh belt 46 are closest to each other, for example, be 0.05 mm or more and 0.5 mm or less.

A pipe 54A connected to the first mixing unit 50A and a pipe 54B connected to the second mixing unit 50B branch and are connected to the downstream side of the pipe 7. The material MC is transported from the pipe 7 together with the airflow in the pipes 54A and 54B.

The first mixing unit 50A (first material supply unit) includes a first additive supply unit 52A and a first mixing blower 56A which are disposed in the pipe 54A. The first additive supply unit 52A supplies an additive to be mixed with the material MC to the pipe 54A. The first mixing blower 56A is a blower that suctions the air of the pipe 54A and includes, for example, a motor (not illustrated), a blade (not illustrated) that is driven by the motor and rotates, and a case (not illustrated) that accommodates the blade therein. The first mixing blower 56A may have a configuration in which the blade and the case are connected to each other. The additive to be supplied by the first additive supply unit 52A and the material MC are mixed with each other when passing through the first mixing blower 56A and become a first mixture MX1 (first material). Further, the first mixing blower 56A may include a mixer that mixes the material MC and the additives with each other in addition to the blade that generates the airflow.

The downstream side of the pipe 54A is connected to the first depositing unit 60A. The first mixture MX1 mixed by the first mixing unit 50A is transported to the first depositing unit 60A by the airflow generated by the first mixing blower 56A.

The second mixing unit 50B (second material supply unit) includes a second additive supply unit 52B and a second mixing blower 56B which are disposed in the pipe 54B. The second additive supply unit 52B supplies an additive to be mixed with the material MC to the pipe 54B. The second mixing blower 56B is a blower that suctions the air in the pipe 54B, and is configured similarly to the first mixing blower 56A. The additive to be supplied by the second additive supply unit 52B and the material MC are mixed with each other when passing through the second mixing blower 56B and become a second mixture MX2 (second material).

The downstream side of the pipe 54B is connected to the second depositing unit 60B. The second mixture MX2 mixed by the second mixing unit 50B is transported to the second depositing unit 60B by the airflow generated by the second mixing blower 56B.

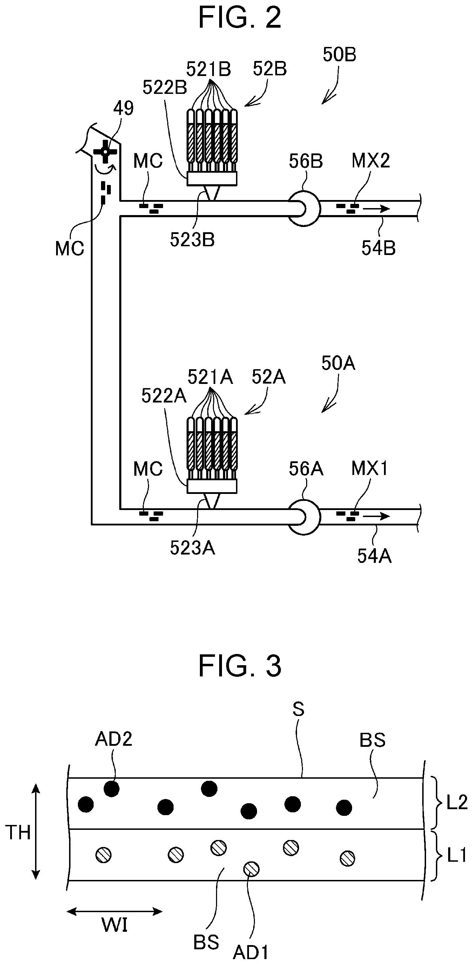

Fig. 2 is a view illustrating the configuration of the first mixing unit 50A and the second mixing unit 50B in detail.

In the first additive supply unit 52A of the first mixing unit 50A, an additive cartridge 521A that stores the additives is set. In this specification, the expressions 'additive' and 'additives' are used interchangeably, and any reference to an additive in the singular should be construed to include the possibility of there being two or more additives. The same applies with the expressions 'additive cartridge' and 'additive cartridges'. The additive cartridge 521A may be attachable to and detachable from the first additive supply unit 52A. The first additive supply unit 52A includes an additive extracting unit 522A for extracting the additive from the additive cartridge 521A and an additive charging unit 523A for discharging the additive extracted by the additive extracting unit 522A to a pipe 54. The additive extracting unit 522A includes a feeder (not illustrated) for sending out the additive containing fine powder or fine particles from the additive cartridge 521A, and extracts the additive from some or all of the additive cartridges 521A. The additive extracted by the additive extracting unit 522A is sent to the additive charging unit 523A. The additive charging unit 523A accommodates the additive extracted by the additive extracting unit 522A. The additive charging unit 523A includes a shutter (not illustrated) which can be opened and closed at a connecting portion with the pipe 54, and by opening the shutter, the additive extracted by the additive extracting unit 522A is sent out to the pipe 54.

The second additive supply unit 52B of the second mixing unit 50B is configured similarly to the first additive supply unit 52A, and includes an additive extracting unit 522B and an additive charging unit 523B. An additive cartridge 521B is set in the second additive supply unit 52B. Since the configuration and the function of the additive cartridge 521B, the additive extracting unit 522B, and the additive charging unit 523B are similar to those of the additive cartridge 521A, the additive extracting unit 522A, and the additive charging unit 523A, respectively, the description thereof will be omitted. In this specification, each additive supply unit may also be conceptualized as supplying one additive comprising one or more components or one or more additives. The component(s) or additive(s) respectively may have one or more ingredients.

The additive to be supplied from at least one of the first additive supply unit 52A and the second additive supply unit 52B contains a resin (binder) for binding a plurality of fibers to each other. The resin contained in the additive is melted by being heated to a temperature equal to or higher than a glass transition point in the manufacturing unit 102 and binds the fibers of the material MC to each other. The resin is, for example, a thermoplastic resin or a thermosetting resin. Specific examples thereof include AS resin, ABS resin, polypropylene, polyethylene, polyvinyl chloride, polystyrene, acrylic resin, polyester resin, polyethylene terephthalate, polyphenylene ether, polybutylene terephthalate, nylon, polyamide, polycarbonate, polyacetal, polyphenylene sulfide, polyether ether ketone, and the like. As the additive, a plurality of types of resins may be mixed with each other and used.

The additive to be supplied from the first additive supply unit 52A and the second additive supply unit 52B which may be different to or the same as the above-described additive may contain a component other than the resin for binding the plurality of fibers to each other. For example, in accordance with the type of the sheet S to be manufactured, a coloring agent for coloring the fibers, a coagulation preventing agent for suppressing coagulation of fibers or coagulation of resins, a flame retardant for making fibers and the like unlikely to burn, and the like, may be included. Further, the additive may be in a form of fiber or powder.

The first additive supply unit 52A and the second additive supply unit 52B can be individually and independently controlled by the control device 110. Therefore, the amount and components of the additive added by the first additive supply unit 52A and the amount and components of the additives added by the second additive supply unit 52B can be individually set. Further, the additive accommodated in the additive cartridge 521A and the additive accommodated in the additive cartridge 521B may also be different components.

In addition, the additive to be supplied by the first additive supply unit 52A and the second additive supply unit 52B may be or include a functional material having a function appropriate for a specific use in addition to the above-described resin, the coloring agent, and the flame retardant. Examples of the functional material include a deodorant, a pest repellent, a water absorbing agent, and the like, which will be described later in detail.

Both the first mixture MX1 mixed by the first additive supply unit 52A and the second mixture MX2 mixed by the second additive supply unit 52B contain the material MC, but may contain other different components or may contain common components.

The web forming unit 70 includes a first depositing unit 60A, a second depositing unit 60B, a mesh belt 72, and a stretching roller 74. In addition, a pipe 66, a second dust collecting unit 67, a second collection blower 68, and a suction mechanism 76 are provided.

The first depositing unit 60A includes a first drum unit 61A and a first housing unit 63A. Further, the first depositing unit 60A has a first introduction port 62A for introducing the first mixture MX1 into the first drum unit 61A. The first drum unit 61A is, for example, a cylindrical structure configured similarly to the drum unit 41, rotates by the power of a motor (not illustrated) similarly to the drum unit 41, and functions as a sieve. The first drum unit 61A (dispersing unit) has an opening, disperses the first mixture MX1 disentangled by the rotation of the first drum unit 61A in the air, and makes the first mixture MX1 descend from the opening.

In addition, the second depositing unit 60B includes a second drum unit 61B and a second housing unit 63B. The second depositing unit 60B has a second introduction port 62B for introducing the second mixture MX2 into the second drum unit 61B. The second drum unit 61B is configured similarly to the first drum unit 61A, rotates by the power of a motor (not illustrated), and functions as a sieve. The second drum unit 61B has an opening, disperses the second mixture MX2 disentangled by the rotation of the second drum unit 61B in the air, and makes the second mixture MX2 descend from the opening.

Below the first depositing unit 60A and the second depositing unit 60B, a mesh belt 72 (deposition surface) is disposed. The mesh belt 72 is configured with an endless metal belt similar to the mesh belt 46, and is stretched over the plurality of stretching rollers 74. The mesh belt 72 circulates around a track configured with the stretching rollers 74. A part of the track of the mesh belt 72 has a flat portion below the first drum unit 61A and below the second drum unit 61B, and the mesh belt 72 configures a flat surface in the flat portion.

The mesh belt 72 has multiple openings. In the mixture MX1 that descends from the first drum unit 61A, a component larger than the opening of the mesh belt 72 is deposited on the mesh belt 72. In addition, a component smaller than the opening of the mesh belt 72 in the mixture MX1 passes through the opening below the first drum unit 61A.

The suction mechanism 76 is disposed below the first drum unit 61A. The suction mechanism 76 suctions the air from the side opposite to the first drum unit 61A with respect to the mesh belt 72, and due to the suction force, the airflow that flows through the opening of the mesh belt 72 from the first drum unit 61A side to the opposite side is generated.

The suction mechanism 76 is connected to the pipe 66. The pipe 66 is connected to the second collection blower 68 via the second dust collecting unit 67. The second dust collecting unit 67 includes a filter (not illustrated) that collects particles or fibers that have passed through the mesh belt 72. The second collection blower 68 is a blower that suctions the air through the pipe 66. The suction mechanism 76 suctions the air from below the mesh belt 72 by the suction force of the second collection blower 68 and collects the particles or fibers contained in the suctioned air by the second dust collecting unit 67. The airflow suctioned by the second collection blower 68 has an effect of attracting the mixture MX1 that descends from the first drum unit 61A to the mesh belt 72 and promoting the deposition. In addition, the suctioned airflow of the second collection blower 68 forms a downflow in a path where the mixture MX1 falls from the first drum unit 61A, and it is also possible to expect an effect of preventing the fibers from being intertwined while falling. The mixture MX1 deposited on the mesh belt 72 has a web shape in the flat portion of the mesh belt 72 and configures a first web W2. The suction mechanism 76 and the second collection blower 68 configure an airflow generation unit.

Further, the density of the first web W2 can be adjusted by the suction force of the second collection blower 68. Specifically, the suction force of the second collection blower 68 has an effect of increasing the density of the first web W2 compared to a case where the second collection blower 68 does not suction the air.

The mesh belt 72 transports the first web W2 from below the first drum unit 61A in the transport direction indicated by a symbol F in the drawing and reaches below the second drum unit 61B. Below the second drum unit 61B, the second mixture MX2 that descends due to the operation of the second drum unit 61B is deposited on the first web W2. Accordingly, a second web W3 is formed on the mesh belt 72.

In the embodiment, the suction mechanism 76 is not disposed below the second drum unit 61B. In other words, in the second depositing unit 60B, the mixture MX2 that has descended by the gravity from the second drum unit 61B is deposited on the first web W2.

In a transport path of the mesh belt 72, a humidity control unit 78 is provided on the downstream side of the second depositing unit 60B. The humidity control unit 78 is a mist type humidifier that supplies water to the mesh belt 72 in a form of mist. The humidity control unit 78 includes, for example, a tank for storing the water and an ultrasonic vibrator for making the water mist. Since a moisture content of the second web W3 is adjusted by the mist to be supplied by the humidity control unit 78, it is possible to expect an effect of suppressing adsorption or the like of the fibers onto the mesh belt 72 due to static electricity.

The second web W3 that has passed through the humidity control unit 78 is transported to the processing unit 80. The processing unit 80 performs processing including heating with respect to the second web W3. The processing unit 80 of the embodiment includes a pressurizing unit 82 that pressurizes the second web W3 and a heating unit 89 that heats the second web W3 pressurized by the pressurizing unit 82.

The pressurizing unit 82 is configured with a pair of calendar rollers 85 and 85. The pressurizing unit 82 is connected to a press mechanism (not illustrated) for applying a nip pressure to the calendar rollers 85 and 85 by a hydraulic pressure and a driving unit (not illustrated), such as a motor for rotating the calendar rollers 85 and 85 toward the heating unit 89. The pressurizing unit 82 pressurizes the second web W3 with a predetermined nip pressure by the calendar rollers 85 and 85, and transports the second web W3 toward the heating unit 89.

The pressurizing unit 82 may strongly pressurize the second web W3 and increase the density, but without increasing the density of the second web W3, the weak pressure may be applied to the second web W3. In this way, the pressurizing unit 82 can adjust the thickness of the second web W3 or adjust the density of the second web W3. In other words, without extremely changing the hardness and/or elasticity of the second web W3 before and after passing through the pressurizing unit 82, it is possible to change the density or thickness of the second web W3.

The heating unit 89 has a furnace 891 that accommodates the upper portion and preferably the lower portion of the transport path of the second web W3, and a heat source 892 accommodated in the furnace 891. The second web W3 passes through the inside of the furnace 891 between the pressurizing unit 82 and the cutting unit 90.

The heat source 892 is an infrared heater that emits infrared rays for heating, or a heater having a heating element. As a heating element, a resistance heating element can be used, and for example, a nichrome wire heater or a ceramic heater can be adopted. The heating unit 89 heats the second web W3 in the furnace 891 by radiant heating by the infrared rays emitted from the heat source 892 or transfer heating by raising the temperature of the internal space of the furnace 891 by the heat source 892.

In addition, the furnace 891 may be configured to perform induction heating, dielectric heating, or heating of the second web W3 by a heat pump type heater. Further, without providing the furnace 891, the second web W3 may be heated by the heat source 892 disposed to be close to the transport path of the second web W3.

In addition, in the furnace 891, another heat source may be provided below the second web W3, that is, on a side opposite to the heat source 892 nipping the second web W3. The configuration is effective in a case where an infrared heater or a heating element heater is used as a heat source, and there is an advantage that the second web W3 can be heated from both surfaces.

The heating unit 89 is a non-pressurizing type heating unit that heats the second web W3 without pressurizing the second web W3. In another expression, a heat source 892 that is not in contact with the second web W3 is provided, and the second web W3 is heated in a non-contact manner.

The second web W3 is heated to a temperature that exceeds the glass transition point of the resin contained in the additive by the heating unit 89. Accordingly, the resin is melted, and the fibers derived from the material MC contained in the second web W3 are bound to each other, and the fibers and the various components contained in the additive are bound to each other. After the heating of the heating unit 89, the second web W3 becomes the sheet S bound in a state where the fibers are not easily disentangled.

The cutting unit 90 cuts the sheet S processed by the processing unit 80. The cutting unit 90 includes a first cutting unit 92 that cuts the sheet S in a direction intersecting the transport direction F, and a second cutting unit 94 that cuts the sheet S in a direction parallel to the transport direction F. The cutting unit 90 cuts the length and the width of the sheet S to a predetermined size and forms a single-cut sheet S. The sheet S cut by the cutting unit 90 is accommodated in a discharge unit 96. The discharge unit 96 includes a tray or a stacker for accommodating the manufactured sheet therein, and the user can take out and use the sheet S discharged onto the tray.

The control device 110 controls each portion of the sheet manufacturing apparatus 100 and executes the manufacturing of the sheet S. For example, the control device 110 executes a starting sequence when starting the manufacturing of the sheet S. In the starting sequence, each portion of the sheet manufacturing apparatus 100 is sequentially started. The control device 110 executes a control of hydraulic pressure of the pressurizing unit 82, a control of disconnection timing in the cutting unit 90, and the like. Further, the control device 110 controls the start and stop of each of the blowers including the first collection blower 28, the first mixing blower 56A, the second mixing blower 56B, and the second collection blower 68. In addition, the control device 110 controls humidification by the humidity control unit 78. In addition, as described above, the control device 110 drives the first additive supply unit 52A and the second additive supply unit 52B, respectively, and adjusts the type and addition amount of the additives.

In the sheet manufacturing apparatus 100, the defibration processing unit 101 and the manufacturing unit 102 may be configured integrally or may be configured separately.

For example, after manufacturing the web-like material W1 by the defibration processing unit 101, the web-like material W1 can also be extracted from the sheet manufacturing apparatus 100 and stored without being transferred to the rotation body 49. Further, the manufactured product may be enclosed in a predetermined package and may be in a form that can be transported and traded. The manufacturing unit 102 can be configured to be separated from the defibration processing unit 101, and in this case, the web-like material W1 may be configured to be supplied to each of the first mixing unit 50A and the second mixing unit 50B.

### 1-2. Manufacturing Example of Sheet

Fig. 3 is a sectional view of the sheet S manufactured by the sheet manufacturing apparatus 100.

Fig. 3 illustrates an example in which one different type of additive is added by the first additive supply unit 52A and the second additive supply unit 52B, respectively. In the sectional view of Fig. 3, the thickness direction of the sheet S is indicated by a symbol TH and the width direction of the sheet S is indicated by a symbol WI. The main component that configures the sheet S is a base BS. The base BS is configured by binding the fibers contained in the raw material MA to each other.

The sheet S has a multilayer structure having a first layer L1 configured with a first mixture MX1 and a second layer L2 configured with a second mixture MX2. In addition to the base BS, the first layer L1 contains additive particles AD1 as a component added by the first additive supply unit 52A. In addition to the base BS, the second layer L2 contains additive particles AD2 configured with a component added by the second additive supply unit 52B. The additive particles AD1 and AD2 may be particles which are melted or modified as the additive added by the first additive supply unit 52A and the second additive supply unit 52B is heated by the heating unit 89. In addition to the additive particles AD1, the first layer L1 may contain the additives, and the second layer L2 is also the same. In other words, each of the first layer L1 and the second layer L2 may contain a plurality of additives, or may contain common components as additive particles.

In the sheet manufacturing apparatus 100, it is possible to make the density of the first layer L1 and the density of the second layer L2 different from each other. The density of the first layer L1 reflects the density of the first web W2 and the pressure with which the pressurizing unit 82 pressurizes the second web W3. The density of the first web W2 is influenced by the suction airflow suctioned by the suction mechanism 76 below the first drum unit 61A, and the density of the first web W2 increases as the airflow of the suction mechanism 76 increases. The strength of the airflow of the suction mechanism 76 may be assessed using either the wind speed or the air volume as indicators.

Meanwhile, the density of the second layer L2 reflects the pressure with which the pressurizing unit 82 pressurizes the second web W3.

Therefore, in the sheet S, it is possible to make the densities of the first layer L1 and the second layer L2 different from each other, and the sheet manufacturing apparatus 100 can manufacture the sheets S having different densities in the thickness direction TH.

Further, the sheet manufacturing apparatus 100 can individually control the thicknesses of the first layer L1 and the second layer L2 of the sheet S. Specifically, the thickness of the first layer L1 is determined by the thickness of the first mixture MX1 deposited on the mesh belt 72 by the first depositing unit 60A and the pressure by which the pressurizing unit 82 pressurizes the second web W3. The thickness of the first mixture MX1 deposited on the mesh belt 72 is determined by the amount of the first mixture MX1 that descends by the first drum unit 61A per unit time, and the control device 110 can perform the control by adjusting a rotational speed of the first drum unit 61A. As the rotational speed of the first drum unit 61A increases, the thickness of the first mixture MX1 deposited on the mesh belt 72 increases. In addition, the thickness of the second mixture MX2 deposited by the second depositing unit 60B is determined by the amount of the second mixture MX2 that descends by the second drum unit 61B per unit time, and the control device 110 can perform the control by adjusting the rotational speed of the second drum unit 61B. As the rotational speed of the second drum unit 61B increases, the thickness of the second mixture MX2 deposited on the mesh belt 72 increases.

In addition, it is also possible to adjust a basis weight of the sheet S by adjusting the thicknesses of the first web W2 and the second web W3.

In this manner, the sheet manufacturing apparatus 100 has a plurality of layers in the thickness direction TH, and it is possible to manufacture the sheets S having different densities, thicknesses, and basis weights for each layer.

### 1-3. Application Example of Sheet

Regarding the sheet S manufactured by the sheet manufacturing apparatus 100, the sheet S having the functionality specialized for a specific use can be manufactured by appropriately selecting the components of the additive. Here, a specific example of the combination of the use of the sheet S and the additive added by the additive supply unit 52 will be presented.

In various specific examples described below, as the additive to be added by the first additive supply unit 52A and the second additive supply unit 52B, a binder for bonding the fibers to each other and a functional material that exhibits a function are used. Bonding materials and functional materials are in a shape of small pieces, particles, powder, or the like. The bonding material is the above-described resin which bonds the fibers originated from the raw material MA to each other by being heated by the heating unit 89. In the following examples, the bonding material is added by the first additive supply unit 52A and the functional material is added by the second additive supply unit 52B.

It is also possible to color the first web W2 by adding the coloring agent to the material MC together with the bonding material by the first additive supply unit 52A. In other words, the first additive supply unit 52A may supply the additives from the additive cartridge 521A in which the bonding material is accommodated and the additive cartridge 521A in which the coloring agent is accommodated. In this case, for example, by changing the type of the functional material or the color of the sheet S for each use of the sheet S, a user who uses the sheet S can identify the type of the sheet S by color. In addition, the particles of the bonding material added by the first additive supply unit 52A may contain the coloring agent. In this case, it is possible to reduce the number of necessary additive cartridges 521A.

In addition, the functional material and the coloring agent may be added by the second additive supply unit 52B, and specifically, the additive may be supplied from the additive cartridge 521B in which the functional material is accommodated and the additive cartridge 521B in which the coloring agent is accommodated. In this case, the user who uses the sheet S can identify the type of the sheet S by color. In addition, by combining the colors of the first layer L1 and the second layer L2 with each other, it is possible to indicate the direction of the surface, that is, the front and rear sides, in a case where the sheet S is used. In addition, the particles of the functional material added by the second additive supply unit 52B may contain the coloring agent. In this case, it is possible to reduce the number of necessary additive cartridges 521B.

In addition, the functional material is molded into small pieces, particles, or powders by being mixed or adsorbed to a base material containing wood flour, a wood flour, a mineral powder, such as talc, and other organic or inorganic powders. In this case, it is possible to make an extremely small amount of functional material contained in the sheet S with an accurate content, and even when the functional material is liquid, it is possible to add the functional material by the second additive supply unit 52B (or 52A). Further, a mixture obtained by mixing the plurality of types of functional materials with each other may be added by the second additive supply unit 52B (or 52A).

In each of the following examples, as the first additive supply unit 52A adds the bonding material, the first layer L1 contains the fibers and the bonding material and the second layer L2 contains the functional material. In the example of Fig. 3, the additive particle AD1 is the bonding material and the additive particle AD2 is a particle of the functional material. The first layer L1 can have a higher density than that of the second layer L2. Since the first layer L1 contains the fibers and the bonding material, the strength of the sheet S can be ensured. In addition, since the second layer L2 having a lower density than that of the first layer L1 contains the functional material, it is easy to bring the functional material and the external air or moisture into contact with each other. Therefore, it is possible to realize a state where the functional material easily exhibits the function. (1) Plant Cultivation Sheet

The sheet manufacturing apparatus 100 can manufacture the sheet S that can be used as a medium for sowing seeds of plants. In the example, as the functional material, a material used for plant cultivation is added. Specifically, as the functional material, a fertilizer and a soil conditioner can be used. As the fertilizer, nitrogenous fertilizers, such as ammonium sulfate, ammonium chloride, ammonium nitrate and the like, can be employed. In addition, particles of phosphatic fertilizer, such as superphosphate, concentrated superphosphate, or fused phosphate, may be used. In addition, particles of potassium fertilizer, such as potassium chloride or potassium nitrate may be used. Further, among the fertilizers, a mixed fertilizer obtained by mixing the plurality of types of fertilizers with each other may be added as a functional material. The soil conditioner is, for example, a pH adjusting agent, and specifically, for example, organic lime, vegetable ash, quicklime, hydrated lime, and the like can be adopted.

In addition, as the functional material, a water retaining material that retains moisture can also be used, and for example, particles of super absorbent polymer (SAP) can be used as an additive. In addition, the particles of porous ceramics may be used as a water retaining material. The water retaining material may be added as an additive by the first additive supply unit 52A or the second additive supply unit 52B.

The functional materials can be dispersed and disposed inside the sheet S similar to the additive particles AD2 of Fig. 3 by molding the functional materials into small pieces, particles, or powder and by adding the functional materials by the second additive supply unit 52B. Further, a mixture obtained by mixing the plurality of types of functional materials with each other may be molded into small pieces, particles, or powder, and may be added by the second additive supply unit 52B. In addition, the plurality of types of functional materials may be respectively added by the second additive supply unit 52B.

As the sheet S for plant cultivation is buried in soil, for example, it is possible to give the fertilizer or the pH adjuster to the soil. Further, the sheet S can be used as a medium capable of growing plants instead of soil. When using the sheet S, it is possible to easily cultivate plants while saving labor for soil improvement or soil management. In addition, by cutting the sheet S into a long shape by the cutting unit 90, it is possible to use the sheet S for larger-scale plant cultivation.

In this case, the bonding material may be added by the first additive supply unit 52A, or the coloring agent for coloring the sheet S in a color indicating that the sheet S is a sheet for plant cultivation and the bonding material may be added. In the sheet S manufactured in this manner, the first layer L1 contains the fibers and the bonding material, and the second layer L2 contains a fertilizer or a fertilizer and SAP. In other words, the additive particle AD1 in Fig. 3 is the bonding material and the additive particle AD2 is a fertilizer. In addition, SAP particles may be contained in the second layer L2 separately from the additive particles AD2. According to the configuration, it is possible to ensure the strength of the sheet S as described above, and it is possible to expect an effect that the fertilizer is easily eluted from the second layer L2. In addition, in a case where the SAP is contained in the second layer L2, there is an advantage that, even when the SAP contains the moisture and expands, the sheet S is unlikely to be collapsed. (2) Insect Repellent Sheet

By using a pest repellent or an insecticide as a functional material, the sheet S can be made as an insect repellent sheet. As the repellent or the insecticide, in addition to known chemically synthesized medicine, natural materials, such as camphor tree flour or hinoki wood flour can be used. The repellent or the insecticide can also be mixed and used.

In the example of Fig. 3, the additive particle AD1 is a particle containing a bonding material resin and the additive particle AD2 is a particle containing a repellent insecticide. According to the configuration, it is possible to ensure the strength of the sheet S as described above, and it is possible to expect an effect that components of the repellent or the insecticide are likely to be volatilized or eluted from the second layer L2. In this case, by the first additive supply unit 52A, the coloring agent for coloring the sheet S in a color indicating that the sheet S is an insect repellent sheet and the bonding material may be added. (3) Deodorizing Sheet

As the functional material, by using a deodorant functional material, such as a deodorant, an odor adsorbent, or an odor decomposer, the sheet S can be made as a deodorizing sheet. As the odor adsorbent, for example, particles of activated carbon or porous ceramics can be used. As the odor decomposer, for example, titanium oxide can be used. The repellent or the odor adsorbent can also be mixed and used.

In the example of Fig. 3, the additive particle AD1 is a particle containing a bonding material resin and the additive particle AD2 is a particle containing a deodorant functional material. According to the configuration, it is possible to ensure the strength of the sheet S as described above, and since the deodorizing functional material of the second layer L2 is likely to touch the external air, it is possible to expect an effect that it is easy to exhibit a deodorant function. In this case, by the first additive supply unit 52A, the coloring agent for coloring the sheet S in a color indicating that the sheet S is a deodorizing sheet and the bonding material may be added. (4) Dehumidifying Sheet

By using particles containing a hygroscopic material as a functional material, the sheet S can be made as a dehumidifying sheet. As the hygroscopic material, for example, particles of a desiccant, such as silica gel, can be used. It is also possible to use a mixture of a plurality of types of hygroscopic materials. According to the configuration, it is possible to ensure the strength of the sheet S as described above, and since the hygroscopic material of the second layer L2 is likely to touch the external air, it is possible to expect an effect that it is easy to exhibit a hygroscopic function. In this case, by the first additive supply unit 52A, the coloring agent for coloring the sheet S in a color indicating that the sheet S is a hygroscopic sheet and the bonding material may be added. (5) Heat Insulating and Generating Sheet

By using particles containing a material that exhibits heat insulating properties or heat generating properties as a functional material, the sheet S can be made as a heat insulating and generating sheet. For the type of functional material, for example, powder or particles containing capsaicin (for example, red pepper powder) can be used. To the particles, particles of black silica may be mixed as an auxiliary material. In addition, it is also possible to use a mixture of the plurality of types of functional materials. According to the configuration, it is possible to ensure the strength of the sheet S as described above, and since the functional material of the second layer L2 is likely to touch the external air and the components are likely to be volatilized, it is possible to expect an effect that it is easy to exhibit heat insulating properties or heat generating properties. In this case, by the first additive supply unit 52A, the coloring agent for coloring the sheet S in a color indicating that the sheet S is a heat insulating and generating sheet and the bonding material may be added. (6) Moisturizing Sheet

By using particles containing a functional material having water containing properties as a functional material, the sheet S can be made as a moisturising sheet. As the above-described functional material, for example, the SAP particles can be used. It is also possible to use a mixture of the plurality of types of functional materials. According to the configuration, it is possible to ensure the strength of the sheet S as described above, and since the functional material of the second layer L2 is likely to touch the external air, it is possible to expect an effect that it is easy to exhibit moisturizing properties. In this case, by the first additive supply unit 52A, the coloring agent for coloring the sheet S in a color indicating that the sheet S is a moisturizing sheet and the bonding material may be added. (7) Aromatic Sheet

By using an aromatic material as a functional material, the sheet S can be made as an aromatic sheet. As the aromatic material, in addition to known perfumes, natural materials, such as hinoki powder, can be used. It is also possible to use a mixture of the plurality of types of aromatic materials. According to the configuration, it is possible to ensure the strength of the sheet S as described above, and since the functional material of the second layer L2 is likely to touch the external air and the components are likely to be volatilized, it is possible to expect an effect that it is easy to exhibit aroma. In this case, by the first additive supply unit 52A, the coloring agent for coloring the sheet S in a color indicating that the sheet S is an aromatic sheet and the bonding material may be added. Further, the sheet S may be colored in a color indicating the type of aroma.

As described above, the sheet manufacturing apparatus 100 according to the first embodiment to which the invention is applied includes the web forming unit 70 that forms the second web W3 as the web for processing. The web forming unit 70 includes: the first depositing unit 60A that deposits the first mixture MX1 containing fibers and forms the first web W2; and the second depositing unit 60B that deposits the second mixture MX2 containing fibers on the first web W2 and forms the second web W3. The sheet manufacturing apparatus 100 includes the processing unit 80 for manufacturing the sheet by performing the processing including the heating with respect to the second web W3 formed by the web forming unit 70.

The sheet manufacturing apparatus 100 corresponds to an apparatus for realizing a sheet manufacturing method of the invention. The processing of the web forming unit 70 corresponds to a web forming process, the processing of the first depositing unit 60A corresponds to a first depositing process, and the processing of the second depositing unit 60B corresponds a second depositing process of forming the second web W3 as the web for processing. The processing of the processing unit 80 corresponds to a processing process.

According to the sheet manufacturing apparatus of the invention and the sheet manufacturing apparatus 100 to which the sheet manufacturing method is applied, the deposited state of the first depositing unit 60A and the second depositing unit 60B is reflected in the density in the thickness direction of the sheet S to be manufactured. Therefore, it is possible to adjust the density and the like in the thickness direction of the sheet S.

Further, the first depositing unit 60A has the first drum unit 61A that disperses the first mixture MX1. The web forming unit 70 includes the mesh belt 72 which has an opening and on which the first mixture MX1 dispersed by the first drum unit 61A is deposited. In addition, the suction mechanism 76 and the second collection blower 68 that serve as airflow generation units for generating the airflow that passes through the opening of the mesh belt 72 from the first drum unit 61A side to the opposite side are provided.

Accordingly, in the first depositing unit 60A for depositing the first mixture MX1, it is possible to deposit the first mixture MX1 at a higher density by the airflow that passes through the mesh belt 72. The density can be higher than the density at which the second depositing unit 60B deposits the second mixture MX2. Therefore, in the sheet S having a multilayer structure including the layer of the first mixture MX1 and the layer of the second mixture MX2, it is possible to change the density in the thickness direction TH.

In addition, the sheet manufacturing apparatus 100 includes the first mixing unit 50A that supplies the first mixture MX1 containing a first component and the second mixing unit 50B that supplies the second mixture MX2 including a second component different from the first component. With the configuration, each of the plurality of layers that configure the sheet S can be configured with different components.

Further, the processing unit 80 includes a heating unit 89 for heating the second web W3 in a non-pressurized state. Therefore, it is possible to manufacture the sheet S by dissolving the bonding material without substantially changing the densities of the first web W2 and the second web W3 of the mesh belt 72. Therefore, a difference in the density of each layer in the second web W3 can be left, and the sheet S having different densities in the thickness direction TH can be manufactured.

In addition, the processing unit 80 includes the pressurizing unit 82 that pressurizes the second web W3, and the heating unit 89 heats the second web W3 pressurized by the pressurizing unit 82. The density of the second web W3 can be adjusted by the pressurizing unit 82. Therefore, it is possible to adjust the density of the sheet S to a state different from the density of the first web W2 or the second web W3.

### 2. Second Embodiment

### 2-1. Overall Configuration of Sheet Manufacturing Apparatus

Hereinafter, a second embodiment to which the invention is applied will be described.

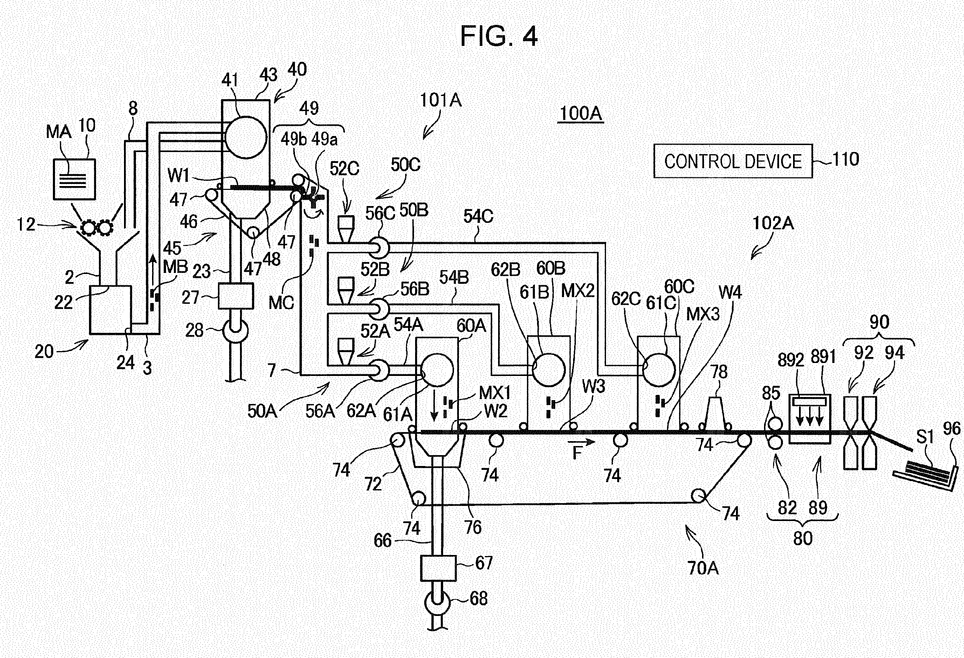

Fig. 4 is a schematic configuration view of a sheet manufacturing apparatus 100A according to the second embodiment. The sheet manufacturing apparatus 100A includes a defibration processing unit 101A instead of the defibration processing unit 101 in the sheet manufacturing apparatus 100 (Fig. 1), and includes a manufacturing unit 102A instead of the manufacturing unit 102.

In the description of the second embodiment, the configurations common to those of the first embodiment will be given the same reference numerals, and the description thereof will be omitted.

As illustrated in Fig. 4, the manufacturing unit 102A includes a web forming unit 70A including a first depositing unit 60A, a second depositing unit 60B, and a third depositing unit 60C. The web forming unit 70A is configured similarly to the web forming unit 70 (Fig. 1), except that the third depositing unit 60C is provided.

Further, in the defibration processing unit 101A, three pipes, that is, the pipes 54A, 54B, and 54C, branch and are connected to the pipe 7. Similar to the sheet manufacturing apparatus 100, the first mixing unit 50A and the second mixing unit 50B are disposed respectively in the pipes 54A and 54B. The pipes 54A and 54B are connected to the first depositing unit 60A and the second depositing unit 60B, respectively.

In addition, similar to the pipes 54A and 54B, the pipe 54C is a pipe for transporting the material MC, and the third mixing unit 50C is provided in the pipe 54C.

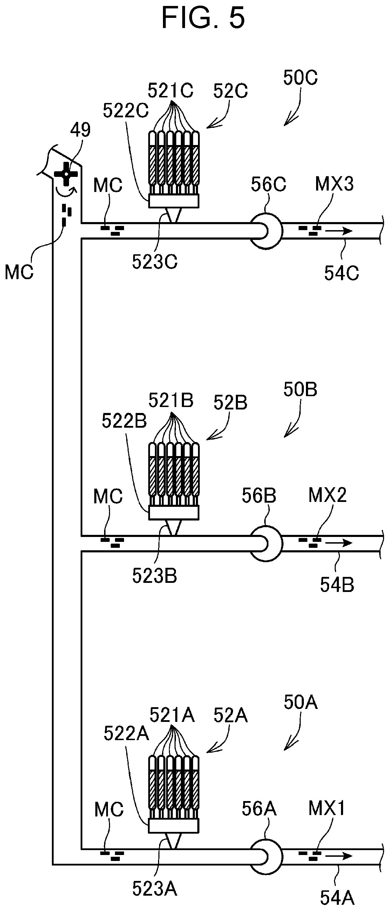

Fig. 5 is a view illustrating the configuration of the first mixing unit 50A, the second mixing unit 50B, and the third mixing unit 50C in detail.

In the third additive supply unit 52C of the third mixing unit 50C, at least one additive cartridge 521C that stores the additives is set. The additive cartridge 521C may be attachable to and detachable from the third additive supply unit 52C. The third additive supply unit 52C includes an additive extracting unit 522C that extracts the additive from the additive cartridge 521C and an additive charging unit 523C that discharges the additive extracted by the additive extracting unit 522C to the pipe 54. The additive extracting unit 522C includes a feeder (not illustrated) for sending out the additive containing fine powder or fine particles from the additive cartridge 521C, and extracts the additive from some or all of the additive cartridges 521C. The additive extracted by the additive extracting unit 522C is sent to the additive charging unit 523C. The additive charging unit 523C accommodates the additive extracted by the additive extracting unit 522C. The additive charging unit 523C includes a shutter (not illustrated) which can be opened and closed in a connecting portion with the pipe 54, and by opening the shutter, the additive extracted by the additive extracting unit 522C is sent out to the pipe 54.

The additive(s) supplied by the third additive supply unit 52C is/are similar to that/those supplied by the first additive supply unit 52A and the second additive supply unit 52B. In other words, the additives may be fibers, resins, coloring agents, flame retardants, and other functional materials.

Similar to the first additive supply unit 52A and the second additive supply unit 52B, the third additive supply unit 52C can be independently controlled by the control device 110. Therefore, the amount and components of the additive added by the third additive supply unit 52C can be controlled by the control device 110 separately from the first additive supply unit 52A and the second additive supply unit 52B. Further, the additive accommodated in the additive cartridge 521C and the additives accommodated in the additive cartridges 521A and 521B may also be different components.

The additive added by the third additive supply unit 52C is mixed with the material MC by the third mixing blower 56C and becomes a third mixture MX3 (third material). The third mixture MX3 may be different from or may be common to the first mixture MX1 and/or the second mixture MX2. The third mixing blower 56C is configured similarly to the first mixing blower 56A and the second mixing blower 56B. The third mixture MX3 is sent to the third depositing unit 60C by the airflow generated by the third mixing blower 56C.

Returning to Fig. 4, the third depositing unit 60C is disposed on the downstream side of the second depositing unit 60B in the transport path of the mesh belt 72. Further, the humidity control unit 78 is disposed on the downstream side of the third depositing unit 60C.

Similar to the first depositing unit 60A and the second depositing unit 60B, the third depositing unit 60C includes a third drum unit 61C positioned above the mesh belt 72 and a third introduction port 62C for introducing the third mixture MX3 into the third drum unit 61C. The third drum unit 61C functions as a sieve, disperses the third mixture MX3 introduced into the third introduction port 62C in the air, and makes the mixture MX3 descend toward the mesh belt 72. The third depositing unit 60C does not include the suction mechanism 76. Therefore, the third mixture MX3 dispersed by the third drum unit 61C falls toward the mesh belt 72 due to the gravity.

In the transport path of the mesh belt 72, the first depositing unit 60A deposits the first mixture MX1 on the mesh belt 72 and forms the first web W2. The second depositing unit 60B deposits the second mixture MX2 on the first web W2 and forms the second web W3. In addition, the third depositing unit 60C deposits the third mixture MX3 on the second web W3 and forms a third web W4.

The third web W4 is transported to the processing unit 80 after the humidity is controlled by the humidity control unit 78. The processing unit 80 adjusts the density of the third web W4 by the pressurizing unit 82 and heats the third web W4 by the heating unit 89. The third web W4 becomes a sheet S1 by being heated by the heating unit 89.

### 2-2. Manufacturing Example of Sheet

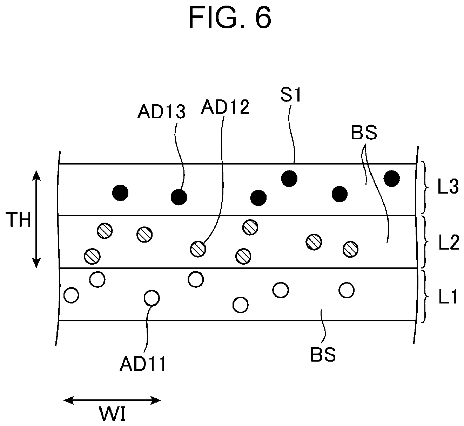

Fig. 6 is a sectional view of the sheet S1 manufactured by the sheet manufacturing apparatus 100A.

Fig. 6 illustrates an example in which one type of additive is added by the first additive supply unit 52A, the second additive supply unit 52B and the third additive supply unit 52C, respectively. In the sectional view of Fig. 6, the thickness direction of the sheet S1 is indicated by the symbol TH and the width direction of the sheet S1 is indicated by the symbol WI. The main component that configures the sheet S1 is the base BS. The base BS is configured by binding the fibers contained in the raw material MA to each other.

The sheet S1 has a multilayer structure having the first layer L1 configured with the first mixture MX1, the second layer L2 configured with the second mixture MX2, and the third layer L3 configured with the third mixture MX3. The first layer L1 contains the base BS and additive particles AD11, and the second layer L2 contains the base BS and additive particles AD12. The additive particles AD11 and AD12 are additives added by the first additive supply unit 52A and the second additive supply unit 52B, respectively.

The third layer L3 contains the base BS and additive particles AD13 which are an additive added by the third additive supply unit 52C.

In addition, each of the first layer L1, the second layer L2, and the third layer L3 may contain a plurality of additives, or may contain common components as additive particles.

In the sheet manufacturing apparatus 100A, it is possible to make the density of the first layer L1 and the densities of the second layer L2 and the third layer L3 different from each other. As described above, the density of the first layer L1 reflects the density of the first web W2 and the pressure with which the pressurizing unit 82 pressurizes the third web W4. The density of the first web W2 is influenced by the suction airflow suctioned by the suction mechanism 76 below the first drum unit 61A, and the density of the first web W2 increases as the airflow of the suction mechanism 76 increases. The strength of the airflow of the suction mechanism 76 may be considered using either the wind speed or the air volume as indicators. Meanwhile, the density of the second layer L2 and the third layer L3 reflect the pressure with which the pressurizing unit 82 pressurizes the third web W4.

Therefore, in the sheet S1, it is possible to make the density of the first layer L1 and the densities of the second layer L2 and the third layer L3 different from each other, and the sheet manufacturing apparatus 100A can manufacture the sheet S1 having different densities in the thickness direction TH.

Further, the sheet manufacturing apparatus 100A can individually control the thicknesses of the first layer L1, the second layer L2, and the third layer L3. Similar to the first layer L1 and the second layer L2, since the thickness of the third layer L3 is determined by the amount of the third mixture MX3 that descends by the third drum unit 61C per unit time, the control device 110 can perform the control by adjusting the rotational speed of the third drum unit 61C. As the rotational speed of the third drum unit 61C increases, the thickness of the third mixture MX3 deposited on the mesh belt 72 increases.

Further, in the sheet manufacturing apparatus 100A, it is also possible to adjust the basis weight of the sheet S1.

In this manner, the sheet manufacturing apparatus 100A has a plurality of layers in the thickness direction TH, and it is possible to manufacture the sheets S1 having different densities, thicknesses, and basis weights for each layer.

According to the sheet manufacturing apparatus 100A, it is possible to obtain the same effect as that of the sheet manufacturing apparatus 100 of the first embodiment except that the third web W4 corresponds to the processing web.

In addition, the web forming unit 70A includes the third depositing unit 60C that deposits the third mixture MX3 containing fibers on the second web W3, and forms the third web W4 in which the third mixture MX3 is deposited by the third depositing unit 60C. By processing the third web W4 and manufacturing the sheet S1, it is possible to manufacture the sheet S1 having a three-layer configuration and having at least one layer different in density from the other layers.

Since the sheet S1 manufactured by the sheet manufacturing apparatus 100A has a three-layer configuration and is multilayered compared to the sheet S, the sheet manufacturing apparatus 100A is more appropriate for manufacturing the sheet S1 to which the functional material is added. For example, a bonding material may be added to the first layer L1 as the additive particle AD11 and a functional material may be added to the second layer L2 and the third layer L3 as the additive particles AD12 and AD13. Further, a coloring matter and a bonding material may be added as the additive particles AD11 and AD12, and a functional material may be added as the additive particle AD13. In this case, when the coloring matter of the additive particles AD11 and the coloring matter of the additive particles AD12 are made to have different colors, according to the mixing effect, the sheet S1 can be colored to a color different from that of the coloring matter that can be added by the first additive supply unit 52A and the second additive supply unit 52B. Further, in a case where the transparency of the base BS is low, as the sheet S1 is colored such that the first layer L1 and the second layer L2 are colored in different colors, it is possible to expect an effect that the front and rear sides of the sheet S1 can be confirmed at the time of use.

An example of adding a fertilizer as the additive particle AD13 to the third layer L3 in the plant cultivation sheet may be employed. The SAP may be added as the additive particle AD12 to the second layer L2.

For the insect repellent sheet, for example, wood flour of camphor tree may be added as a functional material that exhibits an insect repellent effect to the third layer L3, and a colorless bonding material may be added as the additive particles AD11 and AD12.

In the deodorizing sheet, an example in which a functional material, such as activated carbon, is added as the additive particle AD13 of the third layer L3, a cyan bonding material is added as the additive particle AD12, and a magenta bonding material is added as the additive particle AD11 is employed. In addition, titanium oxide may be added as the additive particle AD13 of the third layer L3.

In the dehumidifying sheet, an example in which a functional material, such as silica, is added as the additive particle AD13, a yellow bonding material is added as the additive particle AD12, and a magenta bonding material is added as the additive particle AD11 is employed.

In the heat insulating and generating sheet, an example in which a functional material, such as black silica, is added as the additive particle AD13, a functional material, such as red pepper powder, is added as the additive particle AD12, and a magenta bonding material is added as the additive particle AD11 is employed.

In the moisturizing sheet, an example in which a functional material, such as SAP, is added as the additive particle AD13, and a magenta bonding material is added as the additive particles AD11 and AD12 is employed.

In the aromatic sheet, an example in which a functional material, such as hinoki wood flour, is added as the additive particle AD13, a magenta bonding material is added as the additive particle AD12, and a yellow bonding material is added as the additive particle AD11 is employed.

### 3. Third Embodiment

Hereinafter, a third embodiment to which the invention is applied will be described.

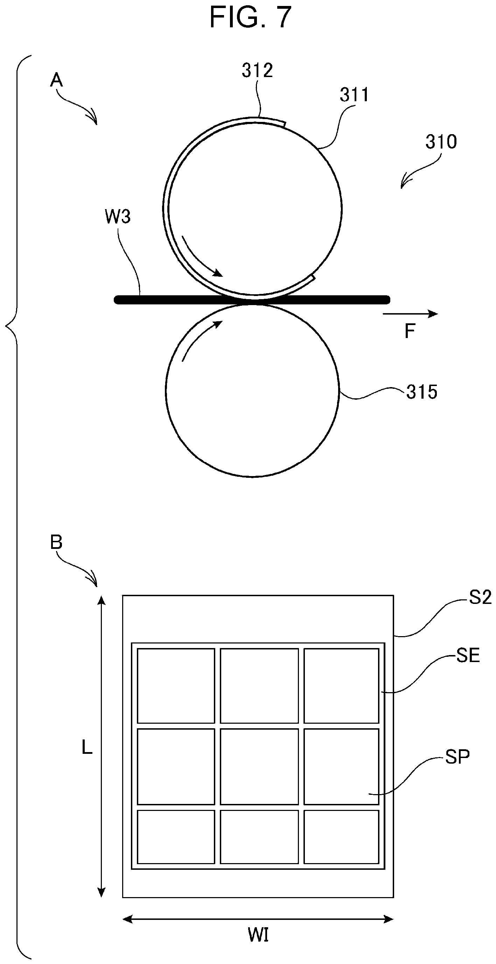

Fig. 7 is an explanatory view illustrating a configuration of a processing roller 310 according to the third embodiment. A sign A of Fig. 7 illustrates a side view illustrating a configuration of the processing roller 310, and a sign B of Fig. 7 illustrates a plan view of a sheet S3 to be manufactured with a configuration including the processing roller 310.

The processing roller 310 (pressing processing unit) is installed instead of the pressurizing unit 82 of the sheet manufacturing apparatus 100 of the first embodiment or the sheet manufacturing apparatus 100A of the second embodiment. The processing roller 310 is installed in the transport path of the web for processing and pressurizes the web for processing. In the following description, a configuration in which the processing roller 310 is applied to the sheet manufacturing apparatus 100 (Fig. 1) will be described. The heating unit 89 and the processing in each of the subsequent processes after the processing of the heating unit 89 are the same as those in the first embodiment.

The processing roller 310 includes a roller 311 and a roller 315 that are provided as a pair. The processing roller 310 nips and transports the second web W3 of the first embodiment between the roller 311 and the roller 315 with a predetermined nip pressure and applies a pressing force to the second web W3. The processing roller 310 includes a pressure applying mechanism (not illustrated) for applying the nip pressure to the roller 311 and the roller 315 and a driving motor (not illustrated) for driving the roller 311 or the roller 315.

Similar to the above-described pressurizing unit 82, the pressing force with which the processing roller 310 presses the second web W3 may be an extent of adjusting the density of the second web W3. Further, the processing roller 310 may be pressurized with a pressure for increasing the density of the second web W3.

On the surface of the roller 311, a projection 312 is formed. In contrast, the roller 315 is a cylindrical roller having a flat circumferential surface.

When the processing roller 310 nips and transports the second web W3, the second web W3 receives the nip pressure from the projection 312. Therefore, the second web W3 partially receives the pressing force, and a part of the second web W3 sinks. Accordingly, unevenness that corresponds to the shape of the projection 312 is formed on the second web W3.

The sign B of Fig. 7 illustrates a plan view of a sheet S2 as an example of the sheet manufactured by using the processing roller 310. A length L in Fig. 7 refers to the size of the sheet S2 in the transport direction F, and a width WI is the same as that in Fig. 3.

In the sheet S2, a lattice-like recess portion SE that extends in the length L direction and the width WI direction is formed. Since the recess portion SE is formed by pressing the second web W3 by the projection 312 and the part other than the recess portion SE is relatively projected, the sheet S2 has unevenness as a whole.

The recess portion SE receives a higher pressure than that at the part other than the recess portion SE with respect to the second web W3. Therefore, in a case where the processing roller 310 applies the pressure to the second web W3 to the extent that high density can be achieved, the first layer L1 and the second layer L2 are joined to each other at high density in the recess portion SE. As being heated by the heating unit 89, the layers of the recess portion SE are reliably joined to each other by the bonding material contained in the second web W3.

On the other hand, the part (for example, the part SP defined by the recess portion SE) other than the recess portion SE is not pressed by the projection 312, but is heated under non-pressure in the heating unit 89. Therefore, the part other than the recess portion SE can maintain the density of the first web W2 and the second web W3 substantially, and can be in a state where flexibility is high.

In this manner, the processing roller 310 partially presses the second web W3 that serves as the web for processing, and gives an uneven shape to the second web W3. The processing roller 310 can form the part SP defined by the recess portion SE on the sheet S2 by partially pressing the second web W3 by the projection 312. In addition, the projection 312 can also form the recess portion SE that serves as a decoration on the sheet S2.

The sheet manufacturing apparatuses 100 and 100A to which the processing roller 310 is applied have an uneven shape, and it is possible to manufacture the sheet S2 having a density difference within the surface. Therefore, it is possible to manufacture the sheet S2 in which the density and the like are different for each layer and the uneven shape due to the density difference is given within the surface.

The processing roller 310 is not limited to an aspect of being installed instead of the pressurizing unit 82 in the sheet manufacturing apparatuses 100 and 100A, but may be additionally installed between the pressurizing unit 82 and the heating unit 89. The lattice pattern is not essential and other patterns are also possible. Similar modifications can be made to the second embodiment.

### 4. Fourth Embodiment

Hereinafter, a fourth embodiment to which the invention is applied will be described.

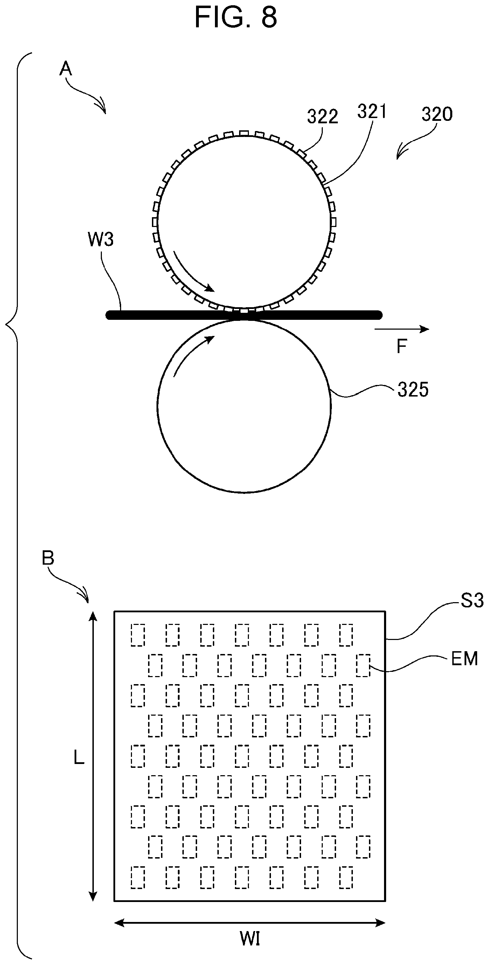

Fig. 8 is an explanatory view illustrating a configuration of a processing roller 320 according to the fourth embodiment. A sign A of Fig. 8 illustrates a side view illustrating a configuration of the processing roller 320, and a sign B of Fig. 8 illustrates a plan view of the sheet S3 to be manufactured with a configuration including the processing roller 320.

The processing roller 320 (pressing processing unit) is installed instead of the pressurizing unit 82 of the sheet manufacturing apparatus 100 of the first embodiment or the sheet manufacturing apparatus 100A of the second embodiment which are described above. The processing roller 320 is installed in the transport path of the web for processing, pressurizes the web for processing, gives an uneven shape to the web for processing, and performs so-called embossing. In the following description, a configuration in which the processing roller 320 is applied to the sheet manufacturing apparatus 100 (Fig. 1) will be described. The second web W3 embossed by the processing roller 320 is heated by the heating unit 89. The heating unit 89 and the processing in each of the subsequent processes after the processing of the heating unit 89 are the same as those in the first embodiment.

The processing roller 320 includes a roller 321 and a roller 325 that are provided as a pair. The processing roller 320 nips and transports the second web W3 between the roller 321 and the roller 325 with a predetermined nip pressure and applies a pressing force to the second web W3. The processing roller 320 includes a pressure applying mechanism (not illustrated) for applying the nip pressure to the roller 321 and the roller 325 and a driving motor (not illustrated) for driving the roller 321 or the roller 325.

Similar to the above-described pressurizing unit 82, the pressing force with which the processing roller 320 presses the second web W3 may be an extent of adjusting the density of the second web W3. Further, the processing roller 320 may be pressurized with a pressure for increasing the density of the second web W3.

On the surface of the roller 321, a projection 322 is formed. In contrast, the roller 325 is a cylindrical roller having a flat circumferential surface.

When the processing roller 320 nips and transports the second web W3, the second web W3 receives the nip pressure from the projection 322. Therefore, the second web W3 partially receives the pressing force, and a part of the second web W3 sinks. Accordingly, unevenness that corresponds to the shape of the projection 322 is formed on the second web W3.

The sign B of Fig. 8 illustrates a plan view of a sheet S3 as an example of the sheet manufactured by using the processing roller 320. The length L and the width WI in Fig. 8 are the same as those in Fig. 7.

In the sheet S3, a recess portion EM that extends in the length L direction and the width WI direction is formed. Since the recess portion EM is a recess portion formed by the so-called embossing, the second web W3 is formed by pressing the second web S3 by the projection 322, and the part other than the recess portion EM is relatively projected, the sheet S3 has unevenness as a whole.

The recess portion EM receives a higher pressure than that at the part other than the recess portion EM with respect to the second web W3. Therefore, in a case where the processing roller 320 applies the pressure to the second web W3 to the extent that high density can be achieved, the first layer L1 and the second layer L2 are joined to each other at high density in the recess portion EM. As being heated by the heating unit 89, the layers of the recess portion EM are reliably joined to each other by the bonding material contained in the second web W3. On the other hand, the part other than the recess portion EM is not pressed by the projection 322 and is heated under non-pressure in the heating unit 89. Therefore, the part other than the recess portion EM can maintain the density of the first web W2 and the second web W3 substantially, and can be in a state where flexibility is high.

In this manner, the processing roller 320 functions as a pressing processing unit that partially presses the second web W3 and gives an uneven shape to the second web W3. The processing roller 320 can perform the embossing with respect to the sheet S by partially pressing the second web W3 by the projection 322. Therefore, it is possible to change the touch of the sheet S3, and it is possible to manufacture the sheet S having the uneven decoration.

The sheet manufacturing apparatuses 100 and 100A to which the processing roller 320 is applied have an uneven shape, and it is possible to manufacture the sheet S3 having a density difference within the surface. Therefore, it is possible to manufacture the sheet S3 in which the density and the like are different for each layer and the uneven shape due to the density difference is given within the surface.

In the fourth embodiment, a configuration in which one-side embossing is performed with respect to the second web W3 by using the rollers 321 and 325 is illustrated as an example, but a configuration in which double-side embossing is performed may be employed. In other words, the projection 322 may also be provided in the roller 325. Further, the shape of the projection 322 is not limited to the example of Fig. 8, and a geometric pattern, a lattice shape, or any other shapes can be employed.

In addition, the processing roller 320 is not limited to an aspect of being installed instead of the pressurizing unit 82 in the sheet manufacturing apparatuses 100 and 100A, but may be additionally installed between the pressurizing unit 82 and the heating unit 89. Similar considerations apply to the third embodiment. As implicit above, the processing roller 320 can also be used in the second embodiment. A combination of the processing rollers 310, 320 is also possible.

### 5. Fifth Embodiment

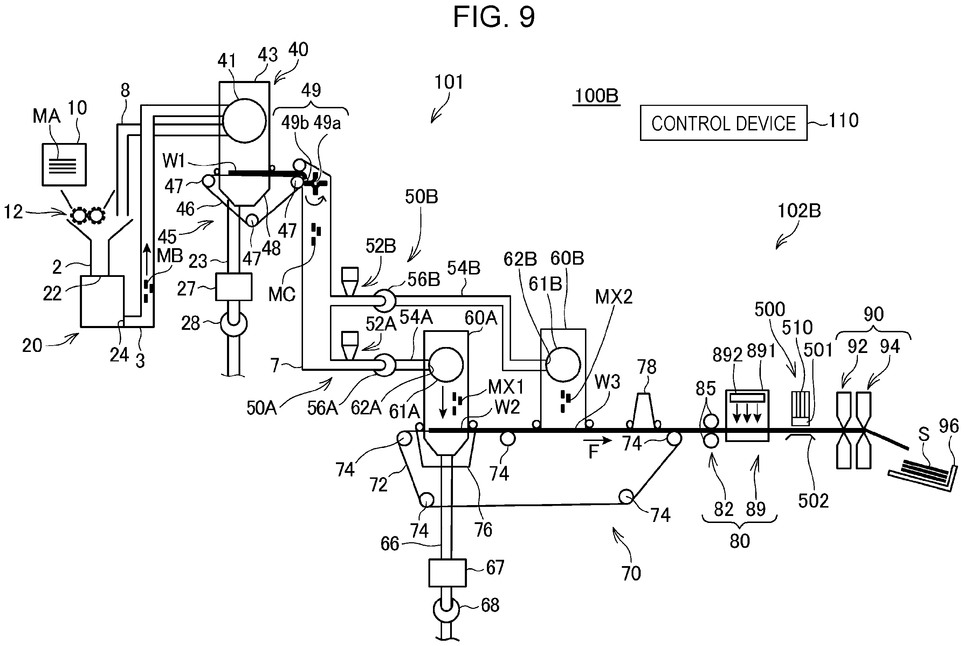

Fig. 9 is a schematic configuration view of a sheet manufacturing apparatus 100B according to the fifth embodiment. The sheet manufacturing apparatus 100B includes a manufacturing unit 102B instead of the manufacturing unit 102 in the sheet manufacturing apparatus 100 (Fig. 1). The manufacturing unit 102B has a configuration in which a printing unit 500 is disposed between the processing unit 80 and the cutting unit 90. In the fifth embodiment, the configurations common to those of the sheet manufacturing apparatus 100 of the first embodiment will be given the same reference numerals, and the description thereof will be omitted.

The printing unit 500 includes a print head 501 that ejects ink toward the sheet S and an ink cartridge 510 that accommodates ink to be ejected by the print head 501. The print head 501 is capable of ejecting a plurality of colors of ink, and the ink cartridge 510 is provided corresponding to the number of colors of the ink ejected by the print head 501. Further, the printing unit 500 includes an ink supply mechanism (not illustrated) for supplying the ink from the ink cartridge 510 to the print head 501, a nozzle (not illustrated) for ejecting the ink from the print head 501, and the like.

In the printing unit 500, an image is formed by ejecting the ink onto the second web W3, that is, the sheet S processed into a sheet shape by the processing unit 80, by the print head 501. In the printing unit 500, for example, an image can be formed in the width direction (for example, the width WI direction in the drawing) of the sheet S by scanning the print head 501 in a direction intersecting the transport direction F. Further, the print head 501 may be a line inkjet head that extends in the width direction of the sheet S.

The second web W3 is pressurized and heated in the processing unit 80 and becomes the sheet S, and an image is formed by the printing unit 500. After this, the sheet S is cut by the cutting unit 90 and is discharged to the discharge unit 96.

The printing unit 500 executes the printing under the control of the control device 110. The control device 110 drives the printing unit 500 according to image data for printing and forms an image. Further, the control device 110 can form an image at a set position on the sheet S cut into a predetermined size by controlling the printing position on the sheet S with reference to the position where the first cutting unit 92 cuts the sheet S.

In this manner, the sheet manufacturing apparatus 100B includes the printing unit 500 that performs printing on the sheet S processed by the processing unit 80, and can form an image on the sheet S.

Further, in the first embodiment, in the examples (1) to (7) described for the use of the sheet S, the description of the use or the usage method of the sheet S may be printed by the printing unit 500. In this case, with respect to the sheet S containing the additives appropriate for a specific use, it is possible to notify the use or the usage method by the image formed on the sheet S. A printing unit can also be included in the other embodiments.

### 6. Sixth Embodiment

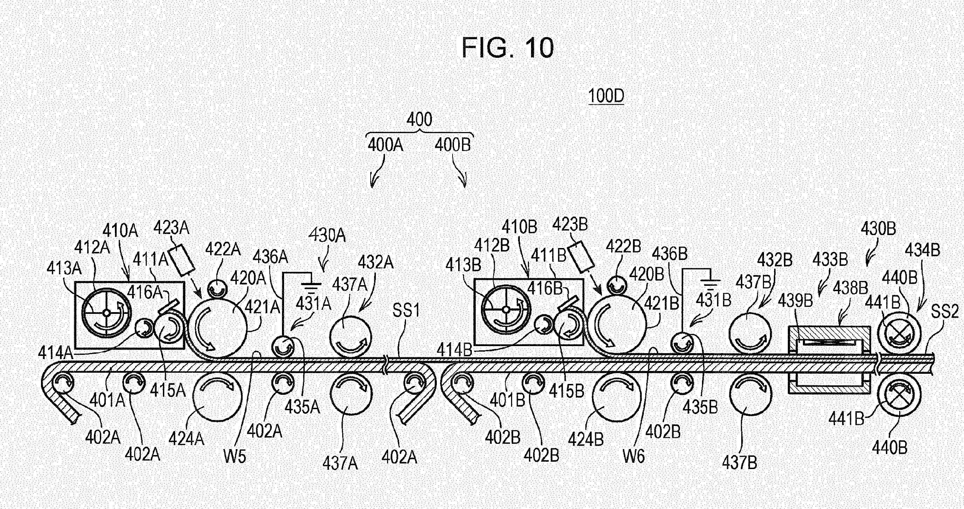

Fig. 10 is a schematic configuration view of a sheet manufacturing apparatus 100C according to a sixth embodiment. In the sixth embodiment, the configurations common to those of the sheet manufacturing apparatus 100 of the first embodiment will be given the same reference numerals, and the description thereof will be omitted.

The sheet manufacturing apparatus 100C includes a manufacturing unit 400 instead of the manufacturing unit 102 in the sheet manufacturing apparatus 100 (Fig. 1). In other words, the manufacturing unit 400 is provided instead of the first depositing unit 60A, the second depositing unit 60B, the web forming unit 70, and the processing unit 80 of the sheet manufacturing apparatus 100.

Further, the manufacturing unit 400 does not have a configuration that corresponds to the cutting unit 90, but these configurations may be provided in the manufacturing unit 400.

The manufacturing unit 400 includes a first manufacturing unit 400A and a second manufacturing unit 400B.

The first manufacturing unit 400A molds the mixture MX1 into a web shape by an electrostatic method and obtains a first web W5. In other words, the first manufacturing unit 400A electrostatically transfers the mixture MX1, which is a fiber-containing material, to a transport belt 401A that is a transfer object and forms the first web W5. The first manufacturing unit 400A manufactures an intermediate sheet SS1 by performing post-treatment for adjusting the surface properties of the first web W5.

In addition, the second manufacturing unit 400B deposits the mixture MX2 in a web shape on the intermediate sheet SS1 manufactured by the first manufacturing unit 400A by the antistatic method and obtains a second web W6. The second manufacturing unit 400B manufactures a sheet SS2 by performing post-treatment for adjusting the surface properties of the second web W6. The second web W6 corresponds to the web for processing.

Since the sheet SS2 has an elongated shape, the cutting unit 90 is provided at the rear stage of the second manufacturing unit 400B, cuts the sheet SS2, and similar to the sheet S of the first embodiment, the sheet SS2 may be cut into a regular size.

The first manufacturing unit 400A includes a supply unit 410A that supplies the mixture MX1, a carrier 420A for carrying the supplied mixture MX1, the transport belt 401A on which the carried mixture MX1 is electrostatically transferred, and a processing unit 430A for post-treatment.

The mixture MX1 is common to the first embodiment, and is a mixture of fibers derived from the raw material MA and additives added by the additive supply unit 52. Further, the mixture MX1 may contain a charge control agent or an electrical charge control agent for obtaining charging properties of the mixture MX1.

The supply unit 410A accommodates a storage unit 412A, an agitator 413A, a roller 414A, a first carrier 415A, and a blade 416A, in a housing unit 411A.

The storage unit 412A stores the mixture MX1 therein. The agitator 413A agitates the mixture MX1 in the storage unit 412A and charges the mixture MX1 by friction during the agitation.

The mixture MX1 is supplied to the first carrier 415A by the rotation of the roller 414A. The first carrier 415A has a potential difference with the roller 414A, and the mixture MX1 electrostatically adheres by the potential difference. The blade 416A adjusts the thickness or the adhesion amount of the mixture MX1 that adheres to the first carrier 415A, adjusts the mixture MX1 into a sheet shape, and charges the mixture MX1 by friction.

The carrier 420A has a potential difference with the first carrier 415A, and the mixture MX1 electrostatically adheres to the carrier 420A. The carrier 420A is a rotating roller member, and transfers the mixture MX1 carried by the carrier 420A to the transport belt 401A.

Around the carrier 420A, a charging unit 422A that charges an outer circumferential surface 421A of the carrier 420A and an exposure unit 423A that adjusts the potential of the outer circumferential surface 421A are provided. Furthermore, around the carrier 420A, a transfer unit 424A for transferring the mixture MX1 to the transport belt 401A is provided by an electrostatic force generated by a potential difference with the carrier 420A.

The transfer unit 424A pressurizes the mixture MX1 transferred to the transport belt 401A with the carrier 420A and makes the thickness of the mixture MX1 uniform. Accordingly, the first web W5 having a uniform thickness is formed on the transport belt 401A.

The transport belt 401A is configured with an endless belt and is transported by a plurality of rollers 402A. It is preferable that a surface of the transport belt 401A to which the mixture MX1 is transferred be made of a resin having medium to high resistance (volume resistivity of 107 to 1011 Ω·cm). As the type of resin, for example, a type in which the carbon black is kneaded in a fluorine-based resin can be used, but it is needless to say that other materials can also be used.

The first processing unit 430A includes a leveling processing unit 431A for smoothing the surface of the first web W5 and a pressurizing processing unit 432A for pressurizing the first web W5. The leveling processing unit 431A has a leveling roller 435A of which at least the outer circumferential surface is made of metal, smooths the surface of the first web W5 by the leveling roller 435A, and removes electricity of the first web W5 via a ground wire 436A.

The pressurizing processing unit 432A bonds the fibers contained in the first web W5 and fibers and resin to each other by performing the pressurizing by a pressurizing roller 437A, and makes the density uniform. The pressurizing force with which the pressurizing processing unit 432A pressurizes the first web W5 may be an extent of adjusting the density of the first web W5, and for example, similar to the pressurizing unit 82, the pressurizing force does not provide enough pressure to significantly increase the density of the first web W5. Through the processing of the pressurizing processing unit 432A, the intermediate sheet SS1 is formed. A blowing fan (not illustrated) or the like for promoting the separation of the intermediate sheet SS1 from the transport belt 401A may be provided on the downstream side of the pressurizing processing unit 432A.

The second manufacturing unit 400B includes a supply unit 410B that supplies the mixture MX2, a carrier 420B for carrying the supplied mixture MX2, a transport belt 401B on which the carried mixture MX2 is electrostatically transferred, and a processing unit 430B for post-treatment.

The mixture MX2 is similar to the first embodiment, and is a mixture of fibers derived from the raw material MA and additives added by the additive supply unit 52. Further, the mixture MX2 may contain a charge control agent or an electrical charge control agent for obtaining charging properties of the mixture MX2. In addition, similar to the first embodiment, the mixture MX2 may contain the same components as those of the mixture MX1 or may contain different components.

Similar to the transport belt 401A, the transport belt 401B is configured with an endless belt and is transported by a plurality of rollers 402B. It is preferable that a surface of the transport belt 401B on which the intermediate sheet SS1 is mounted be made of a resin having medium to high resistance (volume resistivity of 107 to 1011 Ω·cm) as will be described later. As the type of resin, for example, a type in which the carbon black is kneaded in a fluorine-based resin can be used, but it is needless to say that other materials can also be used.

The transport belt 401B is disposed to be adjacent to the transport belt 401A of the first manufacturing unit 400A, and the intermediate sheet SS1 transported by the transport belt 401A is moved from the transport belt 401A to the transport belt 401B. The transport belt 401B transports the intermediate sheet SS1 from the upstream side of the supply unit 410B.

The supply unit 410B accommodates a storage unit 412B, an agitator 413B, a roller 414B, a first carrier 415B, and a blade 416B, in a housing unit 411B.

The storage unit 412B stores the mixture MX2 therein. The agitator 413B agitates the mixture MX2 in the storage unit 412B and charges the mixture MX2 by friction during the agitation.

The mixture MX2 is supplied to the first carrier 415B by the rotation of the roller 414B. The first carrier 415B has a potential difference with the roller 414B, and the mixture MX2 electrostatically adheres by the potential difference. The blade 416B adjusts the thickness or the adhesion amount of the mixture MX2 that adheres to the first carrier 415B, adjusts the mixture MX2 into a sheet shape, and charges the mixture MX2 by friction.

The carrier 420B has a potential difference with the first carrier 415B, and the mixture MX2 electrostatically adheres to the carrier 420B. The carrier 420B is a rotating roller member, and transfers the mixture MX2 carried by the carrier 420B to the transport belt 401B.

Here, the intermediate sheet SS1 manufactured by the first manufacturing unit 400A is placed on the transport belt 401B. Therefore, the web-like mixture MX2 carried by the carrier 420B is transferred onto the intermediate sheet SS1 on the transport belt 401B. Here, the second web W6 on which the intermediate sheet SS1 and the mixture MX2 are laminated is formed.

Around the carrier 420B, a charging unit 422B that charges an outer circumferential surface 421B of the carrier 420B and an exposure unit 423B that adjusts the potential of the outer circumferential surface 421B are provided. Furthermore, around the carrier 420B, a transfer unit 424B for transferring the mixture MX2 to the intermediate sheet SS1 on the transport belt 401B is provided by an electrostatic force generated by a potential difference with the carrier 420B.

The transfer unit 424B pressurizes the second web W6 including the mixture MX2 transferred to the transport belt 401B with the carrier 420B and makes the thickness uniform. Accordingly, the second web W6 having a uniform thickness is formed on the transport belt 401B. The carrier 420B and the transfer unit 424B configure the web forming unit together with the first manufacturing unit 400A.

The second processing unit 430B includes a leveling processing unit 431B that smooths the surface of the second web W6 and a pressurizing processing unit 432B that pressurizes the second web W6. Further, a semi-solidification processing unit 433B for semi-solidifying the surface of the second web W6 and a solidifying unit 434B for solidifying the second web W6 are provided. The leveling processing unit 431B has a leveling roller 435B of which at least the outer circumferential surface is made of metal, smooths the surface of the second web W6 by the leveling roller 435B, and removes electricity of the second web W6 via a ground wire 436B.

The pressurizing processing unit 432B (pressurizing unit) bonds the fibers contained in the second web W6 and fibers and resin to each other by performing the pressurizing by a pressurizing roller 437B, and makes the density uniform.

The semi-solidification processing unit 433B (heating unit) includes a chamber 438B configured with a heat insulating material and a heater 439B provided in the chamber 438B, and the surface of the second web W6 is semi-solidified by performing the heating by the heater 439B.

The pressurizing force with which the pressurizing processing unit 432B pressurizes the second web W6 may be an extent of adjusting the density of the second web W6, and for example, similar to the pressurizing unit 82, the pressurizing force does not satisfy the pressure for increasing the density of the second web W6.

The solidifying unit 434B includes a solidifying roller 440B and a heater 441B provided in the solidifying roller 440B. The solidifying unit 434B heats the solidifying roller 440B by energizing the heater 441B and pressurizes the second web W6 while heating the second web W6 by the solidifying roller 440B. Accordingly, the resin contained in the second web W6 is melted, and the fibers and the resin of the second web W6 are bound to each other, and the sheet SS2 is formed. After the sheet SS2 passes through the solidifying roller 440B, for example, the sheet SS2 is naturally cooled, bound, and solidified.

A blowing fan (not illustrated) or the like for promoting the separation of the sheet SS2 from the transport belt 401B may be provided on the downstream side of the second processing unit 430B.

Compared to the manufacturing unit 102 of the first embodiment, the manufacturing unit 400 does not require defibration, sorting, suctioning, web forming, and the like, and thus, the configuration of the sheet manufacturing apparatus 100C can be simplified. Therefore, there is an advantage that the manufacturing time of the sheet SS2 can be shortened, or the like.

In addition, since the solidifying unit 434B heats the second web W6 under non-pressure, the sheet SS2 maintains the difference in density between the first web W5 and the second web W6. In a case where the pressure with which the pressurizing processing unit 432A pressurizes the first web W5 and the pressure with which the pressurizing processing unit 432B pressurizes the second web W6 are different from each other, in the second web W6, the layer of the first mixture MX1 and the layer of the second mixture MX2 are different from each other in density. When the solidifying unit 434B performs heating under non-pressure, it is possible to give a difference in density between the layer of the first mixture MX1 and the layer of the second mixture MX2 in the sheet SS2.

In this manner, the sheet manufacturing apparatus 100C according to the sixth embodiment includes the manufacturing unit 400 as the web forming unit that forms the second web W6 that serves as the web for processing. The carrier 420B, the transfer unit 424B, and the first manufacturing unit 400A are provided. The web forming unit includes the supply unit 410A and the carrier 420A that serve as the first depositing unit that deposits the first mixture MX1 containing fibers and forms the first web W5. In addition, the supply unit 410B and the carrier 420B that serve as the second depositing unit that deposits the second mixture MX2 containing fibers on the first web W5 and forms the second web W6 are provided. The sheet manufacturing apparatus 100 includes the solidifying unit 434B (processing unit) for manufacturing the sheet by performing the processing including the heating with respect to the second web W6 formed by the web forming unit. According to the configuration, the deposited states of the first depositing unit and the second depositing unit are reflected in the density in the thickness direction of the sheet SS2 to be manufactured. Therefore, it is possible to adjust the density and the like in the thickness direction of the sheet SS2.

In addition, since the solidifying unit 434B that heats the second web W6 in a non-pressurized state is provided, it is possible to manufacture the sheet SS2 by dissolving the bonding material without substantially changing the densities of the first web W5 and the second web W6. Therefore, a difference in the density of each layer in the second web W6 can be left, and the sheet SS2 having different densities in the thickness direction can be manufactured.

Further, the processing unit 80 includes the pressurizing processing unit 432B for pressurizing the second web W6, and the solidifying unit 434B heats the second web W6 pressurized by the pressurizing processing unit 432B. The density of the second web W6 can be adjusted by the pressurizing processing unit 432B. Therefore, it is possible to adjust the density of the sheet SS2 to a state different from the density of the first web W5 or the second web W6.

### 7. Other Embodiments

Each of the above-described embodiments is merely a specific aspect of implementing the invention described in the Claims, does not limit the invention, and can be implemented in various aspects as described below, for example, without departing from the scope of the invention.

For example, in the third embodiment, an example in which the processing roller 310 is applied to the sheet manufacturing apparatuses 100 and 100A has been described. The invention is not limited thereto, and the processing roller 310 can also be applied to the sheet manufacturing apparatuses 100B and 100C. The processing roller 320 is also similar thereto. Further, the printing unit 500 described in the fifth embodiment is not limited to the sheet manufacturing apparatus 100B, and can also be applied to the sheet manufacturing apparatuses 100, 100A, and 100C. Furthermore, it is also possible to combine two or more of the processing rollers 310 and 320 and the printing unit 500 and to apply the combination to one sheet manufacturing apparatus 100, and 100A to 100C.

In addition, the sheet manufacturing apparatuses 100 and 100A to 100C may be configured to manufacture not only the sheet S but also a board-like or web-like manufactured product configured with hard sheets or laminated sheets. In addition, the manufactured product is not limited to paper, but may be a nonwoven fabric. The nature of the sheet S is not particularly limited, may be paper which can be used as a recording paper (for example, a so-called PPC paper sheet) for the purpose of writing or printing, or may be wallpaper, wrapping paper, color paper, drawing paper, Kent paper or the like. In addition, in a case where the sheet S is a nonwoven fabric, in addition to a general nonwoven fabric, the sheet S may be fiber board, tissue paper, kitchen paper, cleaner, filter, liquid absorbing material, sound absorbing material, cushioning material, mat, and the like.

In addition, in the above-described embodiments, as the sheet manufacturing apparatus and the fiber raw material regenerating apparatus of the invention, the dry type sheet manufacturing apparatus 100 that manufactures the sheet S by obtaining the material by defibrating the raw material in the air and by using the material and the resin, has been described. The target of application of the invention is not limited thereto, and the invention can also be applied to a so-called wet type sheet manufacturing apparatus in which the raw material containing fibers are dissolved or suspended in a solvent, such as water, and the raw material is processed into a sheet. In addition, the invention can also be applied to an electrostatic type sheet manufacturing apparatus in which the material containing fibers defibrated in the air is adsorbed onto the surface of the drum by static electricity or the like and the raw material adsorbed onto the drum is processed into a sheet.

### 7. Seventh Embodiment

### 7-1. Overall Configuration of Sheet Manufacturing Apparatus

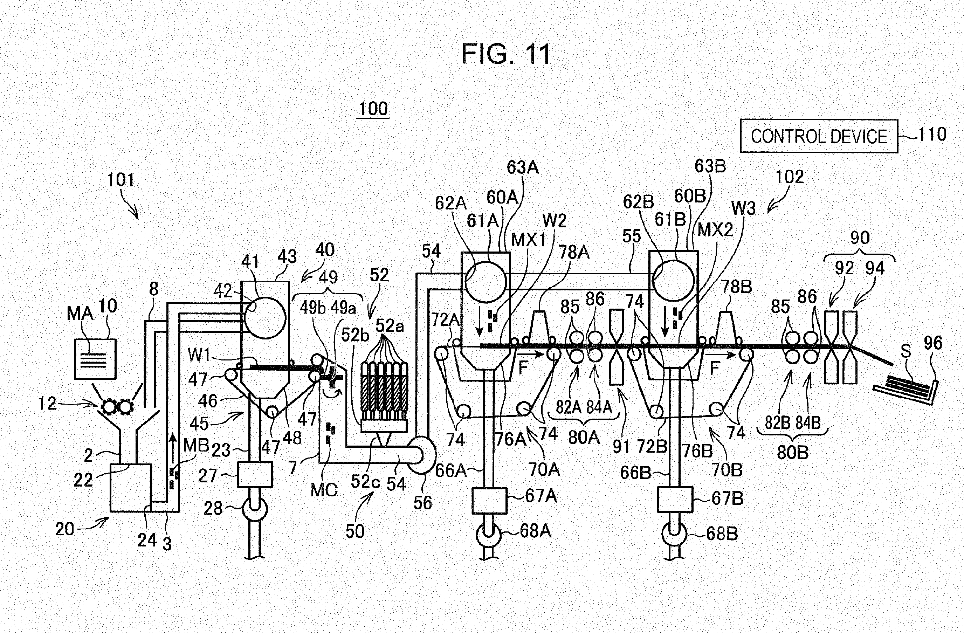

Fig. 11 is a schematic view illustrating a configuration of the sheet manufacturing apparatus 100.

The sheet manufacturing apparatus 100 executes regeneration processing of fiberizing the raw material MA containing fibers and regenerating the fiberized materials into the new sheet S. The sheet manufacturing apparatus 100 is capable of manufacturing the plurality of types of sheets S, and for example, by mixing additives with the fiberized raw material MA, according to the use, it is possible to adjust the bonding strength or whiteness of the sheet S, or to add a function of color, scent, flame retardancy and the like. Further, the sheet manufacturing apparatus 100 can adjust density, thickness, size, and shape of the sheet S. As a representative example of the sheet S, in addition to a sheet-like product, such as a printing paper sheet having a fixed size of A4 or A3, a cleaning sheet (for example, a floor cleaning sheet), an oil stain preventing sheet, or a toilet cleaning sheet, a shape of a paper tray and the like can be employed.

The sheet manufacturing apparatus 100 broadly includes a defibration processing unit 101 that obtains a material of the sheet S by refining the raw material MA and, and a manufacturing unit 102 that manufactures the sheet S by processing the material obtained by the defibration processing unit 101. The defibration processing unit 101 includes the supply unit 10, the coarse crushing unit 12, the defibrating unit 20, the sorting unit 40, the material molding unit 45, and the rotation body 49. Further, the manufacturing unit 102 includes the mixing unit 50, the first web forming unit 70A, a first processing unit 80A, a second web forming unit 70B, a second processing unit 80B, and the cutting unit 90. The first web forming unit 70A includes the first depositing unit 60A, and the second web forming unit 70B includes the second depositing unit 60B. Here, the rotation body 49 may be classified into the defibration processing unit 101 or the manufacturing unit 102. The sheet manufacturing apparatus 100 includes a control device 110 that controls each portion of the sheet manufacturing apparatus 100.

The supply unit 10 is an automatic charging device that accommodates the raw material MA therein and continuously charges the raw material MA into the coarse crushing unit 12. For example, the supply unit 10 includes a stocker (not illustrated) for stacking the raw material MA and a feeder (not illustrated) for feeding the raw material MA from the stocker. The raw material MA may be a material that contains fibers, for example, waste paper, discarded paper, or pulp sheet.

The coarse crushing unit 12 is a shredder that cuts the raw material MA supplied by the supply unit 10 with a blade. The raw material MA cut by the coarse crushing unit 12 is transported to the defibrating unit 20 via the pipe 2.

The defibrating unit 20 defibrates a coarse debris of the raw material MA. Defibration is a process of disentangling the raw material MA in a state where a plurality of fibers are bound to each other into one or a small number of fibers. The raw material MA can also be called a defibrated material. By defibrating the raw material MA by the defibrating unit 20, in addition to the effect of disentangling the fibers contained in the raw material MA, it is possible to expect an effect of separating substances, such as resin particles, ink, toner, blot preventing agent and the like that adhere to the raw material MA, from the fibers. The material that passes through the defibrating unit 20 is called a defibrated material MB.

In addition to the disentangled fibers, the defibrated material MB contains inclusions separated from the fiber when the defibrating unit 20 disentangles the fibers. The inclusions are particles or powder, and the components of the inclusions are, for example, resin particles contained in the raw material MA, colorants, such as ink and toner, or additives, such as blot preventing material or paper strength enhancing agent. The resin particle is a resin mixed for binding a plurality of fibers to each other at the time of manufacturing the raw material MA. The shape of the fiber contained in the defibrated material MB is, for example, a string shape or a ribbon shape. The fibers contained in the defibrated material MB may exist in an independent state while not being intertwined with other fibers, or may exist in a state of forming a so-called "dummy" while being in a form of a mass intertwined with other fibers.

The defibrating unit 20 performs defibration by a dry process. The dry process indicates processing in the air, such as atmosphere, rather than in liquid. The defibrating unit 20 can be configured, for example, by using a defiberizer, such as an impeller-mill. Specifically, the defibrating unit 20 includes a rotor (not illustrated) that rotates and a liner (not illustrated) positioned at an outer circumference of the rotor (not illustrated), and defibrates the coarse debris nipping the coarse debris between the rotor and the liner.

From the coarse crushing unit 12 to the defibrating unit 20, the coarse debris is transported by an airflow. A configuration in which the airflow is generated by the defibrating unit 20 may be employed, or the airflow may be generated by providing a blower (not illustrated) on an upstream side or a downstream side of the defibrating unit 20 in the transport direction of the coarse debris or the defibrated material MB. In addition, the defibrated material MB is sent from the defibrating unit 20 through the pipe 3 to the sorting unit 40 by the airflow. The airflow by which the defibrated material MB is transported to the sorting unit 40 may be generated by the defibrating unit 20, or the airflow of the above-described blower may be used.

The sorting unit 40 sorts the components contained in the defibrated material MB according to the size of the fibers. The size of the fiber mainly refers to the length of the fiber.

The sorting unit 40 has the drum unit 41 and the housing unit 43 that accommodates the drum unit 41 therein. The drum unit 41 is a member that functions as a sieve, and includes, for example, a mesh, a filter, a screen, and the like that function as a sieve having an opening. The drum unit 41 has a cylindrical shape that is rotationally driven by a motor, and at least a part of a circumferential surface is a mesh. The mesh of the drum unit 41 is configured with wire gauze, expanded metal obtained by stretching a metal plate having cuts, punching metal, and the like. The defibrated material MB introduced into the drum unit 41 from the introduction port 42 is divided into a passing material that passes through the opening of the drum unit 41 and a residue that does not pass through the opening, due to the rotation of the drum unit 41. The passing material that has passed through the opening contains fibers or particles smaller than the opening, and the fibers or particles are called a first sorted material. The residue contains fibers, undefibrated pieces, or dams larger than the opening, and the fibers, undefibrated pieces, or dams are called a second sorted material. The first sorted material descends inside the housing unit 43 toward the material molding unit 45. The second sorted material is sent to the defibrating unit 20 as described above.

Instead of the sorting unit 40, the sheet manufacturing apparatus 100 may include a classifier that separates the first sorted material and the second sorted material. The classifier is, for example, a cyclone classifier, an elbow jet classifier, and an eddy classifier. The classifier may be configured to separate smaller components or components having a low density among the components contained in the first sorted material. For example, from the first sorted material, it is possible to adopt a configuration in which resin particles, colorants, or additives which are pulled off from the fibers by the defibrating unit 20 are separated by the classifier and removed.

The material molding unit 45 includes the mesh belt 46, the stretching roller 47, and the suction unit 48. The mesh belt 46 is an endless metal belt, and is stretched over the plurality of stretching rollers 47. The mesh belt 46 circulates around a track configured with the stretching roller 47. A part of the track of the mesh belt 46 is flat under the drum unit 41, and the mesh belt 46 configures a flat surface.

Multiple openings are provided in the mesh belt 46, components larger than the opening of the mesh belt 46 in the first sorted material that descends from the drum unit 41 to the mesh belt 46 are deposited on the mesh belt 46. Components smaller than the opening of the mesh belt 46 in the first sorted material pass through the opening. The components that pass through the opening of the mesh belt 46 are called a third sorted material.

The suction unit 48 is connected to the first dust collecting unit 27 via a pipe 23. The first dust collecting unit 27 includes a filter (not illustrated) for separating the third sorted material from the airflow, and the first collection blower 28 is installed on the downstream side of the first dust collecting unit 27. The first collection blower 28 suctions the air from the first dust collecting unit 27. Due to a suctioning force of the first collection blower 28, the third sorted material that has passed through the opening of the mesh belt 46 is sent from the suction unit 48 to the first dust collecting unit 27, and is collected by the filter of the first dust collecting unit 27. Since the first sorted material that descends from the drum unit 41 is attracted to the mesh belt 46 by the airflow suctioned by the suction unit 48, there is an effect of promoting the deposition.

The components deposited on the mesh belt 46 are molded in a web shape. The components are called the web-like material W1. In this manner, the material molding unit 45 forms the web-like material W1 from the first sorted material sorted by the sorting unit 40. The web-like material W1 has fibers larger than the opening of the mesh belt 46 among the components contained in the first sorted material as the main components thereof, and is formed in a state of being soft and bulging containing a large amount of air. The web-like material W1 is transported to the rotation body 49 according to the movement of the mesh belt 46.

The rotation body 49 includes the base portion 49a connected to a driving unit (not illustrated), such as a motor and the protrusion portion 49b that protrudes from the base portion 49a, and as the base portion 49a rotates in the direction R, the protrusion portion 49b rotates around the base portion 49a. The protrusion portion 49b has, for example, a plate-like shape. In the example of Fig. 11, four protrusion portions 49b are provided at equivalent intervals on the base portion 49a.

The rotation body 49 is positioned in the end portion of the flat part of the track of the mesh belt 46. In the end portion, since the track of the mesh belt 46 is bent downward, the mesh belt 46 bends downward and moves. Therefore, the web-like material W1 transported by the mesh belt 46 protrudes from the mesh belt 46 and comes into contact with the rotation body 49. The web-like material W1 is disentangled by the collision of the protrusion portion 49b with the web-like material W1 and becomes a small fiber mass. The mass is transported through the pipe 7 positioned below the rotation body 49 to the mixing unit 50. Since the web-like material W1 has a soft structure formed by depositing fibers on the mesh belt 46 as described above, the web-like material W1 is easily divided when colliding with the rotation body 49.

The material obtained by dividing the web-like material W1 by the rotation body 49 is called a material MC. The material MC is obtained by removing the third sorted material from the above-described first sorted material, and the main component thereof is fiber.

The position of the rotation body 49 is a position where the protrusion portion 49b can come into contact with the web-like material W1 and is provided at a position where the protrusion portion 49b does not come into contact with the mesh belt 46. It is preferable that the distance between the protrusion portion 49b and the mesh belt 46 at the position where the protrusion portion 49b and the mesh belt 46 are closest to each other, for example, be 0.05 mm or more and 0.5 mm or less.

The mixing unit 50 mixes the material MC and additives with each other. The mixing unit 50 includes the additive supply unit 52 that supplies the additives and the mixing blower 56. In addition, the mixing unit 50 may include the pipe 54 for transporting the material MC and additives.

In the additive supply unit 52, at least one additive cartridge 52a that stores the additives therein is set. The additive cartridge 52a may be attachable to and detachable from the additive supply unit 52. The additive supply unit 52 includes an additive extracting unit 52b that extracts the additive(s) from the additive cartridge(s) 52a and an additive charging unit 52c that discharges the additive(s) extracted by the additive extracting unit 52b to the pipe 54. The additive extracting unit 52b includes a feeder (not illustrated) for sending out the additive(s) containing fine powder or fine particles which are in the additive cartridge(s) 52a, and extracts the additive from some or all of the additive cartridges 52a. The additive extracted by the additive extracting unit 52b is sent to the additive charging unit 52c. The additive charging unit 52c accommodates the additive extracted by the additive extracting unit 52b. The additive charging unit 52c includes a shutter (not illustrated) which can be opened and closed in a connecting portion with the pipe 54, and by opening the shutter, the additive extracted by the additive extracting unit 52b is sent out to the pipe 54.

The additive to be supplied from the additive supply unit 52 contains a resin (binder) for binding a plurality of fibers to each other. The resin contained in the additive is melted by being heated to a temperature equal to or higher than a glass transition point in the manufacturing unit 102 and binds the fibers of the material MC to each other. The resin is, for example, a thermoplastic resin or a thermosetting resin. Specific examples thereof include AS resin, ABS resin, polypropylene, polyethylene, polyvinyl chloride, polystyrene, acrylic resin, polyester resin, polyethylene terephthalate, polyphenylene ether, polybutylene terephthalate, nylon, polyamide, polycarbonate, polyacetal, polyphenylene sulfide, polyether ether ketone, and the like. As the additive, a plurality of types of resins may be mixed with each other and used.

The additive to be supplied from the additive supply unit 52 may contain a component other than the resin for binding the plurality of fibers to each other. For example, in accordance with the type of the sheet S to be manufactured, a coloring agent for coloring the fibers, a coagulation preventing agent for suppressing coagulation of fibers or coagulation of resins, a flame retardant for making fibers and the like unlikely to burn, and the like, may be included. Further, the additive may be in a form of fiber or powder.

The mixing blower 56 generates the airflow from the pipe 7 toward the pipe 54. The material MC and the additive(s) to be supplied to the pipe 54 by the additive supply unit 52 are mixed with each other when passing through the mixing blower 56. For example, the mixing blower 56 can be configured to include a motor (not illustrated), a blade (not illustrated) that is driven by the motor and rotates, and a case (not illustrated) that accommodates the blade therein, and may have a configuration in which the blade and the case are connected to each other. Further, the mixing blower 56 may include a mixer that mixes the material MC and the additives with each other in addition to the blade that generates the airflow. The mixture mixed by the mixing unit 50 is transported to the first depositing unit 60A and the second depositing unit 60B by the airflow generated by the mixing blower 56.

The first depositing unit 60A includes the first drum unit 61A (first dispersing unit) and the first housing unit 63A. Further, the first depositing unit 60A has the first introduction port 62A for introducing the mixture mixed by the mixing blower 56 into the first drum unit 61A. The mixture introduced into the first introduction port 62A is indicated by a symbol MX1. The first drum unit 61A is, for example, a cylindrical structure configured similarly to the drum unit 41, rotates by the power of a motor (not illustrated) similarly to the drum unit 41, and functions as a sieve. The first drum unit 61A has an opening, disperses the mixture MX1 (first material) disentangled by the rotation of the first drum unit 61A in the air, and makes the mixture MX1 descend from the opening.

The first web forming unit 70A is disposed below the first drum unit 61A. The first web forming unit 70A includes a mesh belt 72A (first depositing unit) and a stretching roller 74. The mesh belt 72A is configured with an endless metal belt similar to the mesh belt 46, and is stretched over the plurality of stretching rollers 74. The mesh belt 72A circulates around a track configured with the stretching rollers 74. A part of the track of the mesh belt 72A has a flat portion below the first drum unit 61A and below the second drum unit 61B which will be described later, and the mesh belt 72A configures a flat surface in the flat portion.

The mesh belt 72A has multiple openings. In the mixture MX1 that descends from the first drum unit 61A, a component larger than the opening of the mesh belt 72A is deposited on the mesh belt 72A. In addition, a component smaller than the opening of the mesh belt 72A in the mixture MX1 passes through the opening below the first drum unit 61A.

The first web forming unit 70A has a suction mechanism 76A disposed below the mesh belt 72A. The suction mechanism 76A suctions the air from the side opposite to the first drum unit 61A with respect to the mesh belt 72A.

The suction mechanism 76A is connected to a pipe 66A. The pipe 66A is connected to a second collection blower 68A via a second dust collecting unit 67A. The second dust collecting unit 67A includes a filter (not illustrated) that collects particles or fibers that have passed through the mesh belt 72A. The second collection blower 68A is a blower that suctions the air through the pipe 66A. The suction mechanism 76A suctions the air from below the mesh belt 72A by the suction force of the second collection blower 68A and collects the particles or fibers contained in the suctioned air by the second dust collecting unit 67A. The airflow suctioned by the second collection blower 68A has an effect of attracting the mixture MX1 that descends from the first drum unit 61A to the mesh belt 72A and promoting the deposition. In addition, the suctioned airflow of the second collection blower 68A forms a downflow in a path where the mixture MX1 falls from the first drum unit 61A, and it is also possible to expect an effect of preventing the fibers from being intertwined while falling. The mixture MX1 deposited on the mesh belt 72A has a web shape in the flat portion of the mesh belt 72A and configures the first web W2.

In the transport path of the mesh belt 72A, a first humidity control unit 78A is provided on the downstream side of the first drum unit 61A. The first humidity control unit 78A is a mist type humidifier that supplies water to the mesh belt 72A in a form of mist. The first humidity control unit 78A includes, for example, a tank for storing the water and an ultrasonic vibrator for making the water mist. Since a moisture content of the first web W2 is adjusted by the mist to be supplied by the first humidity control unit 78A, it is possible to expect an effect of suppressing adsorption or the like of the fibers onto the mesh belt 72A due to static electricity.

In the transport path of the mesh belt 72A, the first processing unit 80A is disposed on the downstream side of the first humidity control unit 78A. The first processing unit 80A is a processing unit that applies at least one of pressurizing processing and heating processing to the first web W2. The processing unit 80A of the embodiment includes a first pressurizing unit 82A that pressurizes the first web W2 and a first heating unit 84A that heats the first web W2 pressurized by the first pressurizing unit 82A.

The first pressurizing unit 82A is configured with the pair of calendar rollers 85 and 85 (first pressurizing roller). The first pressurizing unit 82A is connected to a press mechanism (not illustrated) for applying a nip pressure to the calendar rollers 85 and 85 by a hydraulic pressure and a driving unit (not illustrated), such as a motor for rotating the calendar rollers 85 and 85 toward the first heating unit 84A. The first pressurizing unit 82A pressurizes the first web W2 with a predetermined nip pressure by the calendar rollers 85 and 85, and transports the first web W2 toward the first heating unit 84A.

The first heating unit 84A includes a pair of heating rollers 86 and 86 (first heating roller). The first heating unit 84A includes a heater (not illustrated) for heating the circumferential surface of the heating roller 86 to a predetermined temperature and a driving unit (not illustrated), such as a motor for rotating the heating rollers 86 and 86 toward the cutting unit 90. The first heating unit 84A applies the heat while nipping the first web W2 of which the density has increased by the first pressurizing unit 82A.

The temperature at which the first heating unit 84A heats the first web W2 can be set to any temperature, but it is desirable that the first web W2 is heated until the resin contained in the mixture MX1 reaches a temperature equal to or higher than the glass transition point. In this case, the resin contained in the first web W2 is melted and binds the fibers to each other.

The sheet manufacturing apparatus 100 includes an intermediate cutting unit 91 (first cut unit) on the downstream side of the first processing unit 80A. The intermediate cutting unit 91 is a cutter having a plurality of movable blades or a movable blade and a fixed blade, and cuts the first web W2. The first web W2 is transported in the transport direction indicated by the symbol F in the drawing as the mesh belt 72A moves. Here, the transport direction F is the length direction of the first web W2, and the direction orthogonal to the transport direction F is the width direction of the first web W2. The intermediate cutting unit 91 cuts both ends in the width direction of the first web W2. In addition, the intermediate cutting unit 91 may cut the first web W2 in the direction intersecting the transport direction F. Further, the intermediate cutting unit 91 may also include a cutter for cutting the first web W2 along the transport direction F and a cutter for cutting the first web W2 in the direction intersecting the transport direction F.

The first web W2 cut by the intermediate cutting unit 91 is sent to the second web forming unit 70B.

The second web forming unit 70B includes a mesh belt 72B (second depositing unit) and the stretching roller 74. The mesh belt 72B is configured with an endless metal belt similar to the mesh belt 72A, and is stretched over the plurality of stretching rollers 74. The mesh belt 72B circulates around a track configured with the stretching rollers 74.

The second depositing unit 60B is disposed above the second web forming unit 70B. A part of the track of the mesh belt 72B has a flat portion below the second drum unit 61B, and the mesh belt 72B configures a flat surface in the flat portion. The mesh belt 72B transports the first web W2 cut by the intermediate cutting unit 91 in the transport direction F so as to pass through the second drum unit 61B.

The second depositing unit 60B includes the second drum unit 61B (second dispersing unit) and the second housing unit 63B.

A pipe 55 is connected to the housing unit 63A. The pipe 55 is a pipe that branches from the pipe 54, and the mixture mixed by the mixing blower 56 is sent to the second depositing unit 60B by the pipe 55. The mixture sent to the second depositing unit 60B by the pipe 55 is indicated by the symbol MX2.

In the embodiment, the mixture MX2 is the same component as the mixture MX1, but the mixture MX2 may be a component different from the mixture MX1. The configuration can be realized by providing a plurality of additive supply units 52 and mixing blowers 56, for example with one or more additive supply units 52 and mixing blowers 56 provided after the branching point of the pipe 55 from the pipe 54.

The second depositing unit 60B has the second introduction port 62B for introducing the mixture MX2 sent by the pipe 55 into the second drum unit 61B. The second drum unit 61B is a cylindrical structure configured similarly to the first drum unit 61A, rotates by the power of a motor (not illustrated), and functions as a sieve. The second drum unit 61B has an opening, disperses the mixture MX2 (second material) disentangled by the rotation of the second drum unit 61B in the air, and makes the mixture MX2 descend from the opening. The mixture MX2 descends toward the mesh belt 72B on which the first web W2 is placed.

The mesh belt 72B has multiple openings. The opening size of the mesh belt 72B may be the same extent as the opening of the mesh belt 72A, or may be larger or smaller than the opening of the mesh belt 72A. The first web W2 is maintained in a state of being placed on the mesh belt 72B. The mixture MX2 that descends from the second drum unit 61B is deposited on the first web W2 placed on the mesh belt 72B.

By depositing the mixture MX2 on the first web W2, the second web W3 is formed in the second web forming unit 70B. The second web W3 is a two-layer web having a layer of the first web W2 processed by the first processing unit 80A and a layer of the mixture MX2 deposited by the second web forming unit 70B.

The second web forming unit 70B has a suction mechanism 76B disposed below the mesh belt 72B. The suction mechanism 76B suctions the air from the side opposite to the second drum unit 61B with respect to the mesh belt 72B.

The suction mechanism 76B is connected to a pipe 66B. The pipe 66B is connected to a third collection blower 68B via a third dust collecting unit 67B. The third dust collecting unit 67B includes a filter (not illustrated) that collects particles or fibers that have passed through the mesh belt 72B. The third collection blower 68B is a blower that suctions the air through the pipe 66B. The suction mechanism 76B suctions the air from below the mesh belt 72B by the suction force of the third collection blower 68B and collects the particles or fibers contained in the suctioned air by the third dust collecting unit 67B. The airflow suctioned by the third collection blower 68B has an effect of attracting the mixture MX2 that descends from the second drum unit 61B to the mesh belt 72B and promoting the deposition. In addition, the suctioned airflow of the third collection blower 68B forms a downflow in a path where the mixture MX2 falls from the second drum unit 61B, and it is also possible to expect an effect of preventing the fibers from being intertwined while falling.

In the transport path of the mesh belt 72B, a second humidity control unit 78B is provided on the downstream side of the second drum unit 61B. The second humidity control unit 78B is a mist type humidifier that supplies water to the mesh belt 72B in a form of mist. The second humidity control unit 78B includes, for example, a tank for storing the water and an ultrasonic vibrator for making the water mist. Since a moisture content of the second web W3 is adjusted by the mist to be supplied by the second humidity control unit 78B, it is possible to expect an effect of suppressing adsorption or the like of the fibers onto the mesh belt 72B due to static electricity.

In the transport path of the mesh belt 72B, the second processing unit 80B is disposed on the downstream side of the second humidity control unit 78B. The second processing unit 80B is a processing unit that applies at least one of pressurizing processing and heating processing to the second web W3. The second processing unit 80B of the embodiment includes a second pressurizing unit 82B that pressurizes the second web W3 and a second heating unit 84B that heats the second web W3 pressurized by the second pressurizing unit 82B.

The second pressurizing unit 82B is configured with the pair of calendar rollers 85 and 85 (second pressurizing roller). Similar to the first pressurizing unit 82A, the second pressurizing unit 82B pressurizes the second web W3 with a predetermined nip pressure by the calendar rollers 85 and 85, and transports the second web W3 toward the second heating unit 84B.

The second heating unit 84B includes the pair of heating rollers 86 and 86. Similar to the first heating unit 84A, the second heating unit 84B nips the second web W3 and gives heat thereto, and transports the second web W3.

The second web W3 is heated by the second heating unit 84B, and accordingly, the fibers contained in the mixture MX2 are combined with resin and form a sheet shape. Further, the second web W3 has a configuration in which the mixture MX2 is deposited on the first web W2 formed into a sheet shape by the first processing unit 80A. Therefore, by being heated by the second heating unit 84B, the fibers contained in the first web W2 and the fibers of the mixture MX2 are bonded to each other by the resin, and the sheet S to which the plurality of layers are firmly bonded is molded.

The second web W3 is pressurized and heated by the second processing unit 80B, is molded into the sheet shape, and becomes the sheet S. The sheet S has a multilayer structure including a layer configured with the first web W2 and a layer configured with the mixture MX2.

In addition, the nip pressure at which the second pressurizing unit 82B pressurizes the second web W3 can be set to a pressure different from the nip pressure of the first pressurizing unit 82A.

The cutting unit 90 cuts the sheet S processed by the second processing unit 80B. The cutting unit 90 includes the first cutting unit 92 that cuts the sheet S in a direction intersecting the transport direction F, and the second cutting unit 94 that cuts the sheet S in a direction parallel to the transport direction F. The cutting unit 90 cuts the length and the width of the sheet S to a predetermined size and forms a single-cut sheet S. The sheet S cut by the cutting unit 90 is accommodated in the discharge unit 96. The discharge unit 96 includes a tray or a stacker that accommodates the manufactured sheet therein, and the user can take out and use the sheet S discharged onto the tray.

The control device 110 controls each portion of the sheet manufacturing apparatus 100 and executes the manufacturing of the sheet S. For example, the control device 110 executes a starting sequence when starting the manufacturing of the sheet S. In the starting sequence, each portion of the sheet manufacturing apparatus 100 is sequentially started. The control device 110 executes a hydraulic control of the first pressurizing unit 82A and the second pressurizing unit 82B, control of cutting timing in the intermediate cutting unit 91 and cutting unit 90, and the like. Further, the control device 110 controls the start and stop of each of the blowers including the first collection blower 28, the mixing blower 56, and the second collection blower 68A. In addition, the control device 110 controls humidification by the first humidity control unit 78A and the second humidity control unit 78B.

In the sheet manufacturing apparatus 100, the defibration processing unit 101 and the manufacturing unit 102 may be formed integrally or may be configured separately.

For example, after manufacturing the web-like material W1 by the defibration processing unit 101, the web-like material W1 can also be extracted from the sheet manufacturing apparatus 100 and stored without being transferred to the rotation body 49. Further, the manufactured product may be enclosed in a predetermined package and may be in a form that can be transported and traded.

The manufacturing unit 102 can be configured to be separated from the defibration processing unit 101, and in this case, the web-like material W1 may be configured to be supplied to the mixing unit 50.

In the sheet manufacturing apparatus 100 configured in this manner, the nip pressure at which the second pressurizing unit 82B pressurizes the second web W3 can be set to a pressure different from the nip pressure of the first pressurizing unit 82A. For example, when the pressure with which the first pressurizing unit 82A pressurizes the first web W2 and the pressure with which the second pressurizing unit 82B pressurizes the second web W3 are different from each other, in the sheet S to be manufactured by the sheet manufacturing apparatus 100, it becomes possible to change the density in the thickness direction.

### 7-2. Manufacturing Example of Sheet

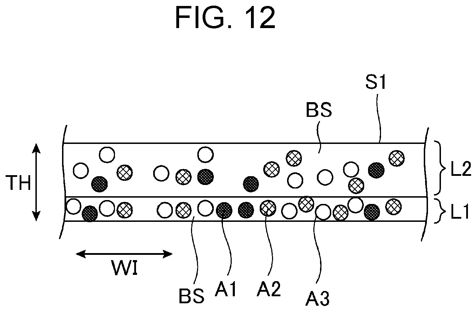

Fig. 12 is a sectional view illustrating the sheet S1 as an example of the sheet S manufactured by the sheet manufacturing apparatus 100.

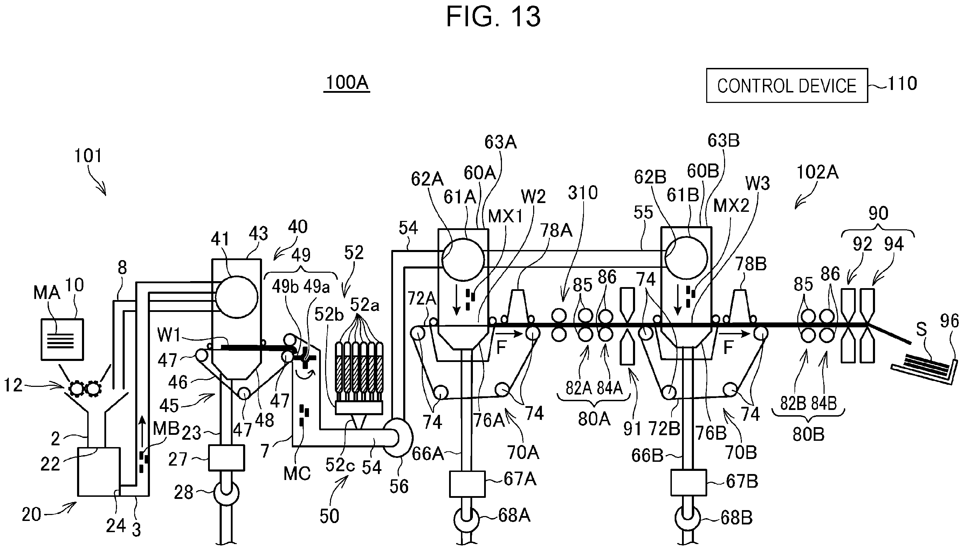

In the sectional view of Fig. 13, the thickness of the sheet S1 is indicated by the symbol TH and the width of the sheet S1 is indicated by the symbol WI. The sheet S1 contains the base BS. In the base BS, the fibers contained in the raw material MA are bonded by the resin of the binder added by the additive supply unit 52. In addition, in a case where the additive supply unit 52 adds an additive that serves as a colorant, particles A1, A2, and A3 of the additive are dispersed and mixed in the base BS.

The sheet S1 has a multilayer structure having a first layer L1 formed by processing the mixture MX1 with the first processing unit 80A and a second layer L2 derived from the mixture MX2 deposited on the first layer L1. The thickness and the density of the first layer L1 are determined by the pressure when the first pressurizing unit 82A pressurizes the first web W2 and the pressure with which the second pressurizing unit 82B pressurizes the second web W3. In addition, the thickness and the density of the second layer L2 are determined by the pressure with which the second pressurizing unit 82B pressurizes the second web W3.

For example, when the pressure applied by the second pressurizing unit 82B is lower than the pressure applied by the first pressurizing unit 82A, the density of the second layer L2 becomes lower than that of the first layer L1. In this manner, by changing the pressures of the first pressurizing unit 82A and the second pressurizing unit 82B, it is possible to manufacture the sheet S1 of which the density changes in the thickness TH direction.

Further, the sheet manufacturing apparatus 100 manufactures the sheet S including the first web W2 deposited on the mesh belt 72A by the first depositing unit 60A and the mixture MX2 deposited on the first web W2 by the second depositing unit 60B. The thickness of the mixture MX1 deposited by the first depositing unit 60A and the thickness of the mixture MX2 deposited by the second depositing unit 60B can be adjusted by controlling the control device 110, for example. Specifically, by controlling the rotational speed of the first drum unit 61A, the amount of the mixture MX1 that descends from the first drum unit 61A per unit time can be adjusted. Further, by controlling a transport speed of the mesh belt 72A, it is possible to adjust the thickness of the first web W2 deposited on the mesh belt 72A. Similarly, by controlling the rotational speed of the second drum unit 61B, the amount of the mixture MX2 that descends from the second drum unit 61B per unit time can be adjusted. Further, by controlling the transport speed of the mesh belt 72B, it is possible to adjust the thickness of the mixture MX2 deposited on the first web W2 by the mesh belt 72B.

Therefore, the sheet manufacturing apparatus 100 has a plurality of layers in the thickness direction TH, and it is possible to manufacture the sheets S having different densities and/or thicknesses for each layer.

### 7-3. Application Example of Sheet

Regarding the sheet S manufactured by the sheet manufacturing apparatus 100, the sheet S having the functionality specialized for a specific use can be manufactured by appropriately selecting the components of the additive. Here, a specific example of the combination of the use of the sheet S and the additive added by the additive supply unit 52 will be presented. (1) Plant Cultivation Sheet

The sheet manufacturing apparatus 100 can manufacture the sheet S that can be used as a medium for sowing seeds of plants. The sheet S contains a material used for plant cultivation as an additive to be added by the additive supply unit 52. Specifically, a particulate fertilizer can be used as an additive. As the fertilizer, nitrogenous fertilizers, such as ammonium sulfate, ammonium chloride, ammonium nitrate and the like, can be employed. In addition, particles of phosphatic fertilizer, such as superphosphate, concentrated superphosphate, or fused phosphate, may be used. In addition, particles of potassium fertilizer, such as potassium chloride or potassium nitrate may be used. Further, among the particles of the fertilizer, the plurality of types of particles may be used as additives, or a mixture of the plurality of types of particles of the fertilizer may be used as an additive. In addition, in a case where the sheet S is used as the plant cultivation sheet, as an additive, a pH adjusting agent may be used, and for example, particles, such as organic lime, vegetable ash, quicklime, hydrated lime, and the like can be adopted. The additives can be dispersed and disposed inside the sheet S as illustrated as the additives A1, A2 and A3 in Fig. 12 by molding the additives into particles and accommodating the additives in the additive cartridge 52a.

In addition, as the additive, a water retaining material that retains moisture can be used, and for example, particles of super absorbent polymer (SAP) can be used as an additive. In addition, the particles of porous ceramics may be used as a water retaining material. The water retaining materials may be added as an additive by the additive supply unit 52.

For example, the plant cultivation sheet S can be used as a medium capable of growing plants instead of soil, and in addition to this, by burying the sheet S in the soil, it is possible to give a fertilizer, a PH adjusting agent, and the like to the soil. Therefore, it becomes possible to eliminate the labor of soil improvement and easily performs the plant cultivation. In addition, in a case where the sheet S is long, it becomes possible to perform the larger-scale plant cultivation by burying the sheet S in the soil of the planter or field without cutting. (2) Insect Repellent Sheet

By using particles containing a pest repellent or an insecticide as an additive, the sheet S can be made as an insect repellent sheet. As the repellent or the insecticide, in addition to known medicine, natural materials, such as camphor tree flour or hinoki wood flour can be used. The repellent or the insecticide can also be mixed and used. (3) Deodorizing Sheet

As the additive, by using particles containing a deodorant, an odor adsorbent, or an odor decomposer, the sheet S can be made as a deodorizing sheet. As the odor adsorbent, for example, particles of activated carbon or porous ceramics can be used. As the odor decomposer, for example, titanium oxide can be used. The repellent or the odor adsorbent can also be mixed and used. (4) Dehumidifying Sheet

By using particles containing a hygroscopic material as an additive, the sheet S can be made as a dehumidifying sheet. As the hygroscopic material, for example, particles of a desiccant, such as silica gel, can be used. It is also possible to use a mixture of a plurality of types of hygroscopic materials. (5) Heat Insulating and Generating Sheet

By using particles containing a functional material that exhibits heat insulating properties or heat generating properties as an additive, the sheet S can be made as a heat insulating and generating sheet. For the type of functional material, for example, particles containing capsaicin (for example, red pepper powder) can be used. To the particles, particles of black silica may be mixed as an auxiliary material. In addition, it is also possible to use a mixture of the plurality of types of functional materials. (6) Moisturizing Sheet

By using particles containing a functional material having water containing properties as an additive, the sheet S can be made as a moisturising sheet. As the above-described functional material, for example, the SAP particles can be used. It is also possible to use a mixture of the plurality of types of functional materials. Further, particles containing the above-described functional material may be added as a functional material by the additive supply unit 52. (7) Aromatic Sheet

By using particles containing an aromatic material as an additive, the sheet S can be made as an aromatic sheet. As the aromatic material, in addition to known perfumes, natural materials, such as hinoki powder, can be used. It is also possible to use a mixture of the plurality of types of aromatic materials. Further, particles containing the above-described aromatic material may be added as a functional material by the additive supply unit 52.

(1) to (7) are merely examples, and the sheet S can be made as a functional sheet appropriate for various uses.

As illustrated in Fig. 12, the sheet S to be manufactured by the sheet manufacturing apparatus 100 has a plurality of layers, and a state where the densities or thicknesses are different for each layer can be achieved. For example, it is possible to achieve the first layer L1 having a high density in order to exhibit the strength of the sheet S, and to achieve the thick second layer L2 having a low density containing a component for exhibiting the functionality of the sheet S. In this case, the sheet S has the first layer L1 having sufficient strength or hardness and the second layer L2 molded with a low density such that the functional material can easily exhibit the function thereof. Therefore, it is possible to manufacture a highly functional sheet S having sufficient strength or hardness.

As described above, in the sheet manufacturing apparatus 100 according to a seventh embodiment to which the invention is applied, the sheet manufacturing apparatus 100 includes the first web forming unit 70A that forms the first web W2 based on the mixture MX1 containing fibers. The sheet manufacturing apparatus 100 includes the first processing unit 80A that performs processing including at least one of the pressurizing processing and the heating processing with respect to the first web W2 formed by the first web forming unit 70A. In addition, the second web forming unit 70B that laminates the mixture MX2 containing fibers on the first web W2 processed by the first processing unit 80A, and forms the second web W3, is provided. In addition, the second processing unit 80B that performs processing including at least one of the pressurizing processing and the heating processing with respect to the second web W3 formed by the second web forming unit 70B and molds the sheet, is provided.

The sheet manufacturing apparatus 100 corresponds to an apparatus for realizing the sheet manufacturing method of the invention. The processing of the first web forming unit 70A corresponds to first web forming, and the processing of the first processing unit 80A corresponds to first processing. The processing of the second web forming unit 70B corresponds to second web forming, and the processing of the second processing unit 80B corresponds to second processing.

According to the sheet manufacturing apparatus 100 to which the sheet manufacturing apparatus and the sheet manufacturing method of the invention are applied, in the process of manufacturing the sheet by laminating the layers configured with the first web W2 and the mixture MX2, it is possible to perform the pressurizing processing and/or the heating processing under different conditions for each layer. Therefore, it is possible to manufacture the sheet S having different states for each layer.

In addition, the sheet manufacturing apparatus 100 includes the intermediate cutting unit 91 that cuts the first web W2 processed by the first processing unit 80A, and the second web forming unit 70B processes the first web W2 cut by the intermediate cutting unit 91. The density of the first web W2 increases by performing the pressurizing processing by the first processing unit 80A, or the hardness increases by performing the heating processing. In the sheet manufacturing apparatus 100, the first web W2 of which the density and/or the hardness increases is cut by the intermediate cutting unit 91, the mixture MX2 is laminated, and the sheet S including the mixture MX2 is cut with the cutting unit 90. The configuration has an advantage that the disorder of the cut is small compared to a case of cutting the plurality of layers of the sheet S, and the quality of the sheet S can be enhanced.

In addition, since the first processing unit 80A includes the first pressurizing unit 82A which nips and pressurizes the first web W2, the first web W2 is nipped by the calendar rollers 85 in the first processing unit 80A, and accordingly, the pressurization can be reliably performed while transporting the first web W2. Further, by controlling the nip pressure of the calendar roller 85 in the first pressurizing unit 82A, the density of the first layer L1 can be easily adjusted.

In addition, since the first processing unit 80A includes the first heating unit 84A which nips and heats the first web W2, the first web W2 is nipped by the heating rollers 86, and accordingly, the heating can be reliably performed while transporting the first web W2.

In addition, since the second processing unit 80B includes the second pressurizing unit 82B which nips and pressurizes the second web W3, the second web W3 is nipped by the calendar rollers 85 in the second processing unit 80B, and accordingly, the pressurization can be reliably performed while transporting the second web W3. Further, by controlling the nip pressure of the calendar roller 85 in the second pressurizing unit 82B, the density of the sheet S can be easily adjusted.

In addition, since the second processing unit 80B includes the second heating unit 84B which heats the second web W3, the second web W3 is nipped by the heating rollers 86, and accordingly, the heating can be reliably performed while transporting the second web W3.

In addition, the first web forming unit 70A includes the first drum unit 61A that disperses the mixture MX1 in the air and the mesh belt 72A for depositing the mixture MX1 dispersed by the first drum unit 61A and forms the web. The second web forming unit 70B includes the second drum unit 61B that disperses the mixture MX2 in the air. The second web forming unit 70B includes the mesh belt 72B for depositing the mixture MX2 dispersed by the second drum unit 61B on the first web W2 processed by the first processing unit 80A and forms the second web W3. With the configuration, the multilayered sheet S having the layer of the mixture MX1 and the layer of the mixture MX2 can be easily manufactured.

### 8. Eighth Embodiment

Hereinafter, an eighth embodiment to which the invention is applied will be described.

Fig. 13 is a schematic configuration view of the sheet manufacturing apparatus 100A according to the eighth embodiment. The sheet manufacturing apparatus 100A includes the manufacturing unit 102A instead of the manufacturing unit 102 in the sheet manufacturing apparatus 100 (Fig. 11). The manufacturing unit 102A includes the processing roller 310 between the first humidity control unit 78A and the first processing unit 80A. In the eighth embodiment, the configurations common to those of the sheet manufacturing apparatus 100 of the seventh embodiment will be given the same reference numerals, and the description thereof will be omitted.

The processing roller 310 is positioned in a path through which the first web W2 of which the humidity is conditioned by the first humidity control unit 78A is transported, partially presses the first web W2, gives an uneven shape to the first web W2, and performs the so-called embossing.

The first web W2 embossed by the processing roller 310 is processed by the first processing unit 80A. The first processing unit 80A and the processing in each of the subsequent processes after the processing of the first processing unit 80A are the same as those in the seventh embodiment.

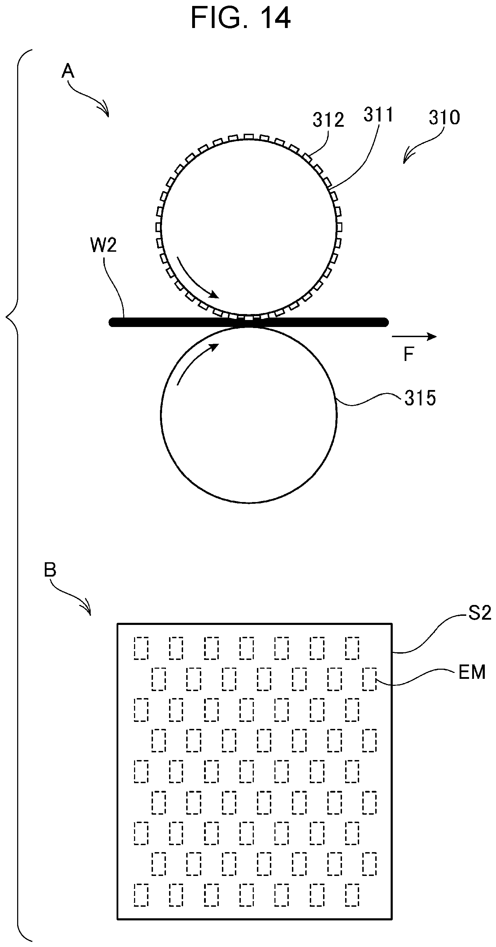

The processing roller 310 (pressing processing unit) is installed in the transport path of the first web W2 and includes the pair of roller 311 and roller 315 (see Fig. 14). The processing roller 310 nips and transports the first web W2 between the roller 311 and the roller 315 with a predetermined nip pressure and applies a pressing force to the first web W2. The processing roller 310 includes a pressure applying mechanism (not illustrated) for applying the nip pressure to the roller 311 and the roller 315 and a driving motor (not illustrated) for driving the roller 311 or the roller 315.

On the surface of the roller 311, the projection(s) 312 is formed. In contrast, the roller 315 is a cylindrical roller having a flat circumferential surface.

When the processing roller 310 nips and transports the first web W2, the first web W2 receives the nip pressure from the projection 312. Therefore, the first web W2 partially receives the pressing force, and a part of the first web W2 sinks. Accordingly, unevenness that corresponds to the shape of the projection 312 is formed on the first web W2. The processing roller 310 presses the first web W2 by the first processing unit 80A before being pressurized and heated and forms unevenness on the first web W2.

A sign B of Fig. 14 illustrates a plan view of a sheet S4 as an example of the sheet S manufactured by using the processing roller 310. The length L in Fig. 14 refers to the size of the sheet S2 in the transport direction F, and the width WI is the same as that in Fig. 12.

In the sheet S2, the recess portion EM that extends in the length L direction and the width WI direction is formed. Since the recess portion EM is a recess portion formed by the so-called embossing, the first web W2 is formed by pressing the first web S2 by the projection 312, and the part other than the recess portion EM is relatively projected, the sheet S4 has unevenness as a whole.

In this manner, the processing roller 310 functions as a pressing processing unit that partially presses the first web W2 and gives an uneven shape to the first web W2. The processing roller 310 can perform the embossing with respect to the sheet S by partially pressing the first web W2 by the projection 312. Therefore, it is possible to change the touch of the sheet S4, and it is possible to manufacture the sheet S having the uneven decoration.

In addition, the uneven shape formed on the first web W2 by the processing roller 310 is formed in the first layer L1 (Fig. 12) in the sheet S2. Therefore, the sheet manufacturing apparatus 100A can manufacture the sheet S2 having the first layer L1 having an uneven shape and the second layer L2 having no uneven shape. Therefore, it is possible to manufacture the sheet S2 in which the density and the like are different for each layer and the uneven shape is given to a layer.

In the eighth embodiment, a configuration in which one-side embossing is performed with respect to the first web W2 by using the roller 311 having the projection 312 and the roller 315 configured with a smooth surface is illustrated as an example, but a configuration in which double-side embossing is performed may be employed. In other words, the projection 312 may also be provided in the roller 315. Further, the shape of the projection 312 of the roller 311 is not limited to the example of Fig. 14, and a geometric pattern, a lattice shape, or any other shapes can be employed.

Further, in the eighth embodiment, a configuration in which the processing roller 310 is provided on the upstream side of the first processing unit 80A, but the processing roller 310 may be provided instead of the first pressurizing unit 82A of the first processing unit 80A. In addition, it is also possible to provide the processing roller 310 between the second humidity control unit 78B and the second processing unit 80B or instead of the second pressurizing unit 82B.

### 9. Ninth Embodiment

Hereinafter, a ninth embodiment to which the invention is applied will be described.

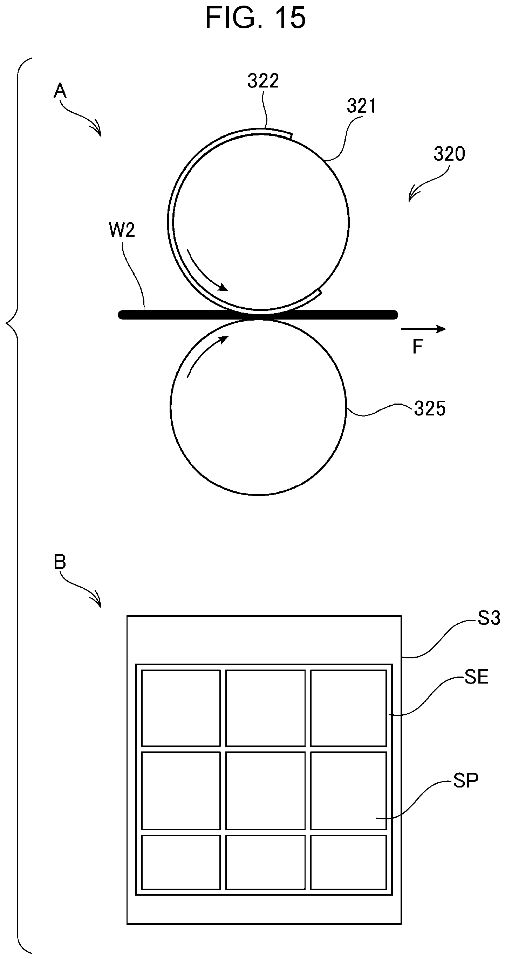

Fig. 15 is an explanatory view illustrating a configuration of the processing roller 320 according to the ninth embodiment. A sign A of Fig. 15 illustrates a side view illustrating a configuration of the processing roller 320, and a sign B of Fig. 15 illustrates a plan view of the sheet S3 to be manufactured with a configuration including the processing roller 320.

The processing roller 320 (pressing processing unit) is installed in the transport path of the first web W2 and includes the pair of roller 321 and roller 325. The processing roller 320 nips and transports the first web W2 between the roller 321 and the roller 325 with a predetermined nip pressure and applies a pressing force to the first web W2. The processing roller 320 includes a pressure applying mechanism (not illustrated) for applying the nip pressure to the roller 321 and the roller 325 and a driving motor (not illustrated) for driving the roller 321 or the roller 325.

On the surface of the roller 321, the projection(s) 322 is formed. In contrast, the roller 325 is a cylindrical roller having a flat circumferential surface.

When the processing roller 320 nips and transports the first web W2, the first web W2 receives the nip pressure from the projection 322. Therefore, the first web W2 partially receives the pressing force, and a part of the first web W2 sinks. Accordingly, unevenness that corresponds to the shape of the projection 322 is formed on the first web W2.

The processing roller 320 can be used instead of the calendar roller 85 of the first pressurizing unit 82A or the second pressurizing unit 82B of the sheet manufacturing apparatus 100 (Fig. 11).

Further, the processing roller 320 can be applied to the sheet manufacturing apparatus 100A instead of the processing roller 310 (Fig. 14).

A sign B of Fig. 15 illustrates a plan view of the sheet S3 as an example of the sheet S manufactured by using the processing roller 320. The length L and the width WI in Fig. 15 are the same as those in Fig. 14.

In the sheet S3, a lattice-like recess portion SE that extends in the length L direction and the width WI direction is formed. Since the recess portion SE is formed by pressing the first web W2 by the projection 322 and the part other than the recess portion SE is relatively projected, the sheet S2 has unevenness as a whole.

In this manner, the processing roller 320 functions as a pressing processing unit that partially presses the first web W2 and gives an uneven shape to the first web W2. The processing roller 320 can form the part SP defined by the recess portion SE on the sheet S3 by partially pressing the first web W2 by the projection 322. In addition, the projection 322 can also form the recess portion SE on the sheet S as a decoration.

In addition, the uneven shape formed on the first web W2 by the processing roller 320 is formed in the first layer L1 (Fig. 12) in the sheet S3. Therefore, the sheet manufacturing apparatuses 100 and 100A to which the processing roller 320 is applied can manufacture the sheet S2 having the first layer L1 having an uneven shape and the second layer L2 having no uneven shape. Therefore, it is possible to manufacture the sheet S2 in which the density and the like are different for each layer and the uneven shape is given to a layer. Similar considerations apply to the eighth and ninth embodiments as to the embodiments in Figs. 7 and 8.

### 10. Tenth Embodiment

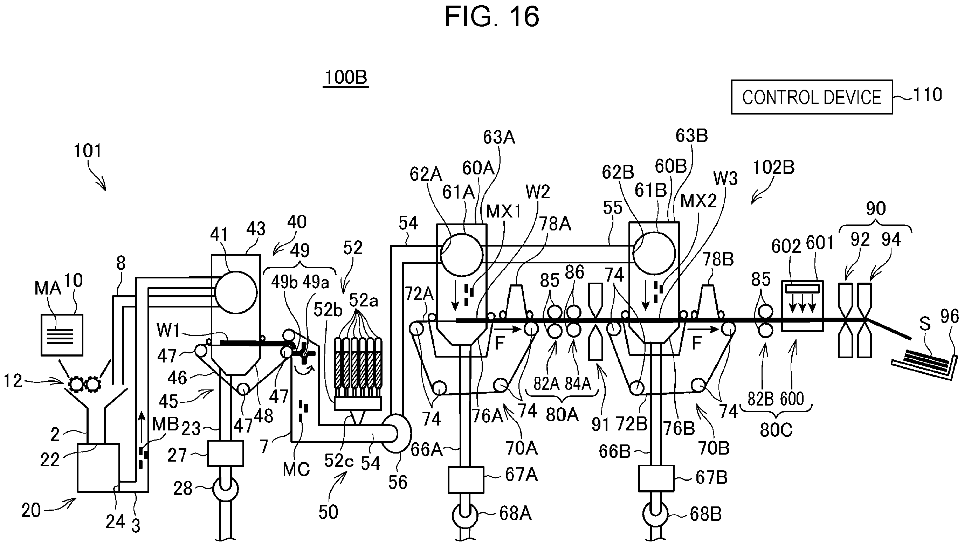

Fig. 16 is a schematic configuration view of the sheet manufacturing apparatus 100B according to a tenth embodiment. The sheet manufacturing apparatus 100B includes a manufacturing unit 102B instead of the manufacturing unit 102 in the sheet manufacturing apparatus 100 (Fig. 11). The manufacturing unit 102B has a configuration including a heating unit 600 instead of the second heating unit 84B.

In the tenth embodiment, the configurations common to those of the sheet manufacturing apparatus 100 of the seventh embodiment will be given the same reference numerals, and the description thereof will be omitted.

The heating unit 600 has a furnace 601 that accommodates the upper portion and preferably the lower portion of the transport path of the second web W3, and a heat source 602 accommodated in the furnace 601. The second web W3 passes through the inside of the furnace 601 between the second pressurizing unit 82B and the cutting unit 90.

The heat source 602 is an infrared heater that emits infrared rays for heating, or a heater having a heating element. As a heating element, a resistance heating element can be used, and for example, a nichrome wire heater or a ceramic heater can be adopted. The heating unit 600 heats the second web W3 in the furnace 601 by radiant heating by the infrared rays emitted from the heat source 602 or transfer heating by raising the temperature of the internal space of the furnace 601 by the heat source 602.

In addition, the furnace 601 may be configured to perform induction heating, dielectric heating, or heating of the second web W3 by a heat pump type heater. Further, without providing the furnace 601, the second web W3 may be heated by the heat source 602 disposed to be close to the transport path of the second web W3.

In addition, in the furnace 601, another heat source may be provided below the second web W3, that is, on a side opposite to the heat source 602 nipping the second web W3. The configuration is effective in a case where an infrared heater or a heating element heater is used as a heat source, and there is an advantage that the second web W3 can be heated from both surfaces.

The heating unit 600 is a non-pressurizing type heating unit that heats the second web W3 without pressurizing the second web W3. In another expression, a heat source 602 that is not in contact with the second web W3 is provided, and the second web W3 is heated in a non-contact manner.

In this manner, in the sheet manufacturing apparatus 100B, the sheet forming unit 80 includes the second pressurizing unit 82B that pressurizes the second web W3, and the heating unit 600 that heats, in a non-pressurized state, the second web W3 pressurized by the second pressurizing unit 82B. According to the sheet manufacturing apparatus 100B, after adjusting the density of the second web W3 by pressurizing using the second pressurizing unit 82B, the adjusted density of the second web W3 is maintained and the resin of the second web W3 is melted, and the sheet S can be manufactured. Therefore, the density of the sheet S can be finely adjusted with high accuracy, and the hardness or basis weight of the sheet S can be adjusted in any manner.

### 11. Eleventh Embodiment

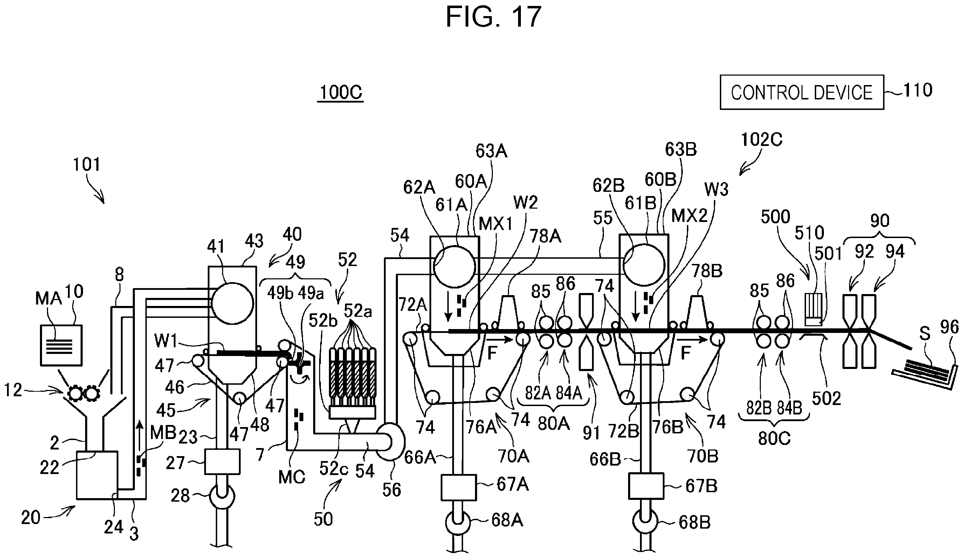

Fig. 17 is a schematic configuration view of the sheet manufacturing apparatus 100C according to an eleventh embodiment. The sheet manufacturing apparatus 100C includes a manufacturing unit 102C instead of the manufacturing unit 102 in the sheet manufacturing apparatus 100 (Fig. 11). The manufacturing unit 102C has a configuration in which the printing unit 500 is disposed between the second processing unit 80B and the cutting unit 90. In the eleventh embodiment, the configurations common to those of the sheet manufacturing apparatus 100 of the seventh embodiment will be given the same reference numerals, and the description thereof will be omitted.

The printing unit 500 includes the print head 501 that ejects ink toward the sheet S and the ink cartridge 510 that accommodates ink ejected by the print head 501. The print head 501 is capable of ejecting a plurality of colors of ink, and the ink cartridge 510 is provided corresponding to the number of colors of the ink ejected by the print head 501. Further, the printing unit 500 includes an ink supply mechanism (not illustrated) for supplying the ink from the ink cartridge 510 to the print head 501, a nozzle (not illustrated) for ejecting the ink from the print head 501, and the like.

In the printing unit 500, an image is formed by ejecting the ink onto the second web W3, that is, the sheet S processed into a sheet shape by the second processing unit 80B, by the print head 501. In the printing unit 500, for example, an image can be formed in the width direction (for example, the width WI direction in Fig. 13) of the sheet S by scanning the print head 501 in a direction intersecting the transport direction F. Further, the print head 501 may be a line inkjet head that extends in the width direction of the sheet S.

The second web W3 is pressurized and heated in the second processing unit 80B and becomes the sheet S, and an image is formed by the printing unit 500. After this, the sheet S is cut by the cutting unit 90 and is discharged to the discharge unit 96.

The printing unit 500 executes the printing under the control of the control device 110. The control device 110 drives the printing unit 500 according to image data for printing and forms an image. Further, the control device 110 can form an image at a set position on the sheet S cut into a predetermined size by controlling the printing position on the sheet S with reference to the position where the first cutting unit 92 cuts the sheet S.

As described above, the sheet manufacturing apparatus 100C includes the printing unit 500 that prints on the sheet S processed by the second processing unit 80B, and can form an image on the sheet S.

Further, in the examples (1) to (7) described for the use of the sheet S, the description of the use or the usage method of the sheet S may be printed by the printing unit 500. In this case, with respect to the sheet S containing the additives appropriate for a specific use, it is possible to notify the use or the usage method by the image formed on the sheet S.

### 12. Twelfth Embodiment

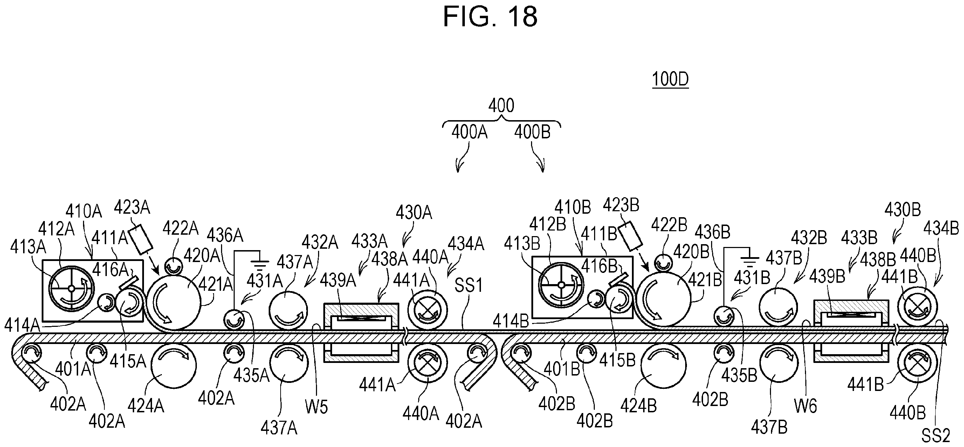

Fig. 18 is a schematic configuration view of a sheet manufacturing apparatus 100D according to a twelfth embodiment. In the twelfth embodiment, the configurations common to those of the sheet manufacturing apparatus 100 of the seventh embodiment will be given the same reference numerals, and the description thereof will be omitted.

The sheet manufacturing apparatus 100D includes the manufacturing unit 400 instead of the manufacturing unit 102 in the sheet manufacturing apparatus 100 (Fig. 11). In other words, the manufacturing unit 400 is provided instead of the first depositing unit 60A, the first web forming unit 70A, the first processing unit 80A, the second depositing unit 60B, the second web forming unit 70B, and the second processing unit 80B of the sheet manufacturing apparatus 100.

Further, the manufacturing unit 400 does not have a configuration that corresponds to the intermediate cutting unit 91 and the cutting unit 90, but these configurations may be provided in the manufacturing unit 400.

The manufacturing unit 400 includes the first manufacturing unit 400A and the second manufacturing unit 400B.

The first manufacturing unit 400A molds the mixture MX1 into a web shape by an electrostatic method and obtains the first web W5. In other words, the first manufacturing unit 400A electrostatically transfers the mixture MX1, which is a fiber-containing material, to the transport belt 401A that is a transfer object and forms the first web W5. The first manufacturing unit 400A manufactures an intermediate sheet SS1 by performing post-treatment for adjusting the surface properties of the first web W5.

In addition, the second manufacturing unit 400B deposits the mixture MX2 in a web shape on the intermediate sheet SS1 manufactured by the first manufacturing unit 400A by the antistatic method and obtains a second web W6. The second manufacturing unit 400B manufactures the intermediate sheet SS2 by performing post-treatment for adjusting the surface properties of the second web W6.

Since the sheet SS2 has an elongated shape, the cutting unit 90 is provided at the rear stage of the second manufacturing unit 400B, cuts the sheet SS2, and similar to the sheet S of the seventh embodiment, the sheet SS2 may be cut into a regular size.

The first manufacturing unit 400A includes the supply unit 410A that supplies the mixture MX1, the carrier 420A for carrying the supplied mixture MX1, the transport belt 401A on which the carried mixture MX1 is electrostatically transferred, and the processing unit 430 (430A) for post-treatment.

The mixture MX1 is common or similar to the seventh embodiment, and is a mixture of fibers derived from the raw material MA and additive(s) added by the additive supply unit 52. Further, the mixture MX1 may contain a charge control agent or an electrical charge control agent for obtaining charging properties of the mixture MX1.

The supply unit 410A accommodates the storage unit 412A, the agitator 413A, the roller 414A, the first carrier 415A, and the blade 416A, in the housing unit 411A.

The storage unit 412A stores the mixture MX1 therein. The agitator 413A agitates the mixture MX1 in the storage unit 412A and charges the mixture MX1 by friction during the agitation.

The mixture MX1 is supplied to the first carrier 415A by the rotation of the roller 414A. The first carrier 415A has a potential difference with the roller 414A, and the mixture MX1 electrostatically adheres by the potential difference. The blade 416A adjusts the thickness or the adhesion amount of the mixture MX1 that adheres to the first carrier 415A, adjusts the mixture MX1 into a sheet shape, and charges the mixture MX1 by friction.

The carrier 420A has a potential difference with the first carrier 415A, and the mixture MX1 electrostatically adheres to the carrier 420A. The carrier 420A is a rotating roller member, and transfers the mixture MX1 carried by the carrier 420A to the transport belt 401A.

Around the carrier 420A, the charging unit 422A that charges the outer circumferential surface 421A of the carrier 420A and the exposure unit 423A that adjusts the potential of the outer circumferential surface 421A are provided. Furthermore, around the carrier 420A, the transfer unit 424A is provided for transferring the mixture MX1 to the transport belt 401A by an electrostatic force generated by a potential difference with the carrier 420A.

The transfer unit 424A pressurizes the mixture MX1 transferred to the transport belt 401A with the carrier 420A and makes the thickness of the mixture MX1 uniform. Accordingly, the first web W5 having a uniform thickness is formed on the transport belt 401A. The carrier 420A and the transfer unit 424A configure the first web forming unit.

The transport belt 401A is configured with an endless belt and is transported by the plurality of rollers 402A. It is preferable that a surface of the transport belt 401A to which the mixture MX1 is transferred be made of a resin having medium to high resistance (volume resistivity of 107 to 1011 Ω·cm). As the type of resin, for example, a type in which the carbon black is kneaded in a fluorine-based resin can be used, but it is needless to say that other materials can also be used.

The first processing unit 430A includes a leveling processing unit 431A for smoothing the surface of the first web W5 and the pressurizing processing unit 432A for pressurizing the first web W5. Further, a semi-solidification processing unit 433A that semi-solidifies the surface of the first web W5 and a solidifying unit 434A for solidifying the first web W5 are provided. The leveling processing unit 431A has the leveling roller 435A of which at least the outer circumferential surface is made of metal, smooths the surface of the first web W5 by the leveling roller 435A, and removes electricity of the first web W5 via the ground wire 436A.

The pressurizing processing unit 432A bonds the fibers contained in the first web W5 and fibers and resin to each other by performing the pressurizing by the pressurizing roller 437A (first pressurizing roller), and makes the density uniform.

The semi-solidification processing unit 433A includes a chamber 438A configured with a heat insulating material and a heater 439A provided in the chamber 438A, and the surface of the first web W5 is semi-solidified by performing the heating by the heater 439A.

The solidifying unit 434A includes a solidifying roller 440A and a heater 441A provided in the solidifying roller 440A. The solidifying unit 434A heats the solidifying roller 440A by energizing the heater 441A and pressurizes the first web W5 while heating the first web W5 by the solidifying roller 440A. Accordingly, the resin contained in the first web W5 is melted, and the fibers and the resin of the first web W5 are bound to each other, and the intermediate sheet SS1 is formed. After the intermediate sheet SS1 passes through the solidifying roller 440A, for example, the intermediate sheet SS1 is naturally cooled, bound, and solidified.

A blowing fan (not illustrated) or the like for promoting the separation of the intermediate sheet SS1 from the transport belt 401A may be provided on the downstream side of the first processing unit 430A.

The second manufacturing unit 400B includes the supply unit 410B that supplies the mixture MX2, the carrier 420B for carrying the supplied mixture MX2, the transport belt 401B on which the carried mixture MX2 is electrostatically transferred, and the processing unit 430 (430B) for post-treatment.

The mixture MX2 is similar to the seventh embodiment, and is a mixture of fibers derived from the raw material MA and additives added by the additive supply unit 52. Further, the mixture MX2 may contain a charge control agent or an electrical charge control agent for obtaining charging properties of the mixture MX2. In addition, similar to the seventh embodiment, the mixture MX2 may contain the same components as those of the mixture MX1 or may contain different components.

Similar to the transport belt 401A, the transport belt 401B is configured with an endless belt and is transported by the plurality of rollers 402B. It is preferable that a surface of the transport belt 401B on which the intermediate sheet SS1 is mounted be made of a resin having medium to high resistance (volume resistivity of 107 to 1011 Ω·cm) as will be described later. As the type of resin, for example, a type in which the carbon black is kneaded in a fluorine-based resin can be used, but it is needless to say that other materials can also be used.

The transport belt 401B is disposed to be adjacent to the transport belt 401A of the first manufacturing unit 400A, and the intermediate sheet SS1 transported by the transport belt 401A is moved from the transport belt 401A to the transport belt 401B. The transport belt 401B transports the intermediate sheet SS1 from the upstream side of the supply unit 410B.

The supply unit 410B accommodates the storage unit 412B, the agitator 413B, the roller 414B, the first carrier 415B, and the blade 416B, in the housing unit 411B.

The storage unit 412B stores the mixture MX2 therein. The agitator 413B agitates the mixture MX2 in the storage unit 412B and charges the mixture MX2 by friction during the agitation.

The mixture MX2 is supplied to the first carrier 415B by the rotation of the roller 414B. The first carrier 415B has a potential difference with the roller 414B, and the mixture MX2 electrostatically adheres by the potential difference. The blade 416B adjusts the thickness or the adhesion amount of the mixture MX2 that adheres to the first carrier 415B, adjusts the mixture MX2 into a sheet shape, and charges the mixture MX2 by friction.

The carrier 420B has a potential difference with the first carrier 415B, and the mixture MX2 electrostatically adheres to the carrier 420B. The carrier 420B is a rotating roller member, and transfers the mixture MX2 carried by the carrier 420B to the transport belt 401B.

Here, the intermediate sheet SS1 manufactured by the first manufacturing unit 400A is placed on the transport belt 401B. Therefore, the web-like mixture MX2 carried by the carrier 420B is transferred onto the intermediate sheet SS1 on the transport belt 401B. Here, the second web W6 on which the intermediate sheet SS1 and the mixture MX2 are laminated is formed.

Around the carrier 420B, the charging unit 422B that charges the outer circumferential surface 421B of the carrier 420B and the exposure unit 423B that adjusts the potential of the outer circumferential surface 421B are provided. Furthermore, around the carrier 420B, the transfer unit 424B for transferring the mixture MX2 to the intermediate sheet SS1 on the transport belt 401B is provided by an electrostatic force generated by a potential difference with the carrier 420B.

The transfer unit 424B pressurizes the second web W6 including the mixture MX2 transferred to the transport belt 401B with the carrier 420B and makes the thickness uniform. Accordingly, the second web W6 having a uniform thickness is formed on the transport belt 401B. The carrier 420B and the transfer unit 424B configure the second web forming unit.

The second processing unit 430B includes the leveling processing unit 431B that smooths the surface of the second web W6 and a pressurizing processing unit 432B that pressurizes the second web W6. Further, the semi-solidification processing unit 433B for semi-solidifying the surface of the second web W6 and the solidifying unit 434B for solidifying the second web W6 are provided. The leveling processing unit 431B has the leveling roller 435B of which at least the outer circumferential surface is made of metal, smooths the surface of the second web W6 by the leveling roller 435B, and removes electricity of the second web W6 via the ground wire 436B.

The pressurizing processing unit 432B bonds the fibers contained in the second web W6 and fibers and resin to each other by performing the pressurizing by the pressurizing roller 437B (second pressurizing roller), and makes the density uniform.

The semi-solidification processing unit 433B includes the chamber 438B configured with a heat insulating material and the heater 439B provided in the chamber 438B, and the surface of the second web W6 is semi-solidified by performing the heating by the heater 439B.

The solidifying unit 434B includes the solidifying roller 440B and the heater 441B provided in the solidifying roller 440B. The solidifying unit 434B heats the solidifying roller 440B by energizing the heater 441B and pressurizes the second web W6 while heating the second web W6 by the solidifying roller 440B. Accordingly, the resin contained in the second web W6 is melted, and the fibers and the resin of the second web W6 are bound to each other, and the sheet SS2 is formed. After the sheet SS2 passes through the solidifying roller 440B, for example, the sheet SS2 is naturally cooled, bound, and solidified.

A blowing fan (not illustrated) or the like for promoting the separation of the sheet SS2 from the transport belt 401B may be provided on the downstream side of the second processing unit 430B.

Compared to the manufacturing unit 102 of the seventh embodiment, the manufacturing unit 400 does not require defibration, sorting, suctioning, web forming, and the like, and thus, the configuration of the sheet manufacturing apparatus 100D can be simplified. Therefore, there is an advantage that the manufacturing time of the sheet SS2 can be shortened, or the like.

In this manner, the sheet manufacturing apparatus 100D obtained by applying the manufacturing unit 400 to the sheet manufacturing apparatus 100, includes the carrier 420A and the transfer unit 424A as the first web forming unit that forms the first web W5 based on the mixture MX1 containing fibers. The sheet manufacturing apparatus 100D includes the first processing unit 430A that performs processing including at least one of the pressurizing processing and the heating processing with respect to the first web W5 formed by the carrier 420A and the transfer unit 424A. In addition, the carrier 420B and the transfer unit 424B that serve as the second web forming unit that laminates the mixture MX2 containing fibers on the first web W5 processed by the first processing unit 430A, and forms the second web W6, are provided. In addition, the second processing unit 430B that performs processing including at least one of the pressurizing processing and the heating processing and molding the sheet with respect to the second web W6 formed by the carrier 420B and the transfer unit 424B, is provided.

Accordingly, in a process of manufacturing a sheet by laminating the first web W5 and the mixture MX2, it is possible to perform the pressurizing processing and/or the heating processing under different conditions for each layer. Therefore, it is possible to manufacture the sheet SS2 having different states for each layer.

In addition, since the first processing unit 430A includes the pressurizing roller 437A for nipping and pressurizing the first web W5, in the first processing unit 430A, by nipping the first web W5 using the pressurizing roller 437A, it is possible to reliably perform the pressurizing while transporting the first web W5. Further, by controlling the nip pressure of the pressurizing roller 437A in the pressurizing processing unit 432A, the density of the first web W5 can be easily adjusted.

In addition, since the first processing unit 430A includes the semi-solidification processing unit 433A that heats the first web W5, the first web W5 can be reliably heated.

In addition, since the second processing unit 430B includes the pressurizing roller 437B for nipping and pressurizing the second web W6, in the second processing unit 430B, by nipping the second web W6 using the pressurizing roller 437B, it is possible to reliably perform the pressurizing while transporting the second web W6. Further, by controlling the nip pressure of the pressurizing roller 437B in the pressurizing processing unit 432B, the density of the sheet SS2 can be easily adjusted.

In addition, since the second processing unit 430B includes the semi-solidification processing unit 433B that heats the second web W6, the second web W6 can be reliably heated.

### 13. Other Embodiments

Each of the above-described embodiments is merely a specific aspect of implementing the invention described in the Claims, does not limit the invention, and can be implemented in various aspects as described below, for example, without departing from the scope of the invention.

For example, in the ninth embodiment, an example in which the processing roller 310 is applied to the sheet manufacturing apparatus 100 of the seventh embodiment and the sheet manufacturing apparatus 100A of the eighth embodiment has been described. The invention is not limited thereto, and the processing roller 310 can also be applied to the sheet manufacturing apparatus 100B of the tenth embodiment and the sheet manufacturing apparatus 100C of the eleventh embodiment. Further, the processing roller 310 may be applied to the sheet manufacturing apparatus 100D of the twelfth embodiment. The processing roller 320 is also similar thereto.

Further, the heating unit 600 described in the tenth embodiment is not limited to the sheet manufacturing apparatus 100B, and can also be applied to the sheet manufacturing apparatuses 100, 100A, 100C, and 100D.

In addition, the printing unit 500 described in the eleventh embodiment is not limited to the sheet manufacturing apparatus 100C, and can also be applied to the sheet manufacturing apparatuses 100, 100A, 100B, and 100D.

Furthermore, it is also possible to select and combine two or more of the processing rollers 310 and 320, the printing unit 500, and the heating unit 600, and to apply the combination to one sheet manufacturing apparatus 100, and 100A to 100D.

In addition, the sheet manufacturing apparatus 100 may be configured to manufacture not only the sheet S but also a board-like or web-like manufactured product configured with hard sheets or laminated sheets. In addition, the manufactured product is not limited to paper, but may be a nonwoven fabric. The nature of the sheet S is not particularly limited, may be paper which can be used as a recording paper (for example, a so-called PPC paper sheet) for the purpose of writing or printing, or may be wallpaper, wrapping paper, color paper, drawing paper, Kent paper or the like. In addition, in a case where the sheet S is a nonwoven fabric, in addition to a general nonwoven fabric, the sheet S may be fiber board, tissue paper, kitchen paper, cleaner, filter, liquid absorbing material, sound absorbing material, cushioning material, mat, and the like.

In addition, in the above-described embodiment, as the sheet manufacturing apparatus and the fiber raw material regenerating apparatus of the invention, the dry type sheet manufacturing apparatus 100 that manufactures the sheet S by obtaining the material by defibrating the raw material in the air and by using the material and the resin, has been described. The target of application of the invention is not limited thereto, and the invention can also be applied to a so-called wet type sheet manufacturing apparatus in which the raw material containing fibers is dissolved or suspended in a solvent, such as water, and the raw material is processed into a sheet. In addition, the invention can also be applied to an electrostatic type sheet manufacturing apparatus in which the material containing fibers defibrated in the air is adsorbed onto the surface of the drum by static electricity or the like and the raw material adsorbed onto the drum is processed into a sheet.

### 14. Thirteenth Embodiment

### 14-1. Overall Configuration of Sheet Manufacturing Apparatus

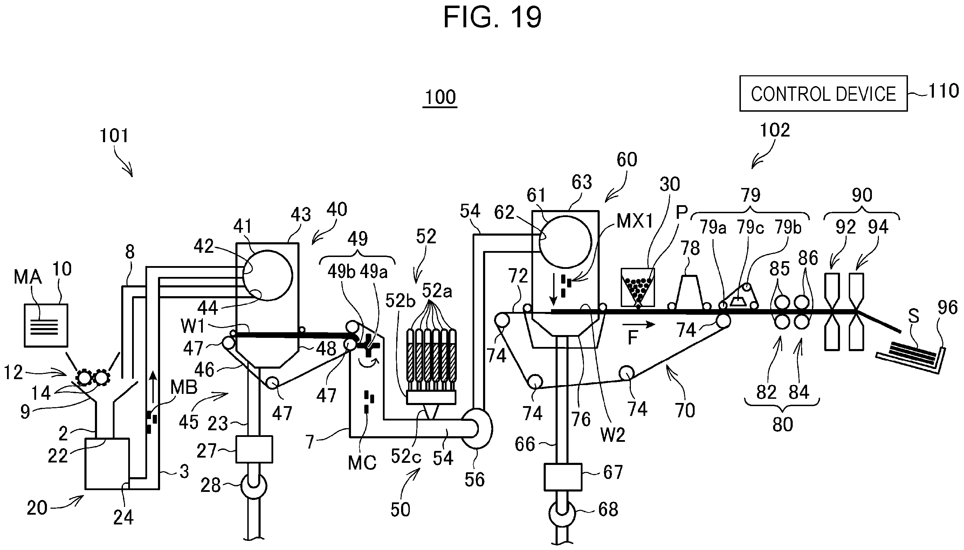

Fig. 19 is a schematic view illustrating a configuration of the sheet manufacturing apparatus 100.

The sheet manufacturing apparatus 100 executes regeneration processing of fiberizing the raw material MA containing fibers and regenerating the fiberized materials into the new sheet S. The sheet manufacturing apparatus 100 is capable of manufacturing the plurality of types of sheets S, and for example, by mixing additives with the fiberized raw material MA, according to the use, it is possible to adjust the bonding strength or whiteness of the sheet S, or to add a function of color, scent, flame retardancy and the like. Further, the sheet manufacturing apparatus 100 can adjust density, thickness, size, and shape of the sheet S. As a representative example of the sheet S, in addition to a sheet-like product, such as a printing paper sheet having a fixed size of A4 or A3, a cleaning sheet (for example, a floor cleaning sheet), an oil stain preventing sheet, or a toilet cleaning sheet, a shape of a paper tray and the like can be employed.

The sheet manufacturing apparatus 100 includes the supply unit 10, the coarse crushing unit 12, the defibrating unit 20, the sorting unit 40, the first web forming unit 45, the rotation body 49, the mixing unit 50, the depositing unit 60, the second web forming unit 70, a transport unit 79, the sheet forming unit 80, and the cutting unit 90. The coarse crushing unit 12, the defibrating unit 20, the sorting unit 40, and the first web forming unit 45 configure the defibration processing unit 101 which obtains the material of the sheet S by refining the raw material MA. In addition, the rotation body 49, the mixing unit 50, the depositing unit 60, the second web forming unit 70, the sheet forming unit 80, and the cutting unit 90 configure the manufacturing unit 102 that manufactures the sheet S by processing the material obtained by the defibration processing unit 101. In addition, the sheet manufacturing apparatus 100 includes the control device 110 that controls each portion of the sheet manufacturing apparatus 100.

The supply unit 10 is an automatic charging device that accommodates the raw material MA therein and continuously charges the raw material MA into the coarse crushing unit 12. For example, the supply unit 10 includes a stocker (not illustrated) for stacking the raw material MA and a feeder (not illustrated) for feeding the raw material MA from the stocker. The raw material MA may be a material that contains fibers, for example, waste paper, discarded paper, or pulp sheet.

The coarse crushing unit 12 includes a coarse crushing blade 14 for cutting the raw material MA supplied by the supply unit 10, and the raw material MA is cut in the air by the coarse crushing blade 14 into strips of several cm square. The shape or the size of the strips is any shape or size. As the coarse crushing unit 12, for example, a shredder can be used. The raw material MA cut by the coarse crushing unit 12 is collected by a hopper 9 and transported to the defibrating unit 20 via the pipe 2.

The defibrating unit 20 defibrates the coarse debris cut by the coarse crushing unit 12. Defibration is a process of disentangling the raw material MA in a state where a plurality of fibers are bound to each other into one or a small number of fibers. The raw material MA can also be called a defibrated material.

By defibrating the raw material MA by the defibrating unit 20, in addition to the effect of disentangling the fibers contained in the raw material MA, it is possible to expect an effect of separating substances, such as resin particles, ink, toner, blot preventing agent and the like that adhere to the raw material MA, from the fibers. The material that passes through the defibrating unit 20 is called a defibrated material MB.

In addition to the disentangled fibers, the defibrated material MB contains inclusions separated from the fiber when the defibrating unit 20 disentangles the fibers. The inclusions are particles or powder, and the components of the inclusions are, for example, resin particles contained in the raw material MA, colorants, such as ink and toner, or additives, such as blot preventing material or paper strength enhancing agent. The resin particle is a resin mixed for binding a plurality of fibers to each other at the time of manufacturing the raw material MA. The shape of the fiber contained in the defibrated material MB is, for example, a string shape or a ribbon shape. The fibers contained in the defibrated material MB may exist in an independent state while not being intertwined with other fibers, or may exist in a state of forming a so-called "dummy" while being in a form of a mass intertwined with other fibers.

The defibrating unit 20 performs defibration by a dry process. The dry process indicates processing in the air, such as atmosphere, rather than in liquid. The defibrating unit 20 can be configured, for example, by using a defiberizer, such as an impeller-mill. Specifically, the defibrating unit 20 includes a rotor (not illustrated) that rotates and a liner (not illustrated) positioned at an outer circumference of the rotor (not illustrated), and defibrates the coarse debris nipping the coarse debris between the rotor and the liner.

From the coarse crushing unit 12 to the defibrating unit 20, the coarse debris is transported by an airflow. A configuration in which the airflow is generated by the defibrating unit 20 may be employed, or the airflow may be generated by providing a blower (not illustrated) on an upstream side or a downstream side of the defibrating unit 20 in the transport direction of the coarse debris or the defibrated material MB. In addition, the defibrated material MB is sent from the defibrating unit 20 through the pipe 3 to the sorting unit 40 by the airflow. The airflow by which the defibrated material MB is transported to the sorting unit 40 may be generated by the defibrating unit 20, or the airflow of the above-described blower may be used.

The sorting unit 40 sorts the components contained in the defibrated material MB according to the size of the fibers. The size of the fiber mainly refers to the length of the fiber.

The sorting unit 40 has the drum unit 41 and the housing unit 43 that accommodates the drum unit 41 therein. The drum unit 41 is a member that functions as a sieve, and includes, for example, a mesh, a filter, a screen, and the like that function as a sieve having an opening. The drum unit 41 has a cylindrical shape that is rotationally driven by a motor, and at least a part of a circumferential surface is a mesh. The mesh of the drum unit 41 is configured with wire gauze, expanded metal obtained by stretching a metal plate having cuts, punching metal, and the like. The defibrated material MB introduced into the drum unit 41 from the introduction port 42 is divided into a passing material that passes through the opening of the drum unit 41 and a residue that does not pass through the opening, due to the rotation of the drum unit 41. The passing material that has passed through the opening contains fibers or particles smaller than the opening, and the fibers or particles are called a first sorted material. The residue contains fibers, undefibrated pieces, or dams larger than the opening, and the fibers, undefibrated pieces, or dams are called a second sorted material. The first sorted material descends inside the housing unit 43 toward the first web forming unit 45. The second sorted material is sent to the defibrating unit 20 as described above.

Instead of the sorting unit 40, the sheet manufacturing apparatus 100 may include a classifier that separates the first sorted material and the second sorted material. The classifier is, for example, a cyclone classifier, an elbow jet classifier, and an eddy classifier. The classifier may be configured to separate smaller components or components having a low density among the components contained in the first sorted material. For example, from the first sorted material, it is possible to adopt a configuration in which resin particles, colorants, or additives which are pulled off from the fibers by the defibrating unit 20 are separated by the classifier and removed. In this case, the first sorted material can be transported to the first web forming unit 45 or the mixing unit 50 in a state where fine particles, such as resin particles, colorants, and additives, are removed.

The first web forming unit 45 includes the mesh belt 46, the stretching roller 47, and the suction unit 48. The mesh belt 46 is an endless metal belt, and is stretched over the plurality of stretching rollers 47. The mesh belt 46 circulates around a track configured with the stretching roller 47. A part of the track of the mesh belt 46 is flat under the drum unit 41, and the mesh belt 46 configures a flat surface.

Multiple openings are formed in the mesh belt 46. Components larger than the opening of the mesh belt 46 in the first sorted material that descends from the drum unit 41 positioned above the mesh belt 46 are deposited on the mesh belt 46. In addition, components smaller than the opening of the mesh belt 46 in the first sorted material pass through the opening. The components that pass through the opening of the mesh belt 46 are called a third sorted material.

The suction unit 48 is connected to the first dust collecting unit 27 via the pipe 23. The first dust collecting unit 27 includes a filter (not illustrated) for separating the third sorted material from the airflow, and the first collection blower 28 is installed on the downstream side of the first dust collecting unit 27. The first collection blower 28 suctions the air from the first dust collecting unit 27. Due to a suctioning force of the first collection blower 28, the third sorted material that has passed through the opening of the mesh belt 46 is sent from the suction unit 48 to the first dust collecting unit 27, and is collected by the filter of the first dust collecting unit 27. Since the first sorted material that descends from the drum unit 41 is attracted to the mesh belt 46 by the airflow suctioned by the suction unit 48, there is an effect of promoting the deposition.

The component deposited on the mesh belt 46 has a web shape and configures the first web W1. In other words, the first web forming unit 45 forms the first web W1 from the first sorted material sorted by the sorting unit 40. The first web W1 has fibers larger than the opening of the mesh belt 46 among the components contained in the first sorted material as the main components thereof, and is formed in a state of being soft and bulging containing a large amount of air. The first web W1 is transported to the rotation body 49 according to the movement of the mesh belt 46.

The rotation body 49 includes the base portion 49a connected to a driving unit (not illustrated), such as a motor and the protrusion portion 49b that protrudes from the base portion 49a, and as the base portion 49a rotates in the direction R, the protrusion portion 49b rotates around the base portion 49a. The protrusion portion 49b has, for example, a plate-like shape. In the example of Fig. 19, four protrusion portions 49b are provided at equivalent intervals on the base portion 49a.

The rotation body 49 is positioned in the end portion of the flat part of the track of the mesh belt 46. In the end portion, since the track of the mesh belt 46 is bent downward, the mesh belt 46 bends downward and moves. Therefore, the first web W1 transported by the mesh belt 46 protrudes from the mesh belt 46 and comes into contact with the rotation body 49. The first web W1 is disentangled by the collision of the protrusion portion 49b with the first web W1 and becomes a small fiber mass. The mass is transported through the pipe 7 positioned below the rotation body 49 to the mixing unit 50. Since the first web W1 has a soft structure formed by depositing fibers on the mesh belt 46 as described above, the web-like material W1 is easily divided when colliding with the rotation body 49.

The material obtained by dividing the first web W1 by the rotation body 49 is called a material MC. The material MC is obtained by removing the third sorted material from the above-described first sorted material, and the main component thereof is fiber.

The position of the rotation body 49 is a position where the protrusion portion 49b can come into contact with the first web W1 and is provided at a position where the protrusion portion 49b does not come into contact with the mesh belt 46. It is preferable that the distance between the protrusion portion 49b and the mesh belt 46 at the position where the protrusion portion 49b and the mesh belt 46 are closest to each other, for example, be 0.05 mm or more and 0.5 mm or less.

The mixing unit 50 mixes the material MC and additives with each other. The mixing unit 50 includes the additive supply unit 52 that supplies the additives, the pipe 54 for transporting the material MC and the additive, and the mixing blower 56.

In the additive supply unit 52, the additive cartridge 52a that stores the additives therein is set. The additive cartridge 52a may be attachable to and detachable from the additive supply unit 52. The additive supply unit 52 includes the additive extracting unit 52b that extracts the additive from the additive cartridge 52a and the additive charging unit 52c that discharges the additive extracted by the additive extracting unit 52b to the pipe 54. The additive extracting unit 52b includes a feeder (not illustrated) for sending out the additive containing fine powder or fine particles which are in the additive cartridge 52a, and extracts the additive from (some or all of) the additive cartridge(s) 52a. The additive extracted by the additive extracting unit 52b is sent to the additive charging unit 52c. The additive charging unit 52c accommodates the additive extracted by the additive extracting unit 52b. The additive charging unit 52c includes a shutter (not illustrated) which can be opened and closed in a connecting portion with the pipe 54, and by opening the shutter, the additive extracted by the additive extracting unit 52b is sent out to the pipe 54.

The additive to be supplied from the additive supply unit 52 contains a resin (binder) for binding a plurality of fibers to each other. The resin contained in the additive is melted by being heated to a temperature equal to or higher than a glass transition point in the manufacturing unit 102 and binds the fibers of the material MC to each other. Examples of the resin include AS resin, ABS resin, polypropylene, polyethylene, polyvinyl chloride, polystyrene, acrylic resin, polyester resin, polyethylene terephthalate, polyphenylene ether, polybutylene terephthalate, nylon, polyamide, polycarbonate, polyacetal, polyphenylene sulfide, polyether ether ketone, and the like. These resins may be used alone or as a mixture thereof.

The additive to be supplied from the additive supply unit 52 may contain a component other than the resin for binding the plurality of fibers to each other. For example, in accordance with the type of the sheet S to be manufactured, a coloring agent for coloring the fibers, a coagulation preventing agent for suppressing coagulation of fibers or coagulation of resins, a flame retardant for making fibers and the like unlikely to burn, and the like, may be included. Further, the additive may be in a form of fiber or powder.

The mixing blower 56 generates the airflow to the pipe 54 connected to the pipe 7 and the depositing unit 60. The material MC and the additives to be supplied to the pipe 54 by the additive supply unit 52 are mixed with each other when passing through the mixing blower 56. For example, the mixing blower 56 can be configured to include a motor (not illustrated), a blade (not illustrated) that is driven by the motor and rotates, and a case (not illustrated) that accommodates the blade therein, and may have a configuration in which the blade and the case are connected to each other. Further, the mixing blower 56 may include a mixer that mixes the material MC and the additives with each other in addition to the blade that generates the airflow. The mixture mixed by the mixing unit 50 is transported to the depositing unit 60 and introduced to the introduction port 62 of the depositing unit 60, by the airflow generated by the mixing blower 56. Here, the mixture introduced into the introduction port 62 is indicated by the symbol MX1.

The depositing unit 60 has a drum unit 61 and a housing unit 63 that accommodates the drum unit 61 therein. The drum unit 61 is, for example, a cylindrical structure configured similarly to the drum unit 41, rotates by the power of a motor (not illustrated) similarly to the drum unit 41, and functions as a sieve. The drum unit 61 (dispersing unit) has an opening, and the mixture MX1 (first material) disentangled by the rotation of the drum unit 61 descends from the opening.

The second web forming unit 70 (web forming unit) is disposed below the drum unit 61. The second web forming unit 70 includes, for example, the mesh belt 72, the stretching roller 74, and the suction mechanism 76.

The mesh belt 72 (depositing unit) is configured with an endless metal belt similar to the mesh belt 46, and is stretched over the plurality of stretching rollers 74. The mesh belt 72 circulates around a track configured with the stretching rollers 74. A part of the track of the mesh belt 72 is flat under the drum unit 61, and the mesh belt 72 configures a flat surface. In addition, multiple openings are formed in the mesh belt 72.

Components larger than the opening of the mesh belt 72 in the mixture MX1 that descends from the drum unit 61 positioned above the mesh belt 72 are deposited on the mesh belt 72. In addition, components smaller than the opening of the mesh belt 72 in the mixture MX1 pass through the opening.

The suction mechanism 76 suctions the air from the side opposite to the drum unit 61 with respect to the mesh belt 72.

The suction mechanism 76 is connected to the pipe 66. The pipe 66 is connected to the second collection blower 68 via the second dust collecting unit 67. The second dust collecting unit 67 includes a filter (not illustrated) that collects particles or fibers that have passed through the mesh belt 72. The second collection blower 68 is a blower that suctions the air through the pipe 66. The suction mechanism 76 suctions the air from below the mesh belt 72 by the suction force of the second collection blower 68 and collects the particles or fibers contained in the suctioned air by the second dust collecting unit 67. The airflow suctioned by the second collection blower 68 has an effect of attracting the mixture MX1 that descends from the drum unit 61 to the mesh belt 72 and promoting the deposition. In addition, the suctioned airflow of the second collection blower 68 forms a downflow in a path where the mixture MX1 falls from the drum unit 61, and it is also possible to expect an effect of preventing the fibers from being intertwined while falling. The mixture MX1 deposited on the mesh belt 72 has a web shape in the flat portion of the mesh belt 72 and configures the second web W2 (web).

In the transport path of the mesh belt 72, a solid feeder 30 (second material disposing unit) is disposed on the downstream side of the depositing unit 60. The solid feeder 30 stores a solid material P that serves as the second material that configures the sheet S, and discharges the solid material P toward the second web W2. The solid material P is disposed on the second web W2 deposited by the depositing unit 60 by the solid feeder 30.

The configuration of the solid feeder 30 will be described later.

In the transport path of the mesh belt 72, the humidity control unit 78 is provided on the downstream side of the solid feeder 30. The humidity control unit 78 is a mist type humidifier that supplies water to the mesh belt 72 in a form of mist. The humidity control unit 78 includes, for example, a tank for storing the water and an ultrasonic vibrator for making the water mist. Since a moisture content of the second web W2 is adjusted by the mist to be supplied by the humidity control unit 78, it is possible to expect an effect of suppressing adsorption or the like of the fibers onto the mesh belt 72 due to static electricity.

The second web W2 is peeled off from the mesh belt 72 by the transport unit 79 and transported to the sheet forming unit 80. The transport unit 79 includes, for example, a mesh belt 79a, a roller 79b, and a suction mechanism 79c. The suction mechanism 79c includes a blower (not illustrated), and generates an upward airflow through the mesh belt 79a by the suction force of the blower. With the airflow, the second web W2 is separated from the mesh belt 72 and is adsorbed to the mesh belt 79a. The mesh belt 79a is moved by the rotation of the roller 79b and transports the second web W2 to the sheet forming unit 80. Such a transport unit 79 can also be used in all the other embodiments.

The sheet forming unit 80 pressurizes and heats the second web W2 and forms the sheet S. The sheet forming unit 80 includes the pressurizing unit 82 that pressurizes the second web W2 and a heating unit 84 that heats the second web W2 pressurized by the pressurizing unit 82. The pressurizing unit 82 is configured with the pair of calendar rollers 85 and 85. The pressurizing unit 82 is connected to a press mechanism (not illustrated) for applying a nip pressure to the calendar rollers 85 and 85 by a hydraulic pressure and a driving unit (not illustrated), such as a motor for rotating the calendar rollers 85 and 85 toward the heating unit 84. The pressurizing unit 82 pressurizes the second web W2 with a predetermined nip pressure by the calendar rollers 85 and 85, and transports the second web W2 toward the heating unit 84.

The heating unit 84 includes the pair of heating rollers 86 and 86. The heating unit 84 includes a heater (not illustrated) for heating the circumferential surface of the heating roller 86 to a predetermined temperature and a driving unit (not illustrated), such as a motor for rotating the heating rollers 86 and 86 toward the cutting unit 90. The heating unit 84 applies the heat while nipping the second web W2 of which the density has increased by the pressurizing unit 82, and transports the second web W2 to the cutting unit 90. The temperature at which the heating unit 84 heats the second web W2 can be set to any temperature, but it is desirable that the second web W2 is heated until the resin contained in the mixture MX1 reaches a temperature equal to or higher than the glass transition point. In this case, the resin contained in the second web W2 is melted and binds the fibers and the fibers to each other, and accordingly, the sheet S is formed.

The cutting unit 90 cuts the sheet S formed by the sheet forming unit 80. The cutting unit 90 includes the first cutting unit 92 that cuts the sheet S in the direction intersecting the transport direction indicated by the symbol F, and the second cutting unit 94 that cuts the sheet S in the direction parallel to the transport direction F. The cutting unit 90 cuts the length and the width of the sheet S to a predetermined size and forms a single-cut sheet S. The sheet S cut by the cutting unit 90 is accommodated in the discharge unit 96. The discharge unit 96 includes a tray or a stacker for accommodating the manufactured sheet therein, and the user can take out and use the sheet S discharged onto the tray.

Each portion of the sheet manufacturing apparatus 100 configures the defibration processing unit 101 and the manufacturing unit 102. The defibration processing unit 101 includes at least the defibrating unit 20 and may include the sorting unit 40 and the first web forming unit 45. The defibration processing unit 101 manufactures the defibrated material from the raw material MA and the first web W1 in which the defibrated material is formed in a web shape. The manufactured product of the defibration processing unit 101 is not only transported to the mixing unit 50 via the rotation body 49, but can also be stored after being extracted from the sheet manufacturing apparatus 100 without being transferred to the rotation body 49. Further, the manufactured product may be enclosed in a predetermined package and may be in a form that can be transported and traded.

The manufacturing unit 102 is a functional unit that reproduces the manufactured product manufactured by the defibration processing unit 101 on the sheet S. The manufacturing unit 102 includes the mixing unit 50, the depositing unit 60, the second web forming unit 70, the transport unit 79, the sheet forming unit 80, and the cutting unit 90, and may include the rotation body 49. In addition, the additive supply unit 52 may be included.

In the sheet manufacturing apparatus 100, the defibration processing unit 101 and the manufacturing unit 102 may be formed integrally or may be configured separately.

The control device 110 controls each portion of the sheet manufacturing apparatus 100 and executes the manufacturing of the sheet S. For example, the control device 110 executes a starting sequence when starting the manufacturing of the sheet S. In the starting sequence, the supply unit 10, the coarse crushing unit 12, the defibrating unit 20, the sorting unit 40, the first web forming unit 45, the rotation body 49, the additive supply unit 52, the mixing blower 56, the depositing unit 60, the second web forming unit 70, the sheet forming unit 80, and the cutting unit 90 are sequentially started. The control device 110 controls the operation rotational speed in the coarse crushing blade 14, the defibrating unit 20, the sorting unit 40, the first web forming unit 45, the rotation body 49, the depositing unit 60, the second web forming unit 70, the sheet forming unit 80, controls the cutting timing by the cutting unit 90, and the like. Further, the control device 110 controls the start and stop of each of the blowers including the first collection blower 28, the mixing blower 56, the second collection blower 68, and the blower (not illustrated) of the suction mechanism 79c. In addition, the control device 110 controls humidification by the humidity control unit 78. Further, the control device 110 controls the solid feeder 30.

### 14-2. Structure of Solid Feeder

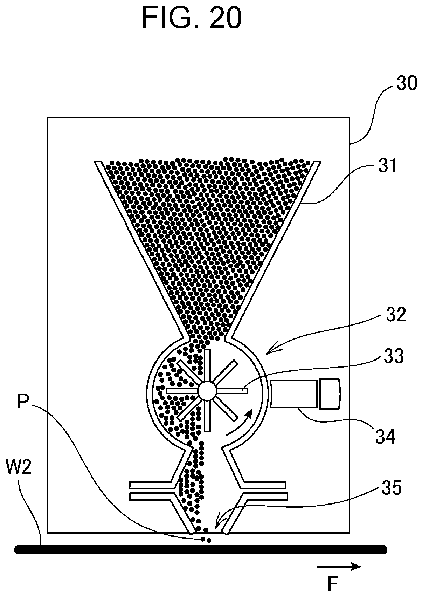

Fig. 20 is a side view of a main part side of the solid feeder 30. The solid feeder 30 includes a hopper 31 for storing the solid material P therein and a rotary valve 32 disposed below the hopper 31. The rotary valve 32 includes a rotor 33 and a driving motor 34 that rotates the rotor 33 in the direction indicated by the arrow in the drawing.

The hopper 31 can accommodate the solid material P which is a powder, particles, or solid material having other shapes. The solid material P is disposed on the second web W2, is a material that configures the sheet S, and has any size or shape. The solid material P may be, for example, a material other than the fibers and additives contained in the mixture MX1, or may be a solid material larger than the particles of the additive contained in the mixture MX1. In addition, the solid material P is not limited to one type, and the plurality of types of solid materials may be mixed and stored in the hopper 31. Further, the solid feeder 30 may include a supply device (not illustrated) for supplying the solid material P from the outside of the solid feeder 30 to the hopper 31.

The rotary valve 32 is a hollow valve, and the rotor 33 rotates by the power of the driving motor 34, and accordingly, the solid material P is extracted from the lower portion of the hopper 31 and sent out downward. A discharge port 35 is opened below the rotary valve 32. The solid material P delivered from the hopper 31 by the rotation of the rotor 33 drops to the upper surface of the second web W2 through the discharge port 35. Accordingly, the solid material P is disposed on the surface of the second web W2.

The disposition of the discharge port 35 in the width direction of the second web W2, that is, the direction orthogonal to the transport direction F is any disposition, but the solid feeder 30 can dispose the solid material P at a plurality of positions in the width direction or along the whole width of the second web W2. For example, the rotor 33 and the discharge port 35 can have a shape that extends in the width direction of the second web W2. Further, a plurality of discharge ports 35 may be disposed side by side along the width direction of the second web W2.

The driving motor 34 operates under the control of the control device 110. Therefore, for example, the timing of the operation of the driving motor 34 can be controlled by the control device 110. In a case where the driving motor 34 rotates at all times, the solid feeder 30 can uniformly dispose the solid material P on the entire surface of the second web W2. Further, in a case where the driving motor 34 rotates intermittently, the solid material P can be disposed at a constant interval in the transport direction F on the surface of the second web W2. The control device 110 controls the timing at which the solid feeder 30 discharges the solid material P and the timing at which the first cutting unit 92 cuts the sheet S, the position at which the solid material P is disposed and the cutting position of the sheet S in the transport direction F can be positioned. For example, it is possible to realize a configuration in which one row of solid materials P is arranged on one sheet S.

### 14-3. Manufacturing Example of Sheet

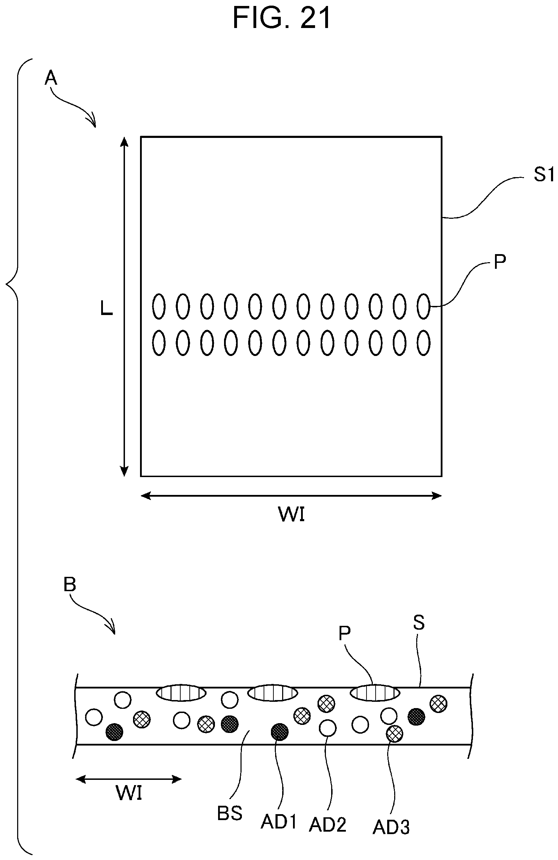

Fig. 21 is a view illustrating the sheet S1 as an example of the sheet S manufactured by the sheet manufacturing apparatus 100. A sign A of Fig. 21 illustrates a plane view of the sheet S1, and a sign B of Fig. 21 illustrates a sectional view of the sheet S1.

The sheet S1 is an example of the sheet S cut into a predetermined size by the cutting unit 90. In the plane view of the sheet S1, the length of the sheet S1 is indicated by L, and the width is indicated by W1. The length L is the size in the transport direction F, and the width WI is the size in the direction orthogonal to the transport direction F. By adjusting the timing at which the control device 110 and the first cutting unit 92 cut the sheet S, the length L can be adjusted. The width WI is determined by the position of the blade of the second cutting unit 94.

In the sheet S1, the solid materials P are disposed side by side in the width WI direction. The solid material P is disposed in two rows in the length L direction. The position of the solid material P in the length L direction can be adjusted as the control device 110 controls the driving motor 34.

As illustrated in B of Fig. 21, the sheet S1 is in a state where the solid material P is embedded in the surface of a base sheet BS configured by binding the fibers to the additive resin. It is considered that the solid material P is exposed on the surface of the sheet S1, but a state of being completely embedded in the sheet S1 is also achieved. For example, in a case where the density of the fibers in the second web W2 is low density and the bulk of the fibers is high, and in a case where the specific gravity of the solid material P is large, there is a case where the solid material P sediments inside the second web W2. In such a case, in the sheet S formed by the sheet forming unit 80, a state where the solid material P is completely embedded is also achieved.

Here, in a case where a material other than a resin that functions as a binder is used as an additive to be added by the additive supply unit 52, as illustrated in a step surface view of Fig. 21, the additives exist being dispersed in the base sheet BS. For example, in a case where the additive supply unit 52 adds a resin that functions as a binder and additives AD1, AD2, and AD3 other than the resin, as illustrated in Fig. 21, the particles of the additive AD1, AD2, and AD3 are respectively scattered in the sheet S1. The additives AD1, AD2, and AD3 are not limited to the colorants, and functional materials can be used. In addition, the number of types of additives added by the additive supply unit 52 is not limited, and five or more types of additives can be added by the additive supply unit 52.

### 14-4. Application Example of Sheet

A specific example of the combination of the use of the sheet Sand the additive added by the additive supply unit 52 will be presented.

### (1) Plant Cultivation Sheet

As a using method of the sheet S containing the solid material P, an example of using the sheet S as a tool for cultivating plants can be employed.

In the example, the seeds of plant are used as the solid material P. For example, it is preferable to dispose approximately 1 to 10 solid materials P along the width WI in one sheet S1. Further, the solid material P may be disposed in a plurality of rows in the length L direction.

In a case of using the sheet S1 as a plant cultivation sheet, as the additive added by the additive supply unit 52, the materials used for plant cultivation are used.

Specifically, a particulate fertilizer can be used as an additive. As the fertilizer, nitrogenous fertilizers, such as ammonium sulfate, ammonium chloride, ammonium nitrate and the like, can be employed. In addition, particles of phosphatic fertilizer, such as superphosphate, concentrated superphosphate, or fused phosphate, may be used. In addition, particles of potassium fertilizer, such as potassium chloride or potassium nitrate may be used. Further, among the particles of the fertilizer, the plurality of types of particles may be used as additives, or a mixture of the plurality of types of particles of the fertilizer may be used as an additive. In addition, in a case where the sheet S1 is used as the plant cultivation sheet, as an additive, a pH adjusting agent may be used, and for example, particles, such as organic lime, vegetable ash, quicklime, hydrated lime, and the like can be adopted.

The additives can be dispersed and disposed inside the sheet S1 as illustrated as the additives AD1, AD2 and AD3 in Fig. 21 by molding the additives into particles and accommodating the additives in the additive cartridge 52a.

In addition, as the additive, a water retaining material that retains moisture can be used, and for example, particles of super absorbent polymer (SAP) can be used as an additive. In addition, the particles of porous ceramics may be used as a water retaining material. The water retaining materials may be added as additives by the additive supply unit 52, but can also be contained in the solid material P. For example, the solid feeder 30 may be configured to discharge the solid material P mixed with the particles of the water retaining material and the seeds of the plant. Further, by using the particles containing bacteria of mushrooms as the solid material P instead of the seeds of plants, the sheet S1 can be used as a sheet for cultivation of the fungus bed.

The cultivation of the plants is possible, for example, by burying the plant cultivation sheet configured as described above in the soil as it is. In addition, when the sheet S1 contains a fertilizer necessary for the growth of plants, a PH regulator, and the like, it becomes possible to easily perform the plant cultivation without improving the soil. In addition, in a case where the sheet S1 is long, it becomes possible to perform the larger-scale plant cultivation by burying the sheet S in the soil of the planter or field without cutting. Furthermore, in a case where the sheet S1 contains the water retaining material, it becomes possible to perform the plant cultivation without burying the sheet S1 in the soil only by giving moisture to the sheet S1. In this case, it is preferable that the base sheet BS that configures the sheet S1 have a thickness capable of holding the root of the plant. In addition, in a case where the sheet S1 is used for cultivation of fungal beds, mushrooms can be easily cultivated by giving moisture to sheet S1.

In a case where the sheet S1 is used as a plant cultivation sheet and in a case where the sheet S1 is used as a sheet for cultivation of fungal beds, the heating unit 84 heats the second web W2 at a temperature that does not affect the growth of seeds or fungi of plants. In this case, as additives to be added by the additive supply unit 52, resins having a relatively low glass transition point are selected.

### (2) Insect Repellent Sheet

By using particles containing a pest repellent or an insecticide as the solid material P, the sheet S1 can be made as an insect repellent sheet. As the repellent or the insecticide, in addition to known medicine, natural materials, such as camphor tree flour or hinoki wood flour can be used. The repellent or the insecticide can also be mixed and used. Further, particles containing the repellent or the insecticide may be added as a functional material by the additive supply unit 52.

### (3) Deodorizing Sheet

As the solid material P, by using particles containing a deodorant, an odor adsorbent, or an odor decomposer, the sheet S1 can be used as a deodorizing sheet. As the odor adsorbent, for example, particles of activated carbon or porous ceramics can be used. As the odor decomposer, for example, titanium oxide can be used. The repellent or the odor adsorbent can also be mixed and used. Further, particles containing the deodorant or odor adsorbent may be added as a functional material by the additive supply unit 52.

### (4) Dehumidifying Sheet

By using particles containing a hygroscopic material as the solid material P, the sheet S1 can be a dehumidifying sheet. As the hygroscopic material, for example, particles of a desiccant, such as silica gel, can be used. It is also possible to use a mixture of a plurality of types of hygroscopic materials. Further, particles containing the hygroscopic material may be added as a functional material by the additive supply unit 52.

### (5) Heat Insulating and Generating Sheet

By using particles containing a functional material that exhibits heat insulating properties or heat generating properties as the solid material P, the sheet S1 can be a heat insulating and generating sheet. For the type of functional material, for example, particles containing capsaicin (for example, red pepper powder) can be used. To the particles, particles of black silica may be mixed as an auxiliary material. In addition, it is also possible to use a mixture of the plurality of types of functional materials. Further, particles containing the above-described functional material or the auxiliary material may be added as a functional material by the additive supply unit 52.

### (6) Moisturizing Sheet

By using particles containing a functional material having water containing properties as the solid material P, the sheet S1 can be a moisturising sheet. As the above-described functional material, for example, the SAP particles can be used. It is also possible to use a mixture of the plurality of types of functional materials. Further, particles containing the above-described functional material may be added as a functional material by the additive supply unit 52.

### (7) Aromatic Sheet

By using particles containing an aromatic material as the solid material P, the sheet S1 can be an aromatic sheet. As the aromatic material, in addition to known perfumes, natural materials, such as hinoki powder, can be used. It is also possible to use a mixture of the plurality of types of aromatic materials. Further, particles containing the above-described aromatic material may be added as a functional material by the additive supply unit 52.

The solid material P is not limited to the examples of (1) to (7) as long as the solid material P is a particulate or the like that can be contained in the hopper 31, and by using various functional materials, the sheet S1 can be a functional sheet appropriate for various uses.

Further, the sheet S1 can also be used as paper for printing or for writing purpose, and in this case, a decorative material can be used as the solid material P.

The sheet S illustrated in (1) to (7) can add a coloring material by the additive supply unit 52. Therefore, the sheet S that contains the above-described functional material may be colored with a color that corresponds to the use of the sheet S and the type of the functional material contained therein.

As described above, the sheet manufacturing apparatus 100 according to a thirteenth embodiment to which the invention is applied includes the second web forming unit 70 that forms the second web W2 based on the mixture MX1 containing fibers. In addition, the sheet manufacturing apparatus 100 includes the solid feeder 30 that disposed the solid material P on the second web W2 formed by the second web forming unit 70 and the sheet forming unit 80 that manufactures the sheet S by processing the second web W2 on which the solid material P is disposed in the second web forming unit 70.

In addition, the sheet manufacturing apparatus 100 corresponds to an apparatus for realizing the sheet manufacturing method of the invention. The processing of the second web forming unit 70 corresponds to web forming, and the processing of the solid feeder 30 corresponds to second material disposing. The processing of the sheet forming unit 80 corresponds to sheet forming.

According to the sheet manufacturing apparatus 100 to which the sheet manufacturing apparatus and the sheet manufacturing method of the invention are applied, it is possible to manufacture the sheet S in a state where the solid material P overlaps the mixture MX1 containing fibers. Therefore, for example, it is possible to obtain the sheet S in which the mixture MX1 and the solid material P overlap each other. In the sheet S, by changing the types of the mixture MX1 and the solid material P, it is possible to change the type of the material inside the sheet S.

Further, in the sheet manufacturing apparatus 100, the mixture MX1 is a mixture containing fibers and resin, and the sheet forming unit 80 heats the second web W2 and manufactures the sheet S. Therefore, it is possible to manufacture the sheet S by melting the resin by the sheet forming unit 80 and binding the fibers contained in the mixture MX1 to a resin. In addition, since the resin contained in the mixture MX1 exhibits the action of binding the solid material P to the fiber, and the solid material P can be reliably held on the base sheet BS, it is possible to prevent the solid material P from falling off.

Further, in the solid feeder 30, it is possible to dispose the solid material P made of a material other than fibers and resin contained in the mixture MX1 on the second web W2. Therefore, it is possible to manufacture the sheet S containing the solid material P different from the fiber derived from the raw material MA and the additive added by the additive supply unit 52.

Further, in the solid feeder 30, it is possible to dispose the solid material P containing a solid material larger than the particles of the resin contained in the mixture MX1 on the second web W2. Therefore, it is possible to manufacture the sheet S on which various materials are disposed, and it is possible to manufacture a sheet having functionality as exemplified in (1) to (7).

In addition, the second web forming unit 70 includes the drum unit 61 that disperses the mixture MX1 in the air and the mesh belt 72 for depositing the mixture MX1 dispersed by the drum unit 61 and forms the second web W2. The solid feeder 30 disposes the solid material P on the second web W2 deposited on the mesh belt 72. With the configuration, by placing the solid material P on the web-like second web W2, the sheet S on which the mixture MX1 and the solid material P are laminated can be obtained.

### 15. Fourteenth Embodiment

Hereinafter, a fourteenth embodiment to which the invention is applied will be described.

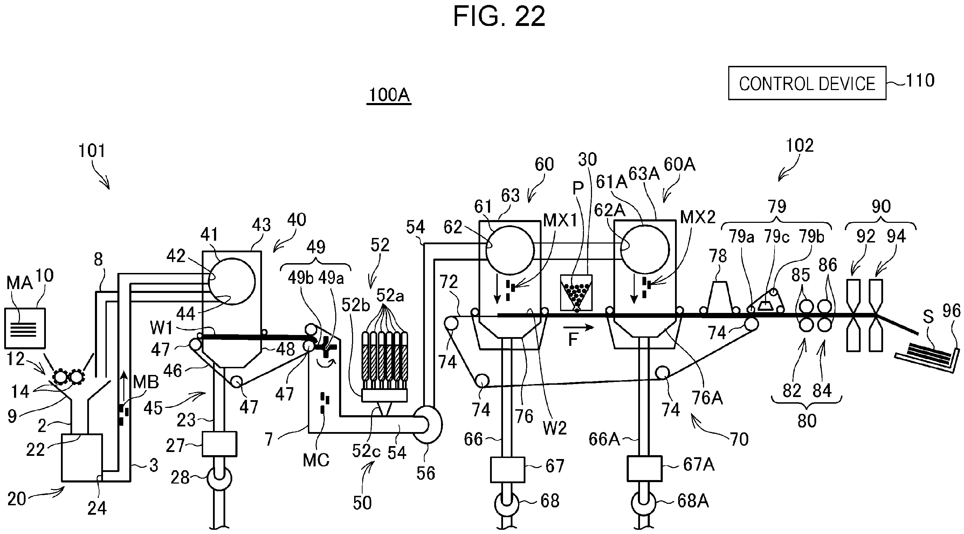

Fig. 22 is a schematic configuration view of the sheet manufacturing apparatus 100A according to the fourteenth embodiment. The sheet manufacturing apparatus 100A has a configuration in which the depositing unit 60A is disposed between the depositing unit 60 and the sheet forming unit 80 in the sheet manufacturing apparatus 100 (Fig. 19), preferably after the solid feeder 30, although it may be before.

In the fourteenth embodiment, the configurations common to those of the sheet manufacturing apparatus 100 of the thirteenth embodiment will be given the same reference numerals, and the description thereof will be omitted.

The depositing unit 60A has the drum unit 61A and the housing unit 63A that accommodates the drum unit 61A therein. The drum unit 61A is a cylindrical structure configured similarly to the drum unit 61, rotates by the power of a motor (not illustrated) similarly to the drum unit 61, and functions as a sieve. An introduction port 62A through which the mixture is introduced from the mixing blower 56 to the drum unit 61A is provided in the housing unit 63A.

In the sheet manufacturing apparatus 100A, the mixture MX1 is supplied to the depositing unit 60 as a mixture of the additive added by the additive supply unit 52 and the material MC. Further, the mixture MX2 (third material) is supplied to the depositing unit 60A (third material disposing unit). The mixture MX2 may be the same as the mixture MX1. In this case, as illustrated in Fig. 22, the pipe 54 that leads from the mixing blower 56 to the depositing unit 60 may be configured to branch and be laid to the depositing unit 60A.

In addition, the mixture MX2 may be a mixture having components different from the mixture MX1. The configuration can be realized by providing the plurality of additive supply units 52, the mixing blowers 56, and the pipe 54 that connects the additive supply units 52 and the mixing blowers 56 to each other.

The drum unit 61A is positioned above the mesh belt 72, and the drum unit 61A disperses the mixture MX2, and accordingly, the mixture MX2 descends toward the mesh belt 72. On the mesh belt 72, the second web W2 deposited by depositing unit 60 and the solid material P disposed by the solid feeder 30 are placed. The depositing unit 60A deposits the mixture MX2 on the second web W2 containing the solid material P.

In addition, the suction mechanism 76A is disposed below the depositing unit 60A. The suction mechanism 76A suctions the air from the side opposite to the drum unit 61A with respect to the mesh belt 72. The suction mechanism 76A is connected to the third dust collecting unit 67A via the pipe 66A, and the third collection blower 68A is connected to the third dust collecting unit 67A. The third dust collecting unit 67A includes a filter (not illustrated) that collects particles or fibers that have passed through the mesh belt 72. The third collection blower 68A is a blower that suctions the air through the pipe 66A. The suction mechanism 76A, the pipe 66A, the third dust collecting unit 67A, and the third collection blower 68A are configured similarly to the suction mechanism 76, the pipe 66, the second dust collecting unit 67, and the second collection blower 68.

The suction mechanism 76A suctions the air from below the mesh belt 72 by the suction force of the third collection blower 68A and collects the particles or fibers contained in the suctioned air by the third dust collecting unit 67A. The airflow suctioned by the third collection blower 68A has an effect of attracting the mixture MX2 that descends from the drum unit 61A to the mesh belt 72 and promoting the deposition. In addition, the suctioned airflow of the third collection blower 68A forms a downflow in a path where the mixture MX2 falls from the drum unit 61A, and it is also possible to expect an effect of preventing the fibers from being intertwined while falling.

The mixture MX2 is uniformly dispersed on the second web W2, for example, in the depositing unit 60A, and deposited to be flat. Therefore, the second web W2 in which the mixture MX1, the solid material P, and the mixture MX2 are laminated is formed on the mesh belt 72.

The second web W2 on which the mixture MX2 is laminated in the depositing unit 60A, is given moisture by the humidity control unit 78 and is transported by the transport unit 79. The processing by the sheet forming unit 80 and the cutting unit 90 is the same as that in the thirteenth embodiment.

The sheet manufacturing apparatus 100A includes the depositing unit 60A that is disposed between the solid feeder 30 and the sheet forming unit 80 and disposes the mixture MX2 on the second web W2 on which the solid material P is placed by the solid feeder 30. In other words, the sheet manufacturing apparatus 100A includes the depositing unit 60 positioned on the upstream side of the solid feeder 30 in the transport direction F of the second web W2 and the depositing unit 60A positioned on the downstream side of the solid feeder 30. In addition, the mixture MX1 is deposited by the depositing unit 60, the mixture MX2 is deposited by depositing unit 60A, and the solid material P is disposed by the solid feeder 30 between the mixture MX1 and the mixture MX2.

According to the configuration, it is possible to manufacture the sheet S having a configuration in which the solid material P is nipped between the mixture MX1 and the mixture MX2. For example, it is possible to manufacture the sheet S having a configuration in which the solid material P is wrapped with fibers.

### 16. Fifteenth Embodiment

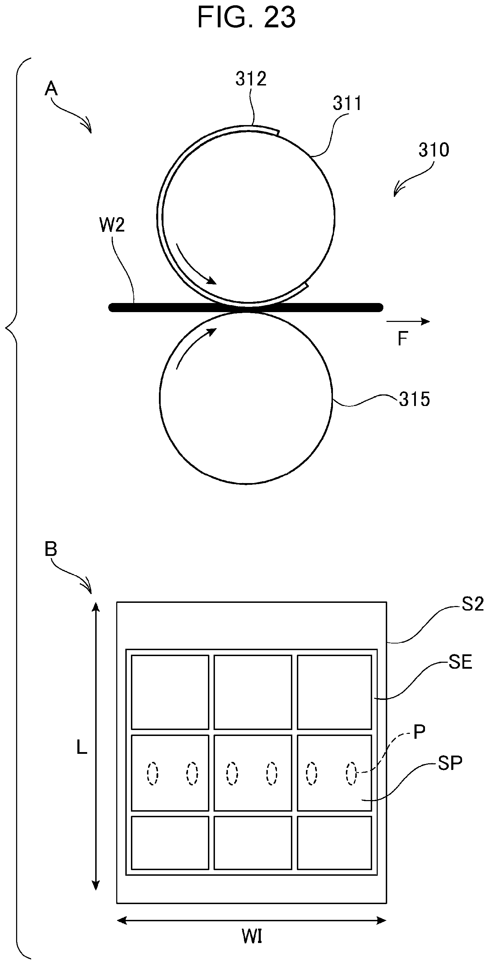

Fig. 23 is an explanatory view illustrating a configuration of the processing roller 310 according to a fifteenth embodiment. A sign A of Fig. 23 illustrates a side view illustrating a configuration of the processing roller 310, and a sign B of Fig. 23 illustrates a plan view of the sheet S2.

The processing roller 310 (pressing processing unit) is installed in the transport path of the second web W2 and includes the pair of roller 311 and roller 315. The processing roller 310 nips and transports the second web W2 between the roller 311 and the roller 315 with a predetermined nip pressure and applies a pressing force to the second web W2. The processing roller 310 includes a pressure applying mechanism (not illustrated) for applying the nip pressure to the roller 311 and the roller 315 and a driving motor (not illustrated) for driving the roller 311 or the roller 315.

On the surface of the roller 311, the projection 312 is formed. In contrast, the roller 315 is a cylindrical roller having a flat circumferential surface.

When the processing roller 310 nips and transports the second web W2, the second web W2 receives the nip pressure from the projection 312. Therefore, the second web W2 partially receives the pressing force, and a part of the second web W2 sinks. Accordingly, unevenness that corresponds to the shape of the projection 312 is formed on the second web W2.

The processing roller 310 is installed instead of the calendar rollers 85 and 85 of the pressurizing unit 82, or on the upstream side of the pressurizing unit 82, in the sheet manufacturing apparatus 100 (Fig. 19) or the sheet manufacturing apparatus 100A (Fig. 22). For example, the processing roller 310 can be installed between the transport unit 79 and the pressurizing unit 82.

The processing roller 310 forms unevenness on the second web W2 before heating the second web W2 after the solid feeder 30 disposes the solid material P by the heating unit 84.

A sign B of Fig. 23 illustrates a plan view of the sheet S2 as an example of the sheet S manufactured by using the processing roller 310. The length L and the width WI in Fig. 23 are the same as those in Fig. 21.

In the sheet S2, a lattice-like recess portion SE that extends in the length L direction and the width WI direction is formed. Since the recess portion SE is formed by pressing the second web W2 by the projection 312 and the part other than the recess portion SE is relatively projected, the sheet S2 has unevenness as a whole.

The recess portion SE of the second web W2 receives a stronger concave pressure than that at the other parts of the second web W2. In the configuration where the processing roller 310 is provided instead of the pressurizing unit 82, the recess portion SE that abuts against the projection 312 receives a stronger pressure than that the other parts pressed by the circumferential surface of the roller 311 other than the projection 312. Further, in the configuration where the processing roller 310 is provided in addition to the pressurizing unit 82, since the recess portion SE is pressed by the projection 312 and the pressurizing unit 82, the integrated value of the pressing force applied to the recess portion SE is larger than that of the other parts. As a result, in the recess portion SE, since the fibers and particles contained in the second web W2 are compressed to a higher density than that at the parts other than the recess portion SE, a sealing effect is exhibited. Therefore, the part SP surrounded by the recess portion SE is separated from the other parts. For example, as illustrated in Fig. 23, the solid material P disposed inside the part SP does not move beyond the recess portion SE to other parts.

In this manner, the processing roller 310 functions as a pressing processing unit that partially presses the second web W2 and gives an uneven shape to the second web W2. The processing roller 310 can form the part SP defined by the recess portion SE on the sheet S2 by partially pressing the second web W2 by the projection 312.

In particular, the sheet S to be manufactured by the sheet manufacturing apparatus 100A (Fig. 22) has the solid material P between the layer of the mixture MX1 and the layer of the mixture MX2. A processing roller 310 is provided in the sheet manufacturing apparatus 100A, the solid feeder 30 disposes the solid material P in accordance with the position of the part SP, and accordingly, the solid material P is accommodated at the part SP. In this case, the pocket-like part SP is formed by compressing two layers on the sheet S2 with the recess portion SE, and the solid material P is accommodated in the pocket.

In addition, the projection 312 can also form the recess portion SE on the sheet S2 as a decoration. In this case, by the configuration in which the processing roller 310 is provided in the sheet manufacturing apparatus 100 or the sheet manufacturing apparatus 100A, it is possible to manufacture the sheet S2 having the uneven decoration.

### 17. Sixteenth Embodiment

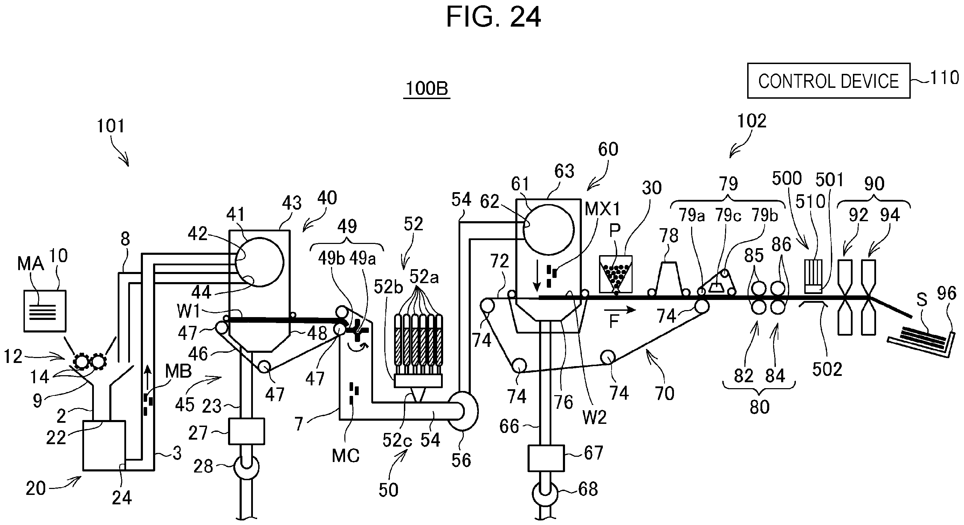

Fig. 24 is a schematic configuration view of the sheet manufacturing apparatus 100B according to a sixteenth embodiment. The sheet manufacturing apparatus 100B has a configuration in which the printing unit 500 is disposed between the sheet forming unit 80 and the cutting unit 90 in the sheet manufacturing apparatus 100 (Fig. 19).

In the sixteenth embodiment, the configurations common to those of the sheet manufacturing apparatus 100 of the thirteenth embodiment will be given the same reference numerals, and the description thereof will be omitted.

The printing unit 500 includes the print head 501 that ejects ink toward the sheet S and the ink cartridge 510 that accommodates ink ejected by the print head 501. The print head 501 is capable of ejecting a plurality of colors of ink, and the ink cartridge 510 is provided corresponding to the number of colors of the ink ejected by the print head 501. Further, the printing unit 500 includes an ink supply mechanism (not illustrated) for supplying the ink from the ink cartridge 510 to the print head 501, a nozzle (not illustrated) for ejecting the ink from the print head 501, and the like.

In the printing unit 500, an image is formed by ejecting the ink onto the second web W2, that is, the sheet S processed into a sheet shape by the sheet forming unit 80, by the print head 501. In the printing unit 500, for example, an image can be formed in the width direction (for example, the width WI direction in Fig. 21) of the sheet S by scanning the print head 501 in a direction intersecting the transport direction F. Further, the print head 501 may be a line inkjet head that extends in the width direction of the sheet S.

The second web W2 is pressurized and heated in the sheet forming unit 80 and becomes the sheet S, and an image is formed by the printing unit 500. After this, the sheet S is cut by the cutting unit 90 and is discharged to the discharge unit 96.

The printing unit 500 executes the printing under the control of the control device 110. The control device 110 drives the printing unit 500 according to image data for printing and forms an image. Further, the control device 110 can form an image at a set position on the sheet S cut into a predetermined size by controlling the printing position on the sheet S with reference to the position where the first cutting unit 92 cuts the sheet S.

In this manner, since the sheet manufacturing apparatus 100B includes the printing unit 500 that performs printing on the sheet S manufactured by the sheet forming unit 80, an image can be formed on the sheet S containing the solid material P.

Further, the control device 110 can form an image in accordance with the position where the solid feeder 30 disposes the solid material P.

In other words, it is possible to form an image in accordance with the position where the solid material P is disposed in the sheet S. For example, in a case where the solid material P is disposed on the sheet S as illustrated in a sign A of Fig. 21, the control device 110 can print an image that shows the position of the solid material P or an image that describes the type or use of the solid material P by the printing unit 500.

Further, in the examples (1) to (7) described for the use of the sheet S, the description of the use or the usage method of the sheet S may be printed by the printing unit 500. In this case, with respect to the solid material P appropriate for a specific use and/or the sheet S containing the additives, it is possible to notify the use or the usage method by the image formed on the sheet S.

### 18. Seventeenth Embodiment

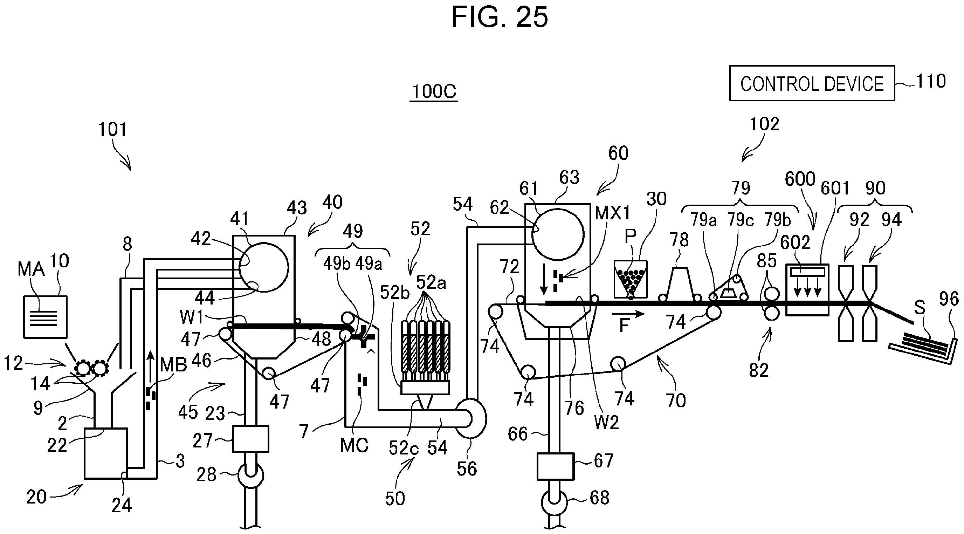

Fig. 25 is a schematic configuration view of the sheet manufacturing apparatus 100C according to a seventeenth embodiment. The sheet manufacturing apparatus 100C has a configuration in which the heating unit 600 is provided instead of the heating unit 84 in the sheet manufacturing apparatus 100 (Fig. 19).

In the seventeenth embodiment, the configurations common to those of the sheet manufacturing apparatus 100 of the thirteenth embodiment will be given the same reference numerals, and the description thereof will be omitted.

The heating unit 600 has a furnace 601 that accommodates the upper portion and preferably the lower portion of the transport path of the second web W2, and a heat source 602 accommodated in the furnace 601. The second web W2 passes through the inside of the furnace 601 between the pressurizing unit 82 and the cutting unit 90.

The heat source 602 is an infrared heater that emits infrared rays for heating, or a heater having a heating element. As a heating element, a resistance heating element can be used, and for example, a nichrome wire heater or a ceramic heater can be adopted. The heating unit 600 heats the second web W2 in the furnace 601 by radiant heating by the infrared rays emitted from the heat source 602 or transfer heating by raising the temperature of the internal space of the furnace 601 by the heat source 602.

In addition, the furnace 601 may be configured to perform induction heating, dielectric heating, or heating of the second web W2 by a heat pump type heater. Further, without providing the furnace 601, the second web W2 may be heated by the heat source 602 disposed to be close to the transport path of the second web W2.

The heating unit 600 is a non-pressurizing type heating unit that heats the second web W2 without pressurizing the second web W2. In another expression, a heat source 602 that is not in contact with the second web W2 is provided, and the second web W2 is heated in a non-contact manner.

In this manner, in the sheet manufacturing apparatus 100C, the sheet forming unit 80 includes the pressurizing unit 82 that pressurizes the second web W2, and the heating unit 600 that heats the second web W2 pressurized by the pressurizing unit 82 in a non-pressurized state. According to the sheet manufacturing apparatus 100, after adjusting the density of the second web W2 by pressurizing using the pressurizing unit 82, the adjusted density of the second web W2 is maintained and the resin of the second web W2 is melted, and the sheet S can be manufactured. Therefore, the density of the sheet S can be finely adjusted with high accuracy, and the hardness or basis weight of the sheet S can be adjusted in any manner.

### 19. Eighteenth Embodiment

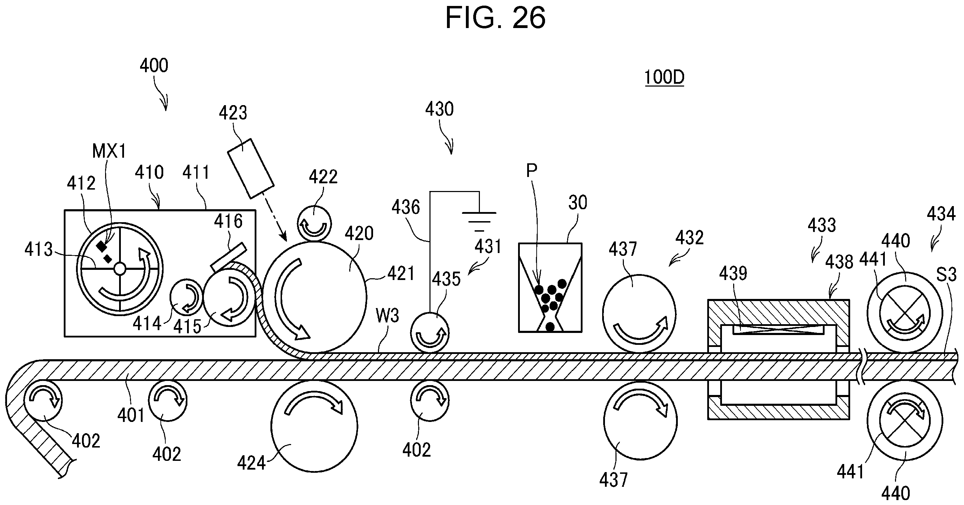

Fig. 26 is a schematic configuration view of the sheet manufacturing apparatus 100D according to an eighteenth embodiment. In the eighteenth embodiment, the configurations common to those of the sheet manufacturing apparatus 100 of the thirteenth embodiment will be given the same reference numerals, and the description thereof will be omitted.

The sheet manufacturing apparatus 100D includes the manufacturing unit 400 instead of the manufacturing unit 102 in the sheet manufacturing apparatus 100 (Fig. 19). In other words, the manufacturing unit 400 is provided instead of the depositing unit 60, the second web forming unit 70, the transport unit 79, and the sheet forming unit 80 of the sheet manufacturing apparatus 100.

The manufacturing unit 400 molds the mixture MX1 in which fibers and additives are mixed with each other by the additive supply unit 52 of the defibration processing unit 101 and the mixing blower 56 into a web shape by an electrostatic method, and obtains second web (which for convenience is termed the third web W3 below) (web). In other words, the manufacturing unit 400 manufactures the sheet S3 by electrostatically transferring the mixture MX1, which is a fiber-containing material, to the transport belt 401, which is an object to be transferred, and performs post-treatment to adjust the surface properties of the mixture MX1.

The manufacturing unit 400 includes the supply unit 410 that supplies the mixture MX1, the carrier 420 for carrying the supplied mixture MX1, the transport belt 401 on which the carried mixture MX1 is electrostatically transferred, and the processing unit 430 for post-treatment.

The mixture MX1 is common or similar to the thirteenth embodiment, and is a mixture of fibers derived from the raw material MA and additives added by the additive supply unit 52. Further, the mixture MX1 may contain a charge control agent or an electrical charge control agent for obtaining charging properties of the mixture MX1.

A supply unit 410 accommodates a storage unit 412, an agitator 413, a roller 414, a first carrier 415, and a blade 416, in the housing unit 411.

The storage unit 412 stores the mixture MX1 therein. The agitator 413 agitates the mixture MX1 in the storage unit 412 and charges the mixture MX1 by friction during the agitation.

The mixture MX1 is supplied to the first carrier 415 by the rotation of the roller 414. The first carrier 415 has a potential difference with the roller 414, and the mixture MX1 electrostatically adheres by the potential difference. The blade 416 adjusts the thickness or the adhesion amount of the mixture MX1 that adheres to the first carrier 415, adjusts the mixture MX1 into a sheet shape, and charges the mixture MX1 by friction.

The carrier 420 has a potential difference with the first carrier 415, and the mixture MX1 electrostatically adheres to the carrier 420. The carrier 420 is a rotating roller member, and transfers the mixture MX1 carried by the carrier 420 to the transport belt 401.

Around the carrier 420, the charging unit 422 that charges the outer circumferential surface 421 of the carrier 420 and the exposure unit 423 that adjusts the potential of the outer circumferential surface 421 are provided. Furthermore, around the carrier 420, the transfer unit 424 is provided for transferring the mixture MX1 to the transport belt 401 by an electrostatic force generated by a potential difference with the carrier 420.

The transfer unit 424 pressurizes the mixture MX1 transferred to the transport belt 401 with the carrier 420 and makes the thickness of the mixture MX1 uniform. Accordingly, the third web W3 having a uniform thickness is formed on the transport belt 401. The carrier 420 and the transfer unit 424 configure the web forming unit.

The transport belt 401 is configured with an endless belt and is transported by the plurality of rollers 402. It is preferable that a surface of the transport belt 401 to which the mixture MX1 is transferred be made of a resin having medium to high resistance (volume resistivity of 107 to 1011 Ω·cm). As the type of resin, for example, a type in which the carbon black is kneaded in a fluorine-based resin can be used, but it is needless to say that other materials can also be used.

The processing unit 430 includes a leveling processing unit 431 that smooths the surface of the third web W3 and a pressurizing processing unit 432 that pressurizes the third web W3. Further, a semi-solidification processing unit 433 for semi-solidifying the surface of the third web W3 and a solidifying unit 434 for solidifying the third web W3 are provided. The leveling processing unit 431 has a leveling roller 435 of which at least the outer circumferential surface is made of metal, smooths the surface of the third web W3 by the leveling roller 435, and removes electricity of the third web W3 via a ground wire 436.

The pressurizing processing unit 432 bonds the fibers contained in the third web W3 and fibers and resin to each other by performing the pressurizing by a pressurizing roller 437, and makes the density uniform.

The semi-solidification processing unit 433 includes a chamber 438 configured with a heat insulating material and a heater 439 provided in the chamber 438, and the surface of the third web W3 is semi-solidified by performing the heating by the heater 439.

The solidifying unit 434 includes a solidifying roller 440 and a heater 441 provided in the solidifying roller 440. The solidifying unit 434 heats the solidifying roller 440 by energizing the heater 441 and pressurizes the third web W3 while heating the third web W3 by the solidifying roller 440. Accordingly, the resin contained in the third web W3 is melted, and the fibers and the resin of the third web W3 are bound to each other, and the sheet S3 is formed. After the sheet S3 passes through the solidifying roller 440, for example, the sheet S3 is naturally cooled, bound, and solidified.

A blowing fan (not illustrated) for promoting the separation of the sheet S3 from the transport belt 401, the cutting unit 90 (not illustrated) that cuts the sheet S3, and the like may be provided on the downstream side of the processing unit 430.

The manufacturing unit 400 includes the solid feeder 30 between the leveling roller 435 and the pressurizing roller 437. The solid feeder 30 is common to the configuration described in the thirteenth embodiment. In the manufacturing unit 400, the solid material P is placed by the solid feeder 30 on the third web W3 from which the electricity is removed by the leveling roller 435. Accordingly, the sheet S3 can be manufactured in a state where the solid material P is placed on the third web W3 made of the mixture MX1. Since the solid material P is disposed on the third web W3 from which the electricity is removed, the solid feeder 30 has an advantage that the position and the like of the solid material P are unlikely to be affected by static electricity.

In addition, the solid feeder 30 may have a configuration in which the solid material P is disposed on the third web W3 before the electricity is removed from it by the leveling roller 435. In this case, there is an advantage that the solid material P can be excellently adsorbed to the third web W3 by the static electricity.

Compared to the manufacturing unit 102 of the thirteenth embodiment, the manufacturing unit 400 does not require defibration, sorting, suctioning, web forming, and the like, and thus, the configuration of the sheet manufacturing apparatus 100D can be simplified. Therefore, there is an advantage that the manufacturing time of the sheet S3 can be shortened, or the like.

### 20. Nineteenth Embodiment

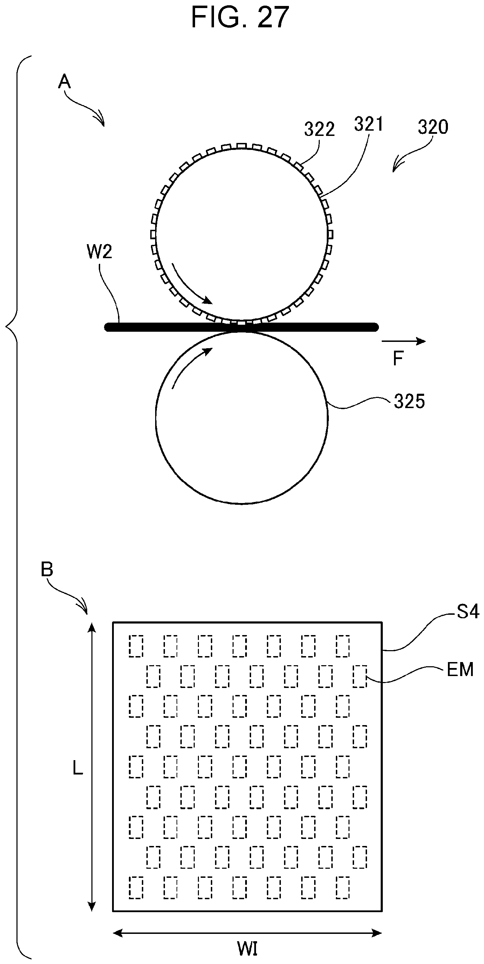

Fig. 27 is an explanatory view illustrating a configuration of the processing roller 320 according to a nineteenth embodiment. A sign A of Fig. 27 illustrates a side view illustrating a configuration of the processing roller 320, and a sign B of Fig. 27 illustrates a plan view of the sheet S4.

The processing roller 320 (pressing processing unit) is installed in the transport path of the second web W2 and includes the pair of roller 321 and roller 325. The processing roller 320 nips and transports the second web W2 between the roller 321 and the roller 325 with a predetermined nip pressure and applies a pressing force to the second web W2. The processing roller 320 includes a pressure applying mechanism (not illustrated) for applying the nip pressure to the roller 321 and the roller 325 and a driving motor (not illustrated) for driving the roller 321 or the roller 325.

On the surface of the roller 321, the projection 322 is formed. In contrast, the roller 325 is a cylindrical roller having a flat circumferential surface.

When the processing roller 320 nips and transports the second web W2, the second web W2 receives the nip pressure from the projection 322. Therefore, the second web W2 partially receives the pressing force, and a part of the second web W2 sinks. Accordingly, unevenness that corresponds to the shape of the projection 322 is formed on the second web W2.

The processing roller 320 is installed instead of the calendar rollers 85 and 85 of the pressurizing unit 82, in the sheet manufacturing apparatus 100 (Fig. 19), the sheet manufacturing apparatus 100A (Fig. 22), the sheet manufacturing apparatus 100B (Fig. 24), and the sheet manufacturing apparatus 100C (Fig. 25). Otherwise, the processing roller 320 may be additionally installed on the upstream side of the pressurizing unit 82. For example, the processing roller 320 can be installed between the transport unit 79 and the pressurizing unit 82.

The processing roller 320 forms unevenness on the second web W2 before heating the second web W2 after the solid feeder 30 disposes the solid material P by the heating unit 84.

A sign B of Fig. 27 illustrates a plan view of the sheet S4 as an example of the sheet S manufactured by using the processing roller 320. The length L and the width WI in Fig. 27 are the same as those in Fig. 21.

In the sheet S4, the recess portion EM that extends in the length L direction and the width WI direction is formed. Since the recess portion EM is a recess portion formed by the so-called embossing, the second web W2 is formed by pressing the second web S2 by the projection 322, and the part other than the recess portion EM is relatively projected, the sheet S4 has unevenness as a whole.

In this manner, the processing roller 320 functions as a pressing processing unit that partially presses the second web W2 and gives an uneven shape to the second web W2. The processing roller 320 can perform the embossing with respect to the sheet S by partially pressing the second web W2 by the projection 322. Therefore, it is possible to change the touch of the sheet S4, and it is possible to manufacture the sheet S4 having the uneven decoration.

In the nineteenth embodiment, a configuration in which one-side embossing is performed with respect to the sheet S4 by using the roller 321 having the projection 322 and the roller 325 configured with a smooth surface is illustrated as an example, but a configuration in which double-side embossing is performed may be employed. In other words, the projection 322 may also be provided in the roller 325.

### 21. Other Embodiments

Each of the above-described embodiments is merely a specific aspect of implementing the invention described in the Claims, does not limit the invention, and can be implemented in various aspects as described below, for example, without departing from the scope thereof.

For example, in the fifteenth embodiment, an example in which the processing roller 310 is applied to the sheet manufacturing apparatus 100 of the thirteenth embodiment and the sheet manufacturing apparatus 100A of the fourteenth embodiment has been described. The invention is not limited thereto, and the processing roller 310 can also be applied to the sheet manufacturing apparatus 100B of the sixteenth embodiment, the sheet manufacturing apparatus 100C of the seventeenth embodiment, and the sheet manufacturing apparatus 100D of the eighteenth embodiment.

Further, the printing unit 500 described in the sixteenth embodiment is not limited to the sheet manufacturing apparatus 100B, and can also be applied to the sheet manufacturing apparatuses 100, 100A, 100C, and 100D.

In addition, the heating unit 600 described in the seventeenth embodiment is not limited to the sheet manufacturing apparatus 100C, and can also be applied to the sheet manufacturing apparatuses 100, 100A, 100B, and 100D.

Furthermore, it is also possible to select and combine two or more of the processing rollers 310 and 320, the printing unit 500, and the heating unit 600, and to apply the combination to one sheet manufacturing apparatus 100, and 100A to 100D.

In addition, the sheet manufacturing apparatus 100 may be configured to manufacture not only the sheet S but also a board-like or web-like manufactured product configured with hard sheets or laminated sheets. In addition, the manufactured product is not limited to paper, but may be a nonwoven fabric. The nature of the sheet S is not particularly limited, may be paper which can be used as a recording paper (for example, a so-called PPC paper sheet) for the purpose of writing or printing, or may be wallpaper, wrapping paper, color paper, drawing paper, Kent paper or the like. In addition, in a case where the sheet S is a nonwoven fabric, in addition to a general nonwoven fabric, the sheet S may be fiber board, tissue paper, kitchen paper, cleaner, filter, liquid absorbing material, sound absorbing material, cushioning material, mat, and the like.

In addition, in the above-described embodiment, as the sheet manufacturing apparatus and the fiber raw material regenerating apparatus of the invention, the dry type sheet manufacturing apparatus 100 that manufactures the sheet S by obtaining the material by defibrating the raw material in the air and by using the material and the resin, has been described. The target of application of the invention is not limited thereto, and the invention can also be applied to a so-called wet type sheet manufacturing apparatus in which the raw material containing fibers are dissolved or suspended in a solvent, such as water, and the raw material is processed into a sheet. In addition, the invention can also be applied to an electrostatic type sheet manufacturing apparatus in which the material containing fibers defibrated in the air is adsorbed onto the surface of the drum by static electricity or the like and the raw material adsorbed onto the drum is processed into a sheet.

## Claims

1. A sheet manufacturing apparatus comprising:
a first web forming unit (60A) configured to form a first web (W2) based on a first material (MX1) containing fibers;
a first processing unit (80A) configured to perform processing including at least any one of pressurizing processing and heating processing with respect to the first web formed by the first web forming unit;
a second web forming unit (60B) configured to form a second web (W3) by laminating a second material (MX2) containing fibers on the first web processed by the first processing unit; and
a second processing unit (80B) configured to mold a sheet by performing processing including at least any one of pressurizing processing and heating processing with respect to the second web formed by the second web forming unit,
wherein the first web forming unit includes a first dispersing unit configured to disperse the first material in the air and a first depositing unit configured to deposit the first material dispersed by the first dispersing unit and form the first web, and
wherein the second web forming unit includes a second dispersing unit configured to disperse the second material in the air and a second depositing unit configured to deposit the second material dispersed by the second dispersing unit on the first web processed by the first processing unit and form the second web.

2. The sheet manufacturing apparatus according to Claim 1, further comprising:
a first cutting unit (11) configured to cut the first web processed by the first processing unit,
wherein the second web forming unit processes the first web cut by the first cutting unit.

3. The sheet manufacturing apparatus according to Claim 1 or Claim 2,
wherein the first processing unit includes first pressurizing rollers (85) to nip and pressurize the first web.

4. The sheet manufacturing apparatus according to any one of claims 1 to 3, wherein the first processing unit includes first heating rollers (86) to nip and heat the first web.

5. The sheet manufacturing apparatus according to any one of claims 1 to 4, wherein the second processing unit includes second pressurizing rollers (85) to nip and pressurize the second web.

6. The sheet manufacturing apparatus according to any one of claims 1 to 5, wherein the second processing unit includes a heating unit (86) to heat the second web.

7. The sheet manufacturing apparatus according to Claim 6,
wherein the second processing unit includes a second pressurizing unit (85) to pressurize the second web and the heating unit (601, 602) to heat the second web pressurized by the second pressurizing unit in a non-pressurized state.

8. The sheet manufacturing apparatus according to any one of claims 1 to 7, further comprising:
a pressing processing unit (310, 320) to partially press the first web and give an uneven shape to the first web,
wherein the first processing unit is configured to process the first web processed by the pressing processing unit.

9. The sheet manufacturing apparatus according to any one of claims 1 to 8, further comprising:
a printing unit (500) to perform printing on the sheet molded by the second processing unit.

10. A sheet manufacturing method comprising:
forming a first web (W2) based on a first material (MX1) containing fibers;
performing processing including at least any one of pressurizing processing and heating processing with respect to the first web formed in the forming;
forming a second web (W3) by laminating a second material (MX2) containing fibers on the first web processed in the performing of the processing; and
molding a sheet by performing processing including at least any one of pressurizing processing and heating processing with respect to the second web formed in the forming of the second web,
wherein forming the first web includes dispersing the first material in the air and depositing the first material to form the first web, and
wherein forming the second web includes dispersing the second material in the air and depositing the second material on the first web to form the second web.

## Patentansprüche

1. Blattherstellungsvorrichtung, umfassend:
eine erste Bahnbildungseinheit (60A), die ausgestaltet ist, eine erste Bahn (W2) auf Basis eines ersten Materials (MX1) zu bilden, das Fasern enthält;
eine erste Bearbeitungseinheit (80A), die ausgestaltet ist, Bearbeitung durchzuführen, enthaltend mindestens eines von Druckbeaufschlagungsbearbeitung und Erhitzungsbearbeitung in Bezug auf die erste Bahn, die durch die erste Bahnbildungseinheit gebildet wird;
eine zweite Bahnbildungseinheit (60B), die ausgestaltet ist, eine zweite Bahn (W3) durch Aufschichten eines zweiten Materials (MX2), das Fasern enthält, auf die erste Bahn, die durch die erste Bearbeitungseinheit bearbeitet wurde, zu bilden; und
eine zweite Bearbeitungseinheit (80B), die ausgestaltet ist, ein Blatt durch Durchführen von Bearbeitung zu formen, die mindestens eines von Druckbeaufschlagungsbearbeitung und Erhitzungsbearbeitung in Bezug auf die zweite Bahn enthält, die durch die zweite Bahnbildungseinheit gebildet wurde,
wobei die erste Bahnbildungseinheit eine erste Dispergiereinheit enthält, die ausgestaltet ist, das erste Material in der Luft zu dispergieren, und eine erste Abscheidungseinheit, die ausgestaltet ist, das erste Material, das durch die erste Dispergiereinheit dispergiert wurde, abzuscheiden und die erste Bahn zu bilden, und
wobei die zweite Bahnbildungseinheit eine zweite Dispergiereinheit enthält, die ausgestaltet ist, das zweite Material in der Luft zu dispergieren, und eine zweite Abscheidungseinheit, die ausgestaltet ist, das zweite Material, das durch die zweite Dispergiereinheit dispergiert wurde, auf der ersten Bahn abzuscheiden, die durch die erste Bearbeitungseinheit bearbeitet wurde, und die zweite Bahn zu bilden.

2. Blattherstellungsvorrichtung nach Anspruch 1, weiter umfassend:
eine erste Schneideeinheit (11), die ausgestaltet ist, die erste Bahn, die durch die erste Bearbeitungseinheit bearbeitet wurde, zu schneiden,
wobei die zweite Bahnbildungseinheit die erste Bahn bearbeitet, die von der ersten Schneideeinheit geschnitten wurde.

3. Blattherstellungsvorrichtung nach Anspruch 1 oder Anspruch 2,
wobei die erste Bearbeitungseinheit erste Druckbeaufschlagungswalzen (85) enthält, um die erste Bahn einzuklemmen und mit Druck zu beaufschlagen.

4. Blattherstellungsvorrichtung nach einem der Ansprüche 1 bis 3,
wobei die erste Bearbeitungseinheit erste Erhitzungswalzen (86) enthält, um die erste Bahn einzuklemmen und zu erhitzen.

5. Blattherstellungsvorrichtung nach einem der Ansprüche 1 bis 4,
wobei die zweite Bearbeitungseinheit zweite Druckbeaufschlagungswalzen (85) enthält, um die zweite Bahn einzuklemmen und mit Druck zu beaufschlagen.

6. Blattherstellungsvorrichtung nach einem der Ansprüche 1 bis 5,
wobei die zweite Bearbeitungseinheit eine Heizeinheit (86) enthält, um die zweite Bahn zu erhitzen.

7. Blattherstellungsvorrichtung nach Anspruch 6,
wobei die zweite Bearbeitungseinheit eine zweite Druckbeaufschlagungseinheit (85), um die zweite Bahn mit Druck zu beaufschlagen, und die Heizeinheit (601, 602) enthält, um die zweite Bahn, die durch die zweite Druckbeaufschlagungseinheit mit Druck beaufschlagt wurde, in einem nicht mit Druck beaufschlagten Zustand zu erhitzen.

8. Blattherstellungsvorrichtung nach einem der Ansprüche 1 bis 7, weiter umfassend:
eine Pressbearbeitungseinheit (310, 320), um die erste Bahn teilweise zu pressen und der ersten Bahn eine unebene Form zu verleihen,
wobei die erste Bearbeitungseinheit ausgestaltet ist, die erste Bahn zu bearbeiten, die durch die Pressbearbeitungseinheit bearbeitet wurde.

9. Blattherstellungsvorrichtung nach einem der Ansprüche 1 bis 8, weiter umfassend:
eine Druckeinheit (500), um Druck auf dem Blatt durchzuführen, das durch die zweite Bearbeitungseinheit geformt wurde.

10. Blattherstellungsverfahren, umfassend:
Bilden einer ersten Bahn (W2) basierend auf einem ersten Material (MX1), das Fasern enthält;
Durchführen einer Bearbeitung, enthaltend mindestens eines von Druckbeaufschlagungsbearbeitung und Erhitzungsbearbeitung in Bezug auf die erste Bahn, die beim Bilden gebildet wird;
Bilden einer zweiten Bahn (W3) durch Aufschichten eines zweiten Materials (MX2), das Fasern enthält, auf die erste Bahn, die beim Durchführen der Bearbeitung bearbeitet wurde; und
Formen eines Blatts durch Durchführen von Bearbeitung, die mindestens eines von Druckbeaufschlagungsbearbeitung und Erhitzungsbearbeitung in Bezug auf die zweite Bahn enthält, die beim Bilden der zweiten Bahn gebildet wurde,
wobei Bilden der ersten Bahn Dispergieren des ersten Materials in der Luft und Abscheiden des ersten Materials enthält, um die erste Bahn zu bilden, und
wobei Bilden der zweiten Bahn Dispergieren des zweiten Materials in der Luft und Abscheiden des zweiten Materials auf der ersten Bahn, um die zweite Bahn zu bilden, enthält.

## Revendications

1. Appareil de fabrication de feuille comprenant :
une unité de formation de première bande (60A) configurée pour former une première bande (W2) sur la base d'un premier matériau (MX1) contenant des fibres ;
une première unité de traitement (80A) configurée pour réaliser un traitement comprenant au moins l'un parmi un traitement de pression et un traitement de chauffage par rapport à la première bande formée par l'unité de formation de première bande ;
une unité de formation de deuxième bande (60B) configurée pour former une deuxième bande (W3) en laminant un deuxième matériau (MX2) contenant des fibres sur la première bande traitée par la première unité de traitement ; et
une deuxième unité de traitement (80B) configurée pour mouler une bande en effectuant un traitement comprenant au moins l'un parmi un traitement de pression et un traitement de chauffage par rapport à la deuxième bande formée par l'unité de formation de deuxième bande,
dans lequel l'unité de formation de première bande comprend une première unité de dispersion configurée pour disperser le premier matériau dans l'air et une première unité de dépôt configurée pour déposer le premier matériau dispersé par la première unité de dispersion et former la première bande, et
dans lequel l'unité de formation de deuxième bande comprend une deuxième unité de dispersion configurée pour disperser le deuxième matériau dans l'air et une deuxième unité de dépôt configurée pour déposer le deuxième matériau dispersé par la deuxième unité de dispersion sur la première bande traitée par la première unité de traitement et former la deuxième bande.

2. Appareil de fabrication de feuille selon la revendication 1, comprenant en outre :
une première unité de coupe (11) configurée pour couper la première bande traitée par la première unité de traitement,
dans lequel l'unité de formation de deuxième bande traite la première bande coupée par la première unité de coupe.

3. Appareil de fabrication de feuille selon la revendication 1 ou la revendication 2,
dans lequel la première unité de traitement comprend des premiers rouleaux de pression (85) pour pincer et exercer une pression sur la première bande.

4. Appareil de fabrication de feuille selon l'une quelconque des revendications 1 à 3,
dans lequel la première unité de traitement comprend des premiers rouleaux de chauffage (86) pour pincer et chauffer la première bande.

5. Appareil de fabrication de feuille selon l'une quelconque des revendications 1 à 4,
dans lequel la deuxième unité de traitement comprend des deuxièmes rouleaux de pression (85) pour pincer et exercer une pression sur la deuxième bande.

6. Appareil de fabrication de feuille selon l'une quelconque des revendications 1 à 5,
dans lequel la deuxième unité de traitement comprend une unité de chauffage (86) pour chauffer la deuxième bande.

7. Appareil de fabrication de feuille selon la revendication 6,
dans lequel la deuxième unité de traitement comprend une deuxième unité de pression (85) pour exercer une pression sur la deuxième bande, et l'unité de chauffage (601, 602) pour chauffer la deuxième bande sur laquelle la deuxième unité de pression a exercé une pression dans un état d'absence de pression.

8. Appareil de fabrication de feuille selon l'une quelconque des revendications 1 à 7, comprenant en outre :
une unité de traitement de pression (310, 320) pour partiellement exercer une pression sur la première bande et donner une forme irrégulière à la première bande,
dans lequel la première unité de traitement est configurée pour traiter la première bande traitée par l'unité de traitement de pression.

9. Appareil de fabrication de feuille selon l'une quelconque des revendications 1 à 8, comprenant en outre :
une unité d'impression (500) pour effectuer l'impression sur la feuille moulée par la deuxième unité de traitement.

10. Procédé de fabrication de feuille comprenant :
la formation d'une première bande (W2) sur la base d'un premier matériau (MX1) contenant des fibres ;
la réalisation d'un traitement comprenant au moins l'un parmi un traitement de pression et un traitement de chauffage par rapport à la première bande formée lors de la formation ;
la formation d'une deuxième bande (W3) en laminant un deuxième matériau (MX2) contenant des fibres sur la première bande traitée lors de la réalisation du traitement ; et
le moulage d'une feuille en effectuant un traitement comprenant au moins l'un parmi un traitement de pression et un traitement de chauffage par rapport à la deuxième bande formée lors de la formation de la deuxième bande,
dans lequel la formation de la première bande comprend la dispersion du premier matériau dans l'air et le dépôt du premier matériau pour former la première bande, et
dans lequel la formation de la deuxième bande comprend la dispersion du deuxième matériau dans l'air et le dépôt du deuxième matériau sur la première bande pour former la deuxième bande.
